# FASCICULE DE BREVET EUROPEEN

(11) **EP 2 406 261 B1**
(45) Date de publication et mention de la délivrance du brevet: **09.01.2013**
(21) Numéro de dépôt: 10715918.8
(22) Date de dépôt: 11.03.2010
(51) Int. Cl.: C07D 471/04, C07D 471/20, C07D 491/22, C07D 495/22, A61K 31/437, A61K 31/438, A61P 35/00

(54) **DÉRIVÉS DE 10-AMINO-1,2,3,4-TÉTRAHYDROPYRIDO[2,1-A]ISOINDOL-6(10BH)-ONES, LEUR PROCÉDÉ DE PRÉPARATION ET LEURS UTILISATIONS THÉRAPEUTIQUES**
10-AMINO-1,2,3,4-TETRAHYDROPYRIDO[2,1-A] ISOINDOL-6(10BH)-ONDERIVATE, VERFAHREN ZU DEREN HERSTELLUNG UND DEREN THERAPEUTISCHE ANWENDUNGEN
10-AMINO-1,2,3,4-TETRAHYDROPYRIDO[2,1-A]ISOINDOL-6(10BH)-ONE DERIVATIVES, METHOD FOR PREPARING SAME, AND THERAPEUTIC USES THEREOF

(30) Priorité: 12.03.2009 FR 0951551
(43) Date de publication de la demande: 18.01.2012
(73) Titulaire: Centre National de la Recherche Scientifique (C.N.R.S.), 75016 Paris (FR); Universite D'Orleans, 45067 Orleans Cedex 2 (FR)
(72) Inventeur: ROUTIER, Sylvain, César, Léonce, F-45510 Tigy (FR); GUILLAUMET, Gérald, F-45650 Saint Jean Le Blanc (FR); MEIJER, Laurent, 29680 Roscoff (FR); CHIURATO, Mattéo, 36061 Bassano del Grappa (IT); BOULAHJAR, Rajâa, 45100 Orléans (FR)
(74) Mandataire: Blot, Philippe Robert Emile
(86) Numéro de dépôt international: PCT/FR2010/050416
(87) Numéro de publication internationale: WO 2010/103240

(56) Documents cités:
- EP-A- 1 199 306

## Description

La présente invention a pour objet des dérivés de 10-amino-1,2,3,4-tétrahydropyrido[2,1-a]isoindol-6(10b*H*)-ones et leurs procédés de préparation. Elle a également pour objet les utilisations thérapeutiques desdits nouveaux dérivés notamment en tant qu'inhibiteurs de kinases.

Les protéines kinases catalysent la phosphorylation de résidus de type sérine, thréonine et tyrosine en utilisant l'ATP ou le GTP comme donneur de phosphate. Les kinases font actuellement partie des cibles biologiques les plus étudiées car elles sont impliquées dans de nombreux processus biologiques. L'inhibition enzymatique sélective constitue une stratégie privilégiée pour la mise au point de nouvelles chimiothérapies. Les protéines kinases sont parmi les cibles biologiques les plus prisées par l'industrie pharmaceutique. Le nombre très élevé de kinases a rendu difficile la détermination du rôle précis de chacune d'entre elles. Elles sont impliquées dans des processus variés comme la croissance et la différenciation cellulaire, ainsi que la promotion tumorale, l'orchestration du cycle cellulaire ou le fonctionnement des cellules neuronales. Une sous- ou sur- expression de ces enzymes a été rapportée dans une grande gamme de tissus néoplasiques et pré-néoplasiques. Lors d'une surexpression, des inhibiteurs puissants de kinases peuvent être utiles comme agents antiprolifératifs.

Considérant l'importance de ces réactions dans les processus physiologiques et cellulaires, il n'est donc pas surprenant que des dysfonctionnements de ces systèmes de régulations deviennent la cause ou la conséquence d'affections humaines. A cet égard, un grand nombre de pathologies résulte de la mutation des kinases et phosphatases. Ainsi, il est couramment accepté que des phosphorylations anormales sont responsables de la majeure partie des pathologies comme les cancers, le diabète, l'arthrite rhumatoïde, la maladie d'Alzheimer, etc.... Actuellement l'erlotinib (tarceva^{®}) et l'imatinib (gleevec^{®}) ont été lancés sur le marché comme inhibiteurs de kinases.

En raison du rôle-clé joué par les kinases cycline-dépendantes (CDKs) pour l'entrée et la progression dans le cycle cellulaire, développer des inhibiteurs pharmacologiques de ces enzymes constitue donc une voie thérapeutique potentielle majeure de lutte contre le cancer. De très nombreux dysfonctionnements des CDKs et de leurs régulateurs ont été décrits dans les tumeurs humaines. Des inhibiteurs de l'activité enzymatique des CDKs peuvent agir indépendamment ou conjointement avec d'autres traitements pour limiter la prolifération tumorale :
Les CDKs 1, 2, 4, 5 et 6 sont plus fréquemment suractivées ou anormalement régulées dans les tumeurs. Les inhibiteurs de CDKs s'avèrent alors de puissants agents antiprolifératifs stoppant les cellules en G1 ou G2/M.

Les inhibiteurs de CDKs peuvent également intervenir dans le processus apoptotique. Les cyclines A, B, D et E et les CDKs1 et 2 peuvent jouer un rôle pro-apoptotique. Les inhibiteurs de CDKs peuvent alors servir en chimiothérapie anticancéreuse pour potentialiser l'action de médicaments cytotoxiques, tout en assurant une protection des cellules saines.

La CDK5 est impliquée directement dans de nombreux processus de neurodégénérescence tels que la maladie d'Alzheimer, la maladie de Parkinson, les traumatismes crâniens ou les accidents vasculaires cérébraux. Les inhibiteurs de CDK5 agissent alors comme neuroprotecteurs.

Enfin, les CDKs semblent impliquées dans la polykystose rénale, et les processus d'inflammation. Les inhibiteurs de CDKs ont des effets très positifs sur des modèles animaux de ces pathologies.

La glycogène synthase kinase 3 (GSK-3) est une sérine/thréonine kinase identifiée à l'origine pour son rôle dans la régulation du métabolisme du glycogène. Outre qu'elle est impliquée dans la transduction indirecte de signaux d'insuline et d'IGF-1, elle est très présente dans le cerveau et un faisceau important de preuves s'est accumulé pour relier le GSK-3 à la neurotoxicité induite. Cela suggère que la dérégulation du GSK-3 pourrait jouer un rôle clé dans la pathogénèse de la maladie d'Alzheimer et, par conséquent, la GSK-3Beta est apparue comme une cible thérapeutique prometteuse pour la maladie d'Alzheimer et d'autres neurodégénérescences. Sur le plan chimique, les thiadiazolidinones (TDZD) hétérocycliques ont été les seules molécules proposées comme nouveaux médicaments pour le traitement efficace des troubles neurodégénératifs où la phosphorylation de la protéine tau joue un rôle clé comme dans le cas de la maladie d'Alzheimer.

La présente invention a pour but de fournir de nouveaux inhibiteurs des CDKs et de la GSK-3.

La présente invention a pour but de fournir de nouveaux inhibiteurs des CDKs ciblant directement et sélectivement lesdites kinases.

Plus particulièrement, la présente invention a pour but de fournir des inhibiteurs spécifiques des kinases CDK1, CDK5 et GSK3.

Ainsi, la présente invention concerne des composés de formule générale (I) suivante : 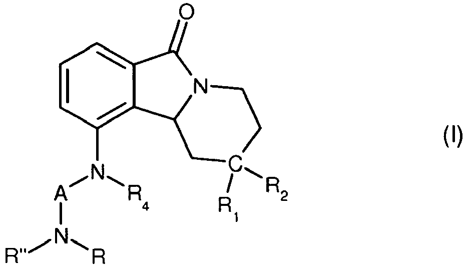 dans laquelle :
- A représente un groupe 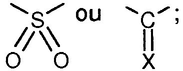 X représentant O ou S ;
- R" représente H ou un groupe alkyle comprenant de 1 à 10 atomes de carbone,
- R₁ représente H et R₂ représente un atome d'hydrogène, un groupe NR₃R'₃ ou un groupe OR₃, R₃ et R'₃ représentant, indépendamment l'un de l'autre, un atome d'hydrogène ou un groupe alkyle comprenant de 1 à 10 atomes de carbone ;
   ou R₁ et R₂ forment ensemble, avec l'atome de carbone sur lequel ils
   sont fixés, un groupe 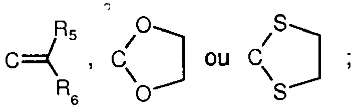 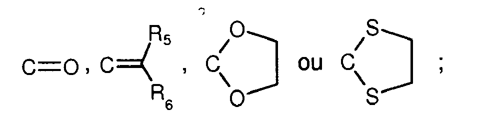 R₅ et R₆ représentant indépendamment l'un de l'autre un atome d'hydrogène ou un groupe alkyle comprenant de 1 à 10 atomes de carbone ;
- R₄ représente un atome d'hydrogène ou un groupe alkyle comprenant de 1 à 10 atomes de carbone ;
- R représente un groupe alkyle comprenant de 1 à 10 atomes de carbone, un groupe aryle comprenant de 6 à 30 atomes de carbone ou un groupe cycloalkyle comprenant de 3 à 20 atomes de carbone, lesdits groupes alkyle, aryle ou cycloalkyle susmentionnés comprenant éventuellement un ou plusieurs hétéroatomes, notamment O, S ou N, et pouvant le cas échéant être substitués,
   R représentant notamment un groupe alkyle éventuellement substitué par un groupe amine,
   ou lorsque A est un groupe -CO-, R et R₄ peuvent former ensemble, avec les atomes d'azote sur lesquels ils sont fixés un cycle de formule (II) suivante : 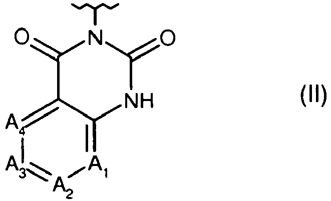 dans laquelle l'un des atomes parmi A₁, A₂, A₃ et A₄ représente N, et les trois autres atomes parmi A₁, A₂, A₃ et A₄ représentent CH,
ainsi que ses sels pharmaceutiquement acceptables,
ledit composé de formule (I) étant sous forme de stéréoisomère pur ou sous forme de mélange d'énantiomères et/ou de diastéréoisomères, y compris de mélanges racémiques.

Selon la présente invention, les radicaux "alkyle" représentent des radicaux hydrocarbonés saturés, en chaîne droite ou ramifiée, comprenant de 1 à 10 atomes de carbone, de préférence de 1 à 5 atomes de carbone (ils peuvent typiquement être représentés par la formule CₙH₂ₙ₊₁, n représentant le nombre d'atomes de carbone). On peut notamment citer, lorsqu'ils sont linéaires, les radicaux méthyle, éthyle, propyle, butyle, pentyle, hexyle, octyle, nonyle et décyle. On peut notamment citer, lorsqu'ils sont ramifiés ou substitués par un ou plusieurs radicaux alkyles, les radicaux isopropyle, tert-butyle, 2-éthylhexyle, 2-méthylbutyle, 2-méthylpentyle, 1-méthylpentyle et 3-méthylheptyle.

Le radical "cycloalkyle" est un radical hydrocarboné mono-, bi- ou tri- cyclique saturé ou partiellement insaturé, non aromatique, comprenant de 3 à 20 atomes de carbone, et de préférence de 3 à 10 atomes de carbone, tel que notamment le cyclopropyle, cyclopentyle, cyclohexyle ou adamantyle, ainsi que les cycles correspondants contenant une ou plusieurs insaturations.

Ainsi, dans le cadre de la présente invention, le terme "cycloalkyle" englobe également les radicaux "hétérocycloalkyles" désignant les systèmes mono ou bicycliques, saturés ou partiellement insaturés, non aromatiques, de 3 à 8 atomes de carbone, comprenant un ou plusieurs hétéroatomes choisis parmi N, O ou S.

Le terme "aryle" désigne un système aromatique hydrocarboné, mono ou bicyclique comprenant de 6 à 30, de préférence de 6 à 10, atomes de carbone. Parmi les radicaux aryle, on peut notamment citer le radical phényle ou naphtyle, plus particulièrement substitué par au moins un atome d'halogène.

Lorsque le radical aryle comprend au moins un hétéroatome, on parle de radical "hétéroaryle". Ainsi, le terme "hétéroaryle" désigne un système aromatique comprenant un ou plusieurs hétéroatomes choisis parmi l'azote, l'oxygène ou le soufre, mono ou bicyclique, comprenant de 5 à 30, et de préférence de 5 à 10, atomes de carbone. Parmi les radicaux hétéroaryles, on pourra citer le pyrazinyle, le thiényle, l'oxazolyle, le furazanyle, le pyrrolyle, le 1,2,4-thiadiazolyle, le naphthyridinyle, le pyridazinyle, le quinoxalinyle, le phtalazinyle, l'imidazo[1,2-a]pyridine, l'imidazo[2,1-b]thiazolyle, le cinnolinyle, le triazinyle, le benzofurazanyle, l'azaindolyle, le benzimidazolyle, le benzothiényle, le thiénopyridyle, le thiénopyrimidinyle, le pyrrolopyridyle, l'imidazopyridyle, le benzoazaindole, le 1,2,4-triazinyle, le benzothiazolyle, le furanyle, l'imidazolyle, l'indolyle, le triazolyle, le tétrazolyle, l'indolizinyle, l'isoxazolyle, l'isoquinolinyle, l'isothiazolyle, l'oxadiazolyle, le pyrazinyle, le pyridazinyle, le pyrazolyle, le pyridyle, le pyrimidinyle, le purinyle, le quinazolinyle, le quinolinyle, l'isoquinolyle, le 1,3,4-thiadiazolyle, le thiazolyle, le triazinyle, l'isothiazolyle, le carbazolyle, ainsi que les groupes correspondants issus de leur fusion ou de la fusion avec le noyau phényle.

Les radicaux "alkyle", "aryle" et "cycloalkyle" susmentionnés peuvent être substitués par un ou plusieurs substituants. Parmi ces substituants, on peut citer les groupes suivants : amino, hydroxy, thio, halogène, alkyle, alkoxy, alkylthio, alkylamino, aryloxy, arylalkoxy, cyano, trifluorométhyle, carboxy ou carboxyalkyle.

Les radicaux "alkoxy" selon la présente invention sont des radicaux de formule -O-alkyle, le groupe alkyle étant tel que défini précédemment.

Le terme "alkylthio" désigne un groupe -S-alkyl, le groupe alkyle étant tel que défini ci-dessus.

Le terme "alkylamino" désigne un groupe -NH-alkyl, le groupe alkyle étant tel que défini ci-dessus.

Parmi les atomes d'halogène, on cite plus particulièrement les atomes de fluor, de chlore, de brome et d'iode.

Le terme "aryloxy" désigne un groupe -O-aryle, le groupe aryle étant tel que défini ci-dessus.

Le terme "arylalkoxy" désigne un groupe aryl-alkoxy-, les groupes aryles et alkoxy étant tels que définis ci-dessus.

Le terme "carboxyalkyle" désigne un groupe HOOC-alkyl-, le groupe alkyle étant tel que défini ci-dessus. Comme exemple de groupes carboxyalkyles, on peut citer notamment le carboxyméthyle ou le carboxyéthyle.

Lorsqu'un radical alkyle est substitué par un groupe aryle, on parle de radical "arylalkyle" ou "aralkyle". Les radicaux « arylalkyles » ou « aralkyles » sont des radicaux aryl-alkyl-, les groupes aryles et alkyles étant tels que définis ci-dessus. Parmi les radicaux arylalkyles, on peut notamment citer le radical benzyle ou phénéthyle.

L'expression "sels pharmaceutiquement acceptables" fait référence aux sels d'addition acide relativement non toxiques, inorganiques et organiques, et les sels d'addition de base, des composés de la présente invention. Ces sels peuvent être préparés *in situ* pendant l'isolement final et la purification des composés. En particulier, les sels d'addition acide peuvent être préparés en faisant réagir séparément le composé purifié sous sa forme épurée avec un acide organique ou inorganique et en isolant le sel ainsi formé. Parmi les exemples de sels d'addition acide, on trouve les sels bromhydrate, chlorhydrate, sulfate, bisulfate, phosphate, nitrate, acétate, oxalate, valérate, oléate, palmitate, stéarate, laurate, borate, benzoate, lactate, phosphate, tosylate, citrate, maléate, fumarate, succinate, tartrate, naphthylate, mésylate, glucoheptanate, lactobionate, sulfamates, malonates, salicylates, propionates, méthylènebis-b-hydroxynaphtoates, acide gentisique, iséthionates, di-p-toluoyltartrates, méthanesulfonates, éthanesulfonates, benzènesulfonates, p-toluènesulfonates, cyclohexylsulfamates et quinateslaurylsulfonate, et analogues (Voir par exemple S.M. Berge et al. « Pharmaceutical Salts » J. Pharm. Sci, 66 :p.1-19 (1977)). Les sels d'addition acide peuvent également être préparés en faisant réagir séparément le composé purifié sous sa forme acide avec une base organique ou inorganique et en isolant le sel ainsi formé. Les sels d'addition acide comprennent les sels aminés et métalliques. Les sels métalliques adaptés comprennent les sels de sodium, potassium, calcium, baryum, zinc, magnésium et aluminium. Les sels de sodium et de potassium sont préférés. Les sels d'addition inorganiques de base adaptés sont préparés à partir de bases métalliques qui comprennent hydrure de sodium, hydroxyde de sodium, hydroxyde de potassium, hydroxyde de calcium, hydroxyde d'aluminium, hydroxyde de lithium, hydroxyde de magnésium, hydroxyde de zinc. Les sels d'addition aminés de base adaptés sont préparés à partir d'amines qui ont une alcalinité suffisante pour former un sel stable, et de préférence comprennent les amines qui sont souvent utilisées en chimie médicinale en raison de leur faible toxicité et de leur acceptabilité pour l'usage médical: ammoniaque, éthylènediamine, N-méthyl-glucamine, lysine, arginine, ornithine, choline, N,N'-dibenzylethylenediamine, chloroprocaïne, diéthanolamine, procaïne, N-benzyl-phénéthylamine, diéthylamine, pipérazine, tris(hydroxyméthyl)-aminométhane, hydroxyde de tétraméthyl-ammonium, triéthylamine, dibenzylamine, éphénamine, dehydroabiétylamine, N-éthylpipéridine, benzylamine, tétra-méthylammonium, tétraéthylammonium, méthylamine, diméthylamine, triméthyl-amine, éthylamine, acides aminés de base, par exemple lysine et arginine, et dicyclohexylamine, et analogues.

L'invention se rapporte également aux formes tautomères, aux énantiomères, diastéréoisomères, épimères et aux sels organiques ou minéraux des composés de formule générale (I).

De préférence, dans la formule (I), le groupe R" est H.

De préférence, dans la formule (I), le groupe R₁ est H et R₂ est H ou est choisi dans le groupe constitué des groupes CH(CH₃)₂, NH₂, OH et OMe.

De préférence, lorsque R₁ et R₂ forment ensemble, avec l'atome de carbone sur lequel ils sont fixés, un groupe 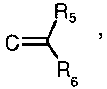 R₅ et R₆ représentent un groupe méthyle.

Une famille préférée de composés de l'invention est constituée de composés de formule (I) dans laquelle R représente un groupe aryle choisi dans le groupe constitué des groupes phényle, benzyle, pyridinyle, pyrimidyle, pyrazinyle, triazinyle, pyrazolyle, isoxazolyle, thiazolyle, benzothiazothiazolyle et quinolinyle, le cas échéant substitués.

Une autre famille préférée de composés de l'invention est constituée de composés de formule (I) dans laquelle R représente un groupe cyclohexyle ou pipéridinyle, le cas échéant substitués.

Selon un mode de réalisation préféré, les composés de l'invention sont des composés de formule (I) telle que définie ci-dessus, dans laquelle R est choisi parmi l'un des groupes suivants : 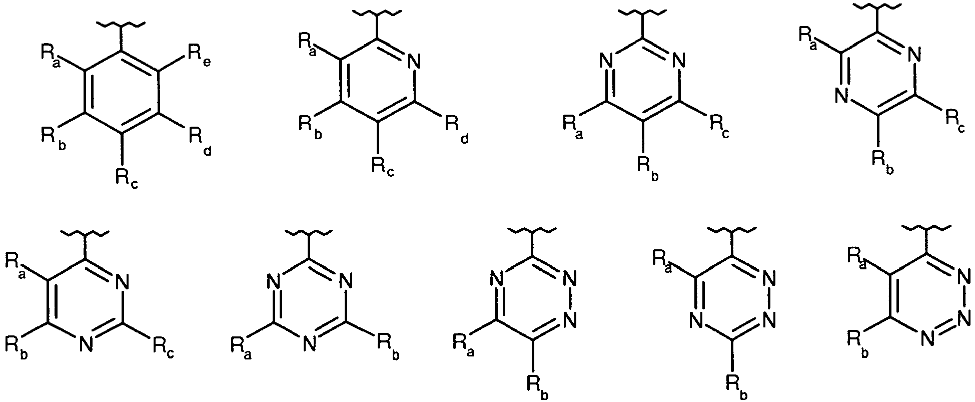 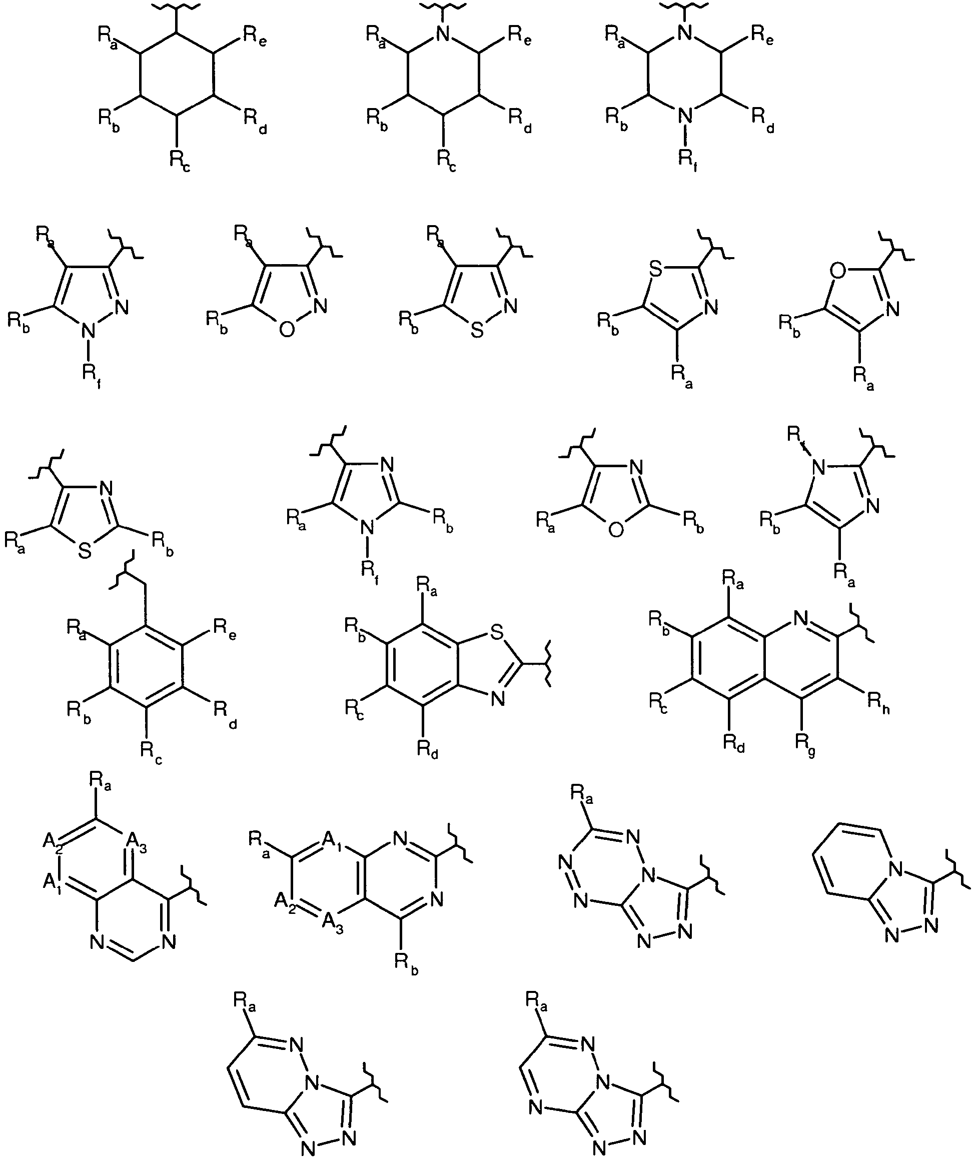 les groupes Rₐ, R_{b}, R_{c}, R_{d}, Rₑ, Rg et Rₕ étant choisis, indépendamment les uns des autres, dans le groupe constitué des substituants suivants :
- un atome d'hydrogène,
- un atome d'halogène, notamment Br, Cl ou F,
- un groupe alkyle comprenant de 1 à 10 atomes de carbone, et étant de préférence un groupe méthyle,
   ledit groupe alkyle étant éventuellement substitué notamment par un ou plusieurs substituants choisis dans le groupe constitué des substituants suivants :
   - atomes d'halogène,
   - groupes alcényles ou alcynyles comprenant de 2 à 10 atomes de carbone,
   - groupes aryles comprenant de 6 à 30 atomes de carbone,
   - groupes CHO, COR_{α}, COOR_{α}, SR_{α}, OR_{α} ou NR_{α}R_{β}, R_{α} et R_{β} représentant indépendamment l'un de l'autre un atome d'hydrogène, un groupe alkyle comprenant de 1 à 10 atomes de carbone, ou un groupe aryle comprenant de 6 à 30 atomes de carbone,
- un groupe -CHO,
- un groupe -CN,
- un groupe phényle,
- un groupe -SR_{α} ou Ordo, R_{α} étant tel que défini ci-dessus, notamment un groupe -OH, -OCH₃, -SH, -SCH₃, -O-CH(CH₃)₂, -O-CH₂-CH₂-CH₃,
- un groupe -NR_{α}R_{β}, R_{α} et R_{β} étant tels que définis ci-dessus, notamment un groupe NH₂.
- un groupe -COOR_{α}, R_{α} étant tel que défini ci-dessus, notamment -COOMe,
- un groupe -CONR_{α}R_{β}, R_{α} et R_{β} étant tels que définis ci-dessus, notamment - CONH₂,
- un groupe -NHCOR_{α}, R_{α} étant tel que défini ci-dessus, et
- un groupe 2-pyridinyle.
l'un des atomes parmi A₁, A₂ et A₃ représentant N, et les deux autres atomes parmi A₁, A₂ et A₃ représentant CH,
le groupe R_{f} étant un atome d'hydrogène, un groupe alkyle comprenant de 1 à 10 atomes de carbone, ou un groupe -COOR_{α}, R_{α} étant tel que défini ci-dessus, et notamment -COOtBu.

Les radicaux "alcényles" représentent des radicaux hydrocarbonés, en chaîne droite ou linéaire, et comprennent une ou plusieurs insaturations éthyléniques. Lorsqu'ils comprennent une seule double liaison ils peuvent typiquement être représentés par la formule CₙH₂ₙ, n représentant le nombre d'atomes de carbone. Parmi les radicaux alcényles, on peut notamment citer les radicaux allyle ou vinyle.

Les radicaux "alcynyles" représentent des radicaux hydrocarbonés, en chaîne droite ou linéaire, et comprennent une ou plusieurs insaturations acétyléniques. Lorsqu'ils comprennent une seule triple liaison ils peuvent typiquement être représentés par la formule CₙH₂ₙ₋₂, n représentant le nombre d'atomes de carbone. Parmi les radicaux alcynyles, on peut notamment citer l'acétylène.

Les groupes R préférés sont les suivants : 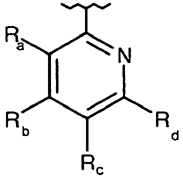 Rₐ, R_{b}, R_{c} et R_{d}, étant choisis, indépendamment les uns des autres, dans le groupe constitué des substituants suivants : un atome d'hydrogène, un atome d'halogène, notamment Br, Cl ou F, et un groupe alkyle comprenant de 1 à 10 atomes de carbone, et étant de préférence un groupe méthyle.

Parmi ceux-ci, on peut notamment citer les groupes suivants :

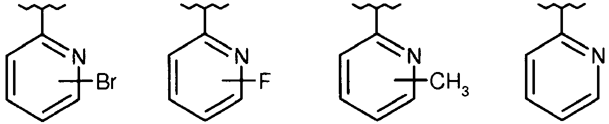

D'autres groupes R préférés sont les suivants : 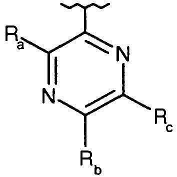 Rₐ, R_{b} et R_{c} étant choisis, indépendamment les uns des autres, dans le groupe constitué des substituants suivants : un atome d'hydrogène, un atome d'halogène, notamment Br, Cl ou F, et un groupe alkyle comprenant de 1 à 10 atomes de carbone, et étant de préférence un groupe méthyle.

Parmi ceux-ci, on peut notamment citer le groupe suivant :

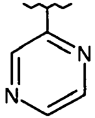

Selon un autre mode de réalisation préféré, le groupe R répond à la formule suivante : 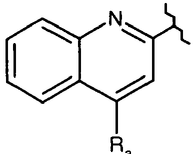 Rₐ étant H ou un groupe -OR_{α}, R_{α} étant tel que défini ci-dessus, et de préférence étant H ou Me.

Parmi les composés de l'invention, on peut citer les composés de formule générale (I-1) suivante : 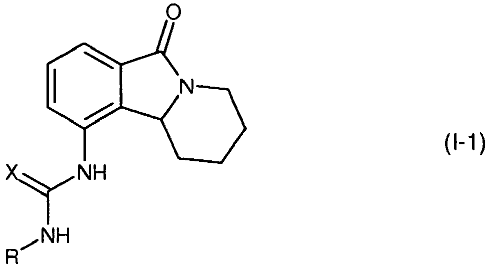 R et X étant tels que définis ci-dessus.

Les composés de formule (I-1) sont des composés de formule (I) dans laquelle R₁, R₂, R" et R₄ sont H et A est CX.

Parmi les composés de formule (I-1), on peut notamment citer les composés dans lesquels R est choisi parmi l'un des groupes suivants :

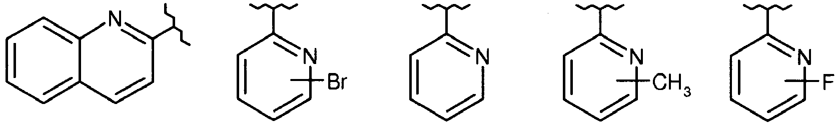

Parmi les composés de l'invention, on peut également citer les composés de formule générale (I-2) suivante : 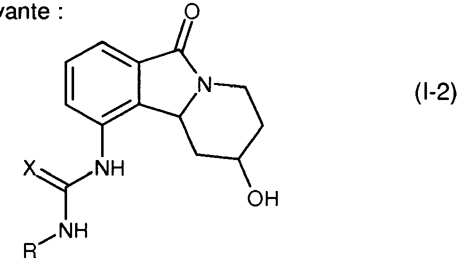 R et X étant tels que définis ci-dessus.

Les composés de formule (I-2) sont des composés de formule (I) dans laquelle R₁, R" et R₄ sont H, A est CX et R₂ est OH.

Parmi les composés de formule (I-2), on peut notamment citer les composés dans lesquels R est

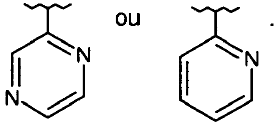

Parmi les composés de l'invention, on peut également citer les composés de formule générale (I-3) suivante : 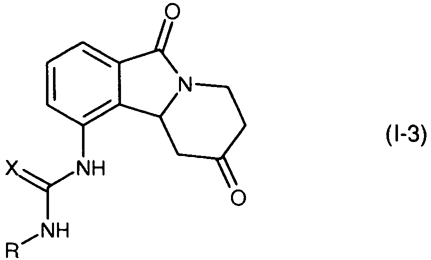 R et X étant tels que définis ci-dessus.

Les composés de formule (I-3) sont des composés de formule (I) dans laquelle R" et R₄ sont H, A est CX et R₁ et R₂ forment ensemble, avec l'atome de carbone sur lequel ils sont fixés, un groupe C=O.

Parmi les composés de formule (I-3), on peut notamment citer les composés dans lesquels R est

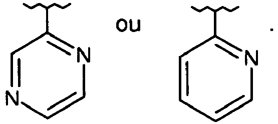

Parmi les composés de l'invention, on peut également citer les composés de formule générale (I-4) suivante : 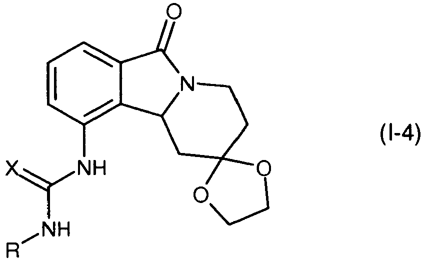 R et X étant tels que définis ci-dessus.

Les composés de formule (I-4) sont des composés de formule (I) dans laquelle R" et R₄ sont H, A est CX et R₁ et R₂ forment ensemble, avec l'atome de carbone sur lequel ils sont fixés, un groupe 1,3-dioxolane.

Parmi les composés de formule (I-4), on peut notamment citer les composés dans lesquels R est

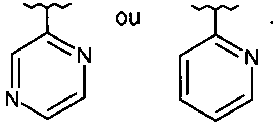

Parmi les composés de l'invention, on peut également citer les composés de formule générale (I-5) suivante : 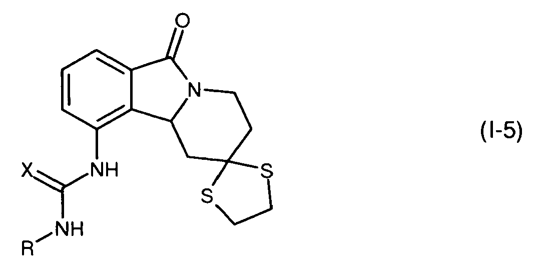 R et X étant tels que définis ci-dessus.

Les composés de formule (I-5) sont des composés de formule (I) dans laquelle R" et R₄ sont H, A est CX et R₁ et R₂ forment ensemble, avec l'atome de carbone sur lequel ils sont fixés, un groupe 1,3-dithiolane.

Parmi les composés de formule (I-5), on peut notamment citer les composés dans lesquels R est

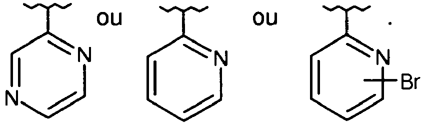

Chaque famille de composés répondant respectivement aux formules (I-1), (I-2), (I-3), (I-4) et (I-5) comprend une sous-famille de composés où X est un atome d'oxygène.

Ainsi, la présente invention concerne les composés répondant respectivement aux formules (I-1'), (I-2'), (I-3'), (I-4') et (I-5') suivantes : 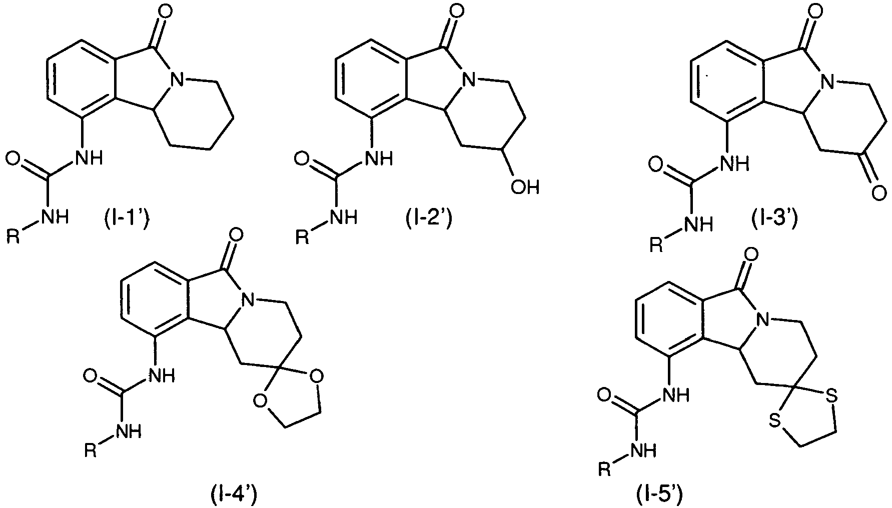 R étant tel que défini ci-dessus.

Une autre famille particulière de composés de l'invention est constituée des composés répondant à la formule générale (I-1-a) suivante :

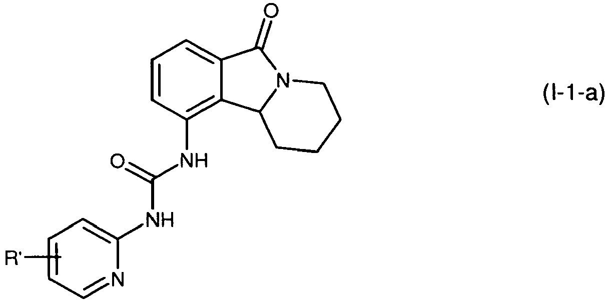

R' représentant un atome d'hydrogène, un atome d'halogène tel que Br ou F ou un groupe alkyle comprenant de 1 à 10 atomes de carbone, de préférence un groupe méthyle.

Ces composés sont des composés de formule (I-1) telle que définie ci-dessus, dans laquelle X est O et R est un groupe 2-pyridinyle (éventuellement substitué par un groupe R').

Parmi les composés de formule (I-1-a), on peut notamment citer les composés suivants :

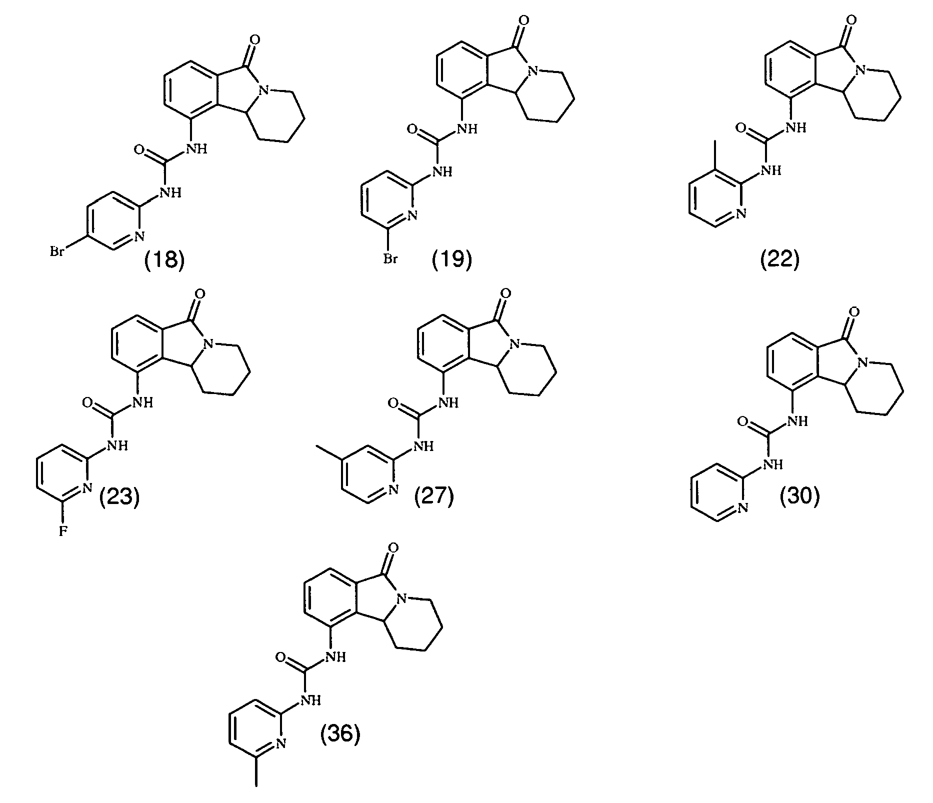

Une autre famille particulière de composés de l'invention est constituée des composés répondant à la formule générale (I-1-b) suivante :

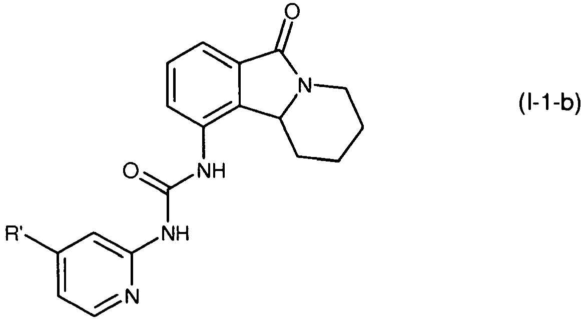

R' étant choisi dans le groupe constitué des groupes alkyle, aminoalkyle, amide (-CONR_{α}R_{β}, R_{α} et R_{α} étant tels que définis ci-dessus), alkoxy, notamment méthoxy ou éthoxy, CHO, CH₂CHO et (CH₂)ₙNR_{γ}R_{δ}, n étant égal à 0 ou représentant un nombre entier compris de 1 à 5, de préférence n étant égal à 0 ou 1, et R_{γ} et R_{δ} représentant indépendamment l'un de l'autre un atome d'hydrogène, un groupe alkyle comprenant de 1 à 10 atomes de carbone, ou un groupe aryle comprenant de 6 à 30 atomes de carbone, ou R_{γ} et R_{δ} formant avec l'atome d'azote auquel ils sont liés un (hétéro)cycloalkyle, lesdits groupes alkyle, aryle et (hétéro)cycloalkyle étant le cas échéant substitués.

Ces composés sont des composés de formule (I-1) telle que définie ci-dessus, dans laquelle X est O et R est un groupe 2-pyridinyle (substitué par un groupe R').

Une autre famille particulière de composés de l'invention est constituée des composés répondant à la formule générale (I-5-a) suivante :

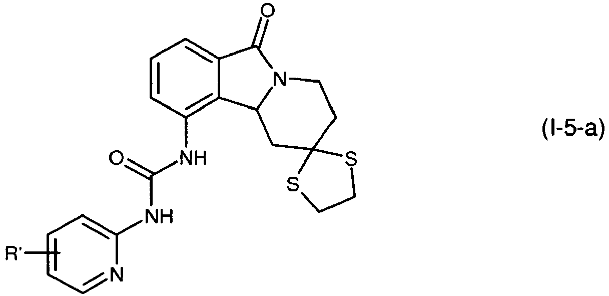

R' représentant un atome d'hydrogène, un atome d'halogène tel que Br ou F ou un groupe alkyle comprenant de 1 à 10 atomes de carbone, de préférence un groupe méthyle.

Ces composés sont des composés de formule (I-5) telle que définie ci-dessus, dans laquelle X est O et R est un groupe 2-pyridinyle (éventuellement substitué par un groupe R').

Parmi les composés de formule (I-5), on peut notamment citer les composés suivants :

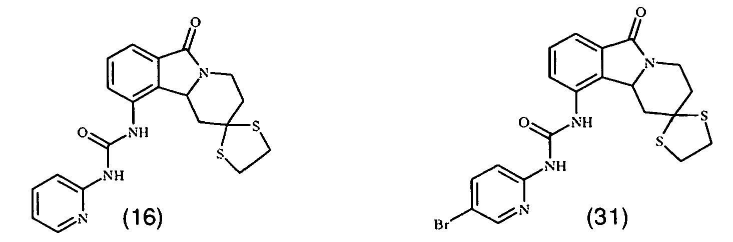

Une autre famille particulière de composés de l'invention est constituée des composés répondant à la formule générale (I-6) suivante : 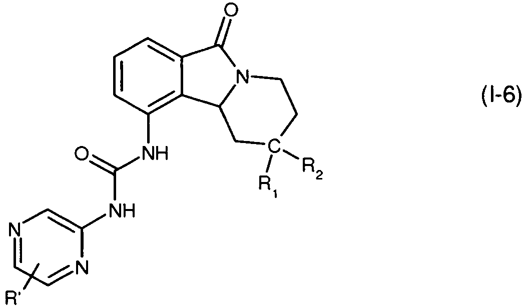 R', R₁ et R₂ étant tels que définis ci-dessus,
et de préférence R' représentant un atome d'hydrogène ou un groupe alkyle comprenant de 1 à 10 atomes de carbone, de préférence un groupe méthyle.

Les composés de formule (I-6) sont des composés de formule (I) dans laquelle R" et R₄ sont H, A est CO et R est un groupe 2-pyrazinyle (éventuellement substitué par un groupe R').

Parmi les composés de formule (I-6), on peut notamment citer les composés suivants :

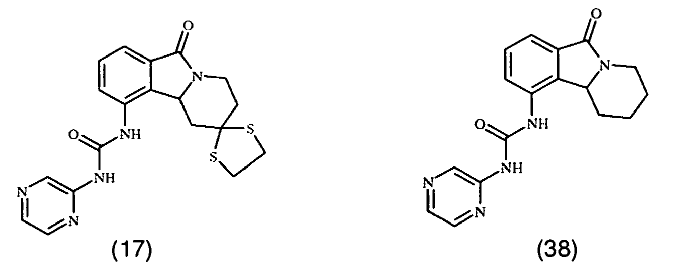

Une autre famille particulière de composés de l'invention est constituée des composés répondant à la formule générale (I-6-a) suivante :

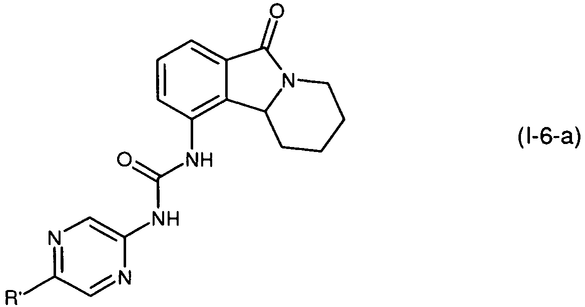

R' étant choisi dans le groupe constitué des groupes alkyle, aminoalkyle, amide (-CONR_{α}R_{β}, R_{α} et R_{β} étant tels que définis ci-dessus), alkoxy, notamment méthoxy ou éthoxy, CHO, CH₂CHO et (CH₂)ₙNR_{γ}R_{δ}, n étant égal à 0 ou représentant un nombre entier compris de 1 à 5, de préférence n étant égal à 0 ou 1, et R_{γ} et R_{δ} représentant indépendamment l'un de l'autre un atome d'hydrogène, un groupe alkyle comprenant de 1 à 10 atomes de carbone, ou un groupe aryle comprenant de 6 à 30 atomes de carbone, ou R_{γ} et R_{δ} formant avec l'atome d'azote auquel ils sont liés un (hétéro)cycloalkyle, lesdits groupes alkyle, aryle et (hétéro)cycloalkyle étant le cas échéant substitués.

Les composés de formule (I-6-a) sont des composés de formule (I-6) dans laquelle R₁ et R₂ sont H.

Une autre famille particulière de composés de l'invention est constituée des composés répondant à la formule générale (I-7) suivante : 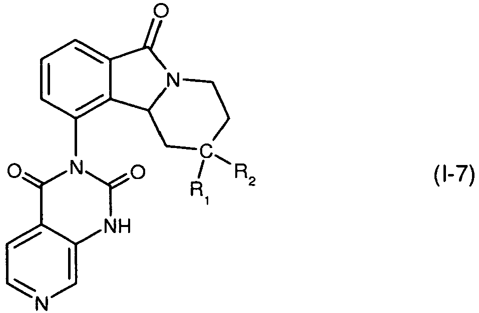 R₁ et R₂ étant tels que définis ci-dessus.

Les composés de formule (I-7) sont des composés de formule (I) dans laquelle A est un groupe -CO- et R et R₄ forment ensemble, avec les atomes d'azote sur lesquels ils sont fixés un cycle de formule (II) telle que définie ci-dessus, dans laquelle A₁, A₃ et A₄ sont H et A₂ est N.

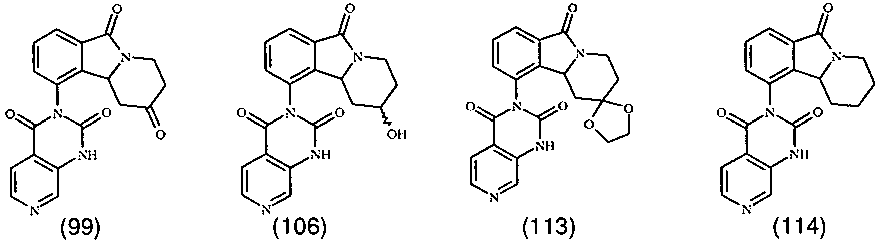

La présente invention concerne également les composés de formule (I-8) suivante : 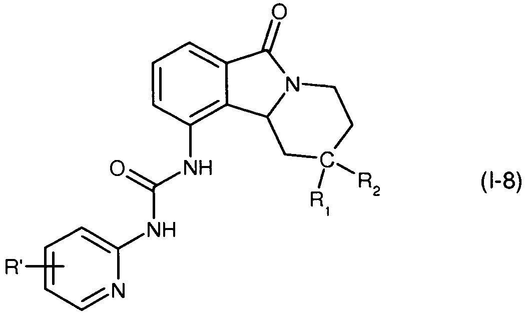 R', R₁ et R₂ étant tels que défini ci-dessus.

La présente invention concerne également une composition pharmaceutique comprenant un composé de formule (I) telle que définie ci-dessus, ou tout composé tel que mentionné ci-dessus, en association avec un vecteur pharmaceutiquement acceptable.

La présente invention concerne donc un composé tel que défini ci-dessus de formule (I) pour son utilisation comme médicament.

Les composés pharmaceutiques selon l'invention peuvent être présentés sous des formes destinées à l'administration par voie parentérale, orale, rectale, permuqueuse ou percutanée.

Les compositions pharmaceutiques incluant ces composés de formule générale (I) seront donc présentées sous forme de solutés ou de suspensions injectables ou flacons multi-doses, sous forme de comprimés nus ou enrobés, de dragées, de capsules, de gélules, de pilules, de cachets, de poudres, de suppositoires ou de capsules rectales, de solutions ou de suspensions, pour l'usage percutané dans un solvant polaire, pour l'usage permuqueux.

Les excipients qui conviennent pour de telles administrations sont les dérivés de la cellulose ou de la cellulose microcristalline, les carbonates alcalinoterreux, le phosphate de magnésium, les amidons, les amidons modifiés, le lactose pour les formes solides.

Pour l'usage rectal, le beurre de cacao ou les stéarates de polyéthylèneglycol sont les excipients préférés.

Pour l'usage parentéral, l'eau, les solutés aqueux, le sérum physiologique, les solutés isotoniques sont les véhicules les plus commodément utilisés.

La posologie peut varier dans les limites importantes (0,5 mg à 1000 mg) en fonction de l'indication thérapeutique et de la voie d'administration, ainsi que de l'âge et du poids du sujet.

La présente invention concerne les composés de l'invention tels que définis ci-dessus, pour leur utilisation en tant qu'inhibiteur des kinases CDK1, CDK5 et/ou GSK3.

Ainsi, la présente invention concerne les composés de l'invention tels que définis ci-dessus, pour leur utilisation dans le cadre du traitement ou de la prévention de maladies liées à une dérégulation des kinases CDK1, CDK5 et/ou GSK3.

Plus particulièrement, lesdites maladies sont choisies dans le groupe constitué des cancers, de la maladie d'Alzheimer, de la maladie de Parkinson, des traumatismes crâniens, des accidents vasculaires cérébraux, de la polykystose rénale, de la sclérose amyotrophique latérale, des infections virales, des maladies autoimmunes, des désordres neurodégénératifs, du psoriasis, de l'asthme, des dermatites atopiques et des glomérulonéphrites.

La présente invention concerne également l'utilisation des composés de l'invention tels que définis ci-dessus, pour la préparation d'un médicament destiné au traitement ou à la prévention de maladies liées à une dérégulation des kinases CDK1, CDK5 et/ou GSK3, et plus particulièrement au traitement et à la prévention des maladies susmentionnées.

La présente invention concerne également le procédé de préparation des composés de formule générale (I) telle que définie ci-dessus, dans laquelle R₄ et R" sont H et A est un groupe CO, et R₁ et R₂ sont H ou forment ensemble, avec l'atome de carbone sur lequel ils sont fixés, un groupe 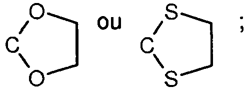 ledit procédé comprenant :
une étape de réaction de l'amine de formule suivante : 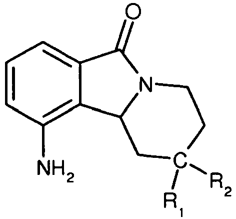 avec un isocyanate de formule RNCO, notamment en présence de dioxane à 100°C pendant 24 heures, R étant tel que défini ci-dessus pour la formule (I).

La présente invention concerne également le procédé de préparation des composés de formule générale (I) telle que définie ci-dessus, dans laquelle R₄ et R" sont H et A est un groupe CS, et R₁ et R₂ sont H ou forment ensemble, avec l'atome de carbone sur lequel ils sont fixés, un groupe 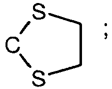 ledit procédé comprenant :
une étape de réaction de l'isothiocyanate de formule suivante : 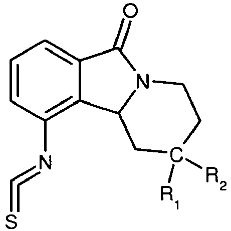
avec une amine de formule RNH₂, notamment en présence de dioxane à 100°C pendant 24 heures, R étant tel que défini ci-dessus pour la formule (I).

La présente invention concerne également le procédé de préparation des composés de formule générale (I) telle que définie ci-dessus, dans laquelle R₄ et R" sont H et A est un groupe CO, et R₁ et R₂ sont H ou forment ensemble, avec l'atome de carbone sur lequel ils sont fixés, un groupe 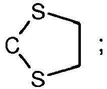 ledit procédé comprenant :
une étape de réaction de la thiourée de formule suivante : 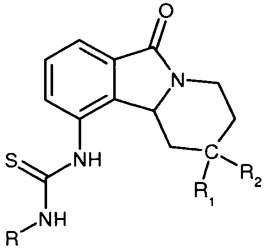 R étant tel que défini ci-dessus pour la formule (I),
en présence d'un mélange CH₃CN/eau et de HgO à température ambiante pendant une durée comprise de 24 à 40 heures.

La présente invention concerne également le procédé de préparation des composés de formule générale (I) telle que définie ci-dessus, dans laquelle R₄ et R" sont H et A est un groupe CO, et R₁ et R₂ sont H ou forment ensemble, avec l'atome de carbone sur lequel ils sont fixés, un groupe 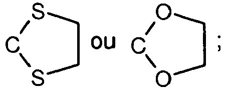 ledit procédé comprenant :
une étape de réaction de l'amine de formule suivante : 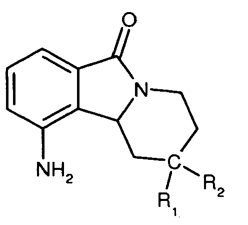 avec une amine de formule RNH₂ et du triphosgène, notamment en présence de iPr₂NEt dans du dichlorométhane à température ambiante pendant 1 heure, R étant tel que défini ci-dessus pour la formule (I).

La présente invention concerne également le procédé de préparation des composés de formule générale (I) telle que définie ci-dessus, dans laquelle R₁ et R₂ forment ensemble, avec l'atome de carbone sur lequel ils sont fixés, un groupe C=O ; ledit procédé comprenant :
une étape de réaction de l'acétal de formule suivante : 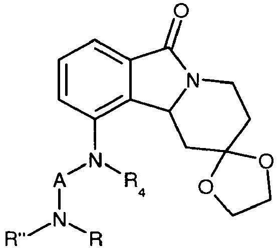 A, R, R" et R₄ étant tels que définis ci-dessus dans la formule (I),
avec de l'acétone et de l'acide chlorhydrique, l'ensemble étant notamment porté à reflux pendant 3 heures.

La présente invention concerne également le procédé de préparation des composés de formule générale (I) telle que définie ci-dessus, dans laquelle R₁ est H et R₂ est OH,
ledit procédé comprenant :
une étape de réduction de la cétone de formule suivante : 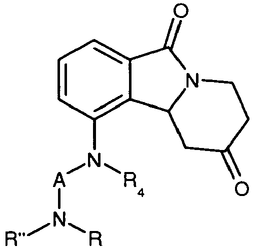 A, R, R" et R₄ étant tels que définis ci-dessus pour la formule (I),
de préférence avec NaBH₄ en présence de THF et de méthanol, l'ensemble étant notamment agité pendant 2 heures à une température comprise de 0°C à 5°C.

La présente invention concerne également le procédé de préparation des composés de formule générale (I-7) telle que définie ci-dessus, dans laquelle R₁ et R₂ sont H ou R₁ et R₂ forment ensemble, avec l'atome de carbone sur lequel ils sont fixés, un groupe 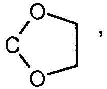 ledit procédé comprenant :
une étape de réaction de l'amine de formule suivante : 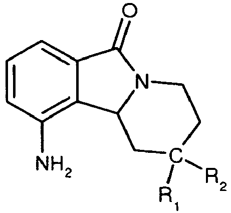 R₁ et R₂ étant tels que définis ci-dessus, avec un isocyanate de formule (a2) 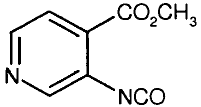
notamment dans du toluène, ladite amine étant par exemple dans de la pyridine, l'ensemble réactionnel étant agité à reflux pendant 24 heures.

La présente invention concerne également les composés intermédiaires (utilisés pour la préparation des composés de formule (I) selon l'invention) répondant à l'une des formules suivantes :

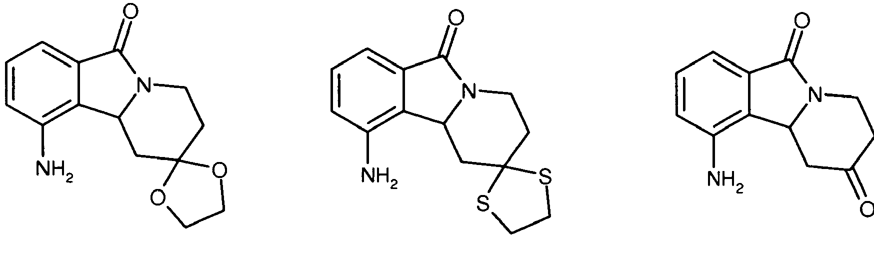

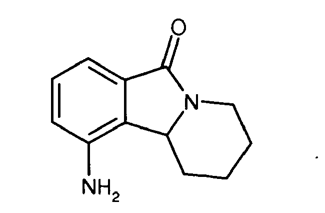

### PARTIE EXPÉRIMENTALE

### PRÉPARATION DES COMPOSÉS DE L'INVENTION

### 1. Préparation des intermédiaires de synthèse

Les procédés de préparation des composés de l'invention sont effectués à partir d'intermédiaires de synthèse (1)-(15, préparés selon le schéma suivant :

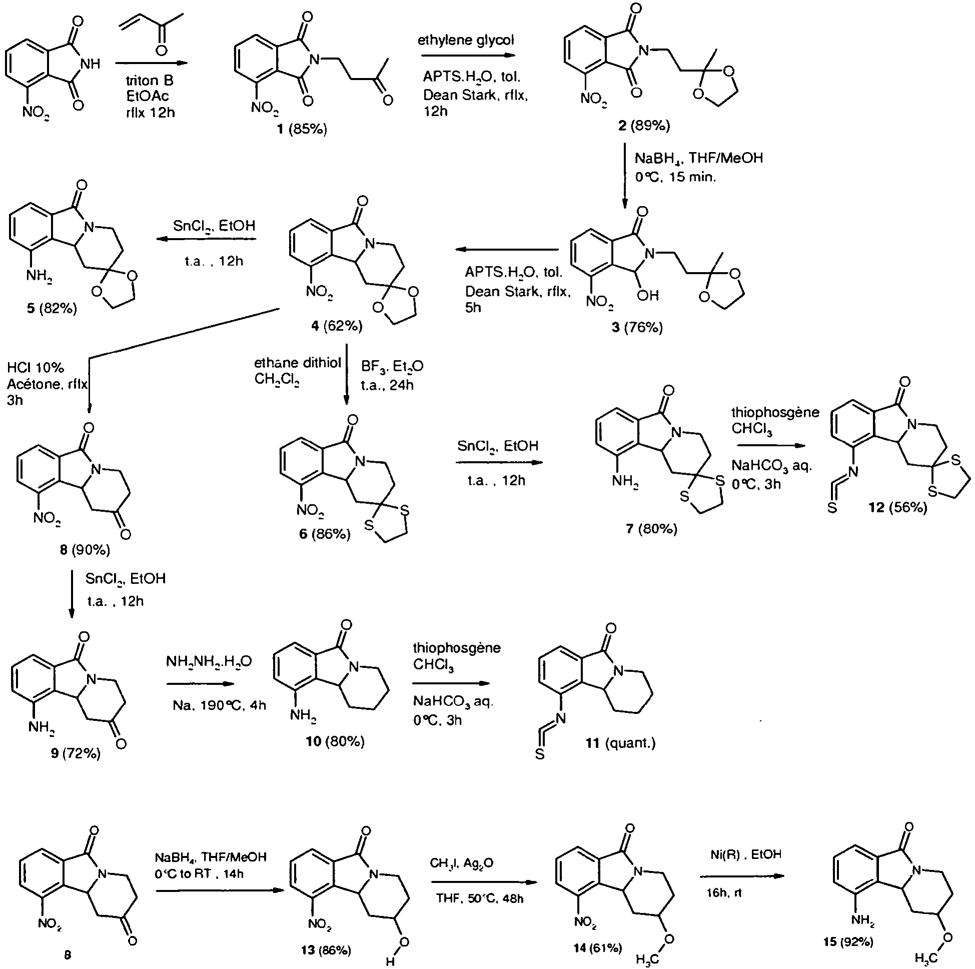

### 4-Nitro-2-(3-oxobutyl)isoindoline-1,3-dione (1).

A une solution contenant le 3-nitrophtalimide (5,00 g ; 26 mmol) dans de l'acétate d'éthyle (30 mL), la méthylvinylcétone (2,8 mL ; 33mmol ; 1,25 éq) est ajoutée goutte à goutte. Après quelques minutes d'agitation, 760 µL de triton B (hydroxyde de benzyltriméthylammonium, 40% dans l'eau) sont additionnés. Le milieu réactionnel est porté au reflux pendant 12 h. Après refroidissement, les solvants sont éliminés sous pression réduite et le résidu solide est recristallisé dans l'éthanol absolu pour donner le composé 1 sous forme de solide jaune avec un rendement de 85 %. F : 122 °C ; IR (ATR-Ge ν cm⁻¹) : 1715 (C=O cétone), 1538 (C=C arom), 1373 (N=O₂), 1127 (C-C); RMN ¹H (CDCl₃ 250 MHz) δ8.19 - 8.07 (m, 2H), 7.93 (dd, J = 7.1 Hz, J = 8.5 Hz, 1H), 3.98 (t, J = 7.3 Hz, 2H), 2.91 (t, J = 7.3 Hz, 2H), 2.20 (s, 3H) ; RMN ¹³C (CDCl₃ ; 63 MHz) δ205.7 (Cq), 165.6 (Cq), 162.9 (Cq), 145.2 (Cq), 135.6 (CH), 134.2 (Cq), 128.7 (CH), 127.2 (CH), 123.9 (Cq), 41.2 (CH₂), 33.8 (CH₂), 30.1 (CH₃). **SM** (IS) m/z : 263.0 [M+H]⁺

### 2-(2-(2-Méthyl-1,3-dioxolan-2-yl)ethyl)-4-nitroisoindoline-1,3-dione (2).

A une solution contenant le composé 1 (5.0 g, 19.0 mmol) dans du toluène (60 mL) sont ajoutés successivement l'éthylène glycol (2.0 mL, 38.0 mmol, 2.0 éq.) et de l'APTS H₂O (21 mg ; 0.1 mmol, cat.). Le milieu réactionnel est porté au reflux pendant une nuit en utilisant un appareil de Dean-Stark. Le mélange réactionnel refroidi est lavé avec une solution saturée de NaHCO₃ (20 mL) puis avec une solution saturée de NaCl (20 mL). L'extrait organique est séché sur Mg₂SO₄ puis les solvants sont évaporés. Le solide est recristallisé dans l'éthanol pour donner le composé 2 sous forme de solide jaune avec un rendement de 89%. **F *:*** 90 °C ; **IR** (ATR-Ge ν cm⁻¹) : 1711 (C=O amide), 1542 (C=C arom), 1366 (N=O₂), 1158 (C-O) ; RMN ¹H (CDCl₃ ; 250 MHz) δ 8.17 - 8.05 (m, 2H), 7.90 (dd, J = 7.4 Hz, J = 8.2 Hz, 1H), 4.00 - 3.90 (m, 4H), 3.86 (t, J = 7.0 Hz, 2H), 2.10 (t, J = 7.0 Hz, 2H), 1.36 (s, 3H) ; RMN ¹³C (CDCl₃ ; 63 MHz) δ 165.9 (Cq), 163.0 (Cq), 145.2 (Cq), 135.3 (CH), 134.4 (Cq), 128.5 (CH), 127.0 (CH), 124.1 (Cq), 108.8 (Cq), 64.8 (2 x CH₂), 36.1 (CH₂), 34.3 (CH₂), 23.9 (CH₃). **SM** (IS) m/z : 307.5 [M+H]⁺, 324.0 [M+NH₄]⁺

### 3-Hydroxy-2-(2-(2-méthyl-1,3-dioxolan-2-yl)éthyl)-4-nitroisoindolin-1-one (3).

Une solution contenant le composé 2 (2.0 g, 6.5 mmol) dissous dans un mélange THF / MeOH 1 / 2 (15 / 30 mL) est placée à -20°C sous agitation. 700 mg de NaBH₄ (19.5 mmol, 3.0 eq.) sont ajoutés par portions puis le milieu réactionnel est agité à cette température pendant 15 min. La température est remontée à 0°C puis une solution aqueuse de NaOH 1M (40 mL) est introduite. Le mélange est amené à température ambiante, les volatils sont évaporés sous pression réduite. La phase aqueuse résiduelle est extraite au dichlorométhane (3 x 20 mL). Les extraits organiques rassemblés sont séchés sur MgSO₄. filtrés et évaporés sous pression réduite. Une chromatographie sur gel de silice flash (dichlorométhane / méthanol 99 / 1) donne le produit **3** sous forme de solide blanc avec un rendement de 76%. **F :** 130 °C **IR** (ATR-Ge ν cm⁻¹) : 3293 (O-H), 1687 (C=O amide), 1540 (C=C arom), 1348 (N=O₂), 1054 (C-O) ; RMN ¹H (CDCl₃ ; 250 MHz) δ 8.32 (dd, J = 0.9 Hz, J = 8.2 Hz, 1H), 8.09 (dd, J = 0.7 Hz, J = 7.4 Hz, 1H), 7.77 - 7.67 (m, 1H), 6.46 (d, J = 5.4 Hz, 1H), 4.23 (d, J = 5.4 Hz, 1H), 3.95 (m, 4H), 3.86 (m, 1H), 3.75 - 3.59 (m, 1H), 2.21 - 2.05 (m, 2H), 1.39 (s, 3H) ; RMN ¹³C (CDCl₃ ; 63 MHz) δ 164.8 (Cq), 143.8 (Cq), 138.9 (Cq), 135.3 (Cq), 131.5 (CH), 129.6 (CH), 127.2 (CH), 109.1 (Cq), 81.4 (CH), 64.8 (CH₂), 64.8 (CH₂), 36.9 (CH₂), 35.8 (CH₂), 24.0 (CH₃). **SM** (IS) m/z : 309.0 [M+H]⁺, 326.0 [M+NH₄]+

### 10'-Nitro-3',4'-dihydro-1'H-spiro[[1,3]dioxolane-2,2'-pyrido[2,1-a]isoindol]-6'(10b'H)-one (4).

Dans un ballon de 100 mL surmonté d'un appareil de Dean Stark, une solution contenant le *β*-hydroxylactame **3** (500 mg, 1.62 mmol) et de l'APTS.H₂O (156 mg, 0.8 mmol, 0.5 éq) dans le toluène (50 mL) est chauffée au reflux pendant 6h. Après refroidissement, une solution saturée de NaHCO₃ (45 mL) est ajoutée. Après extraction, la phase aqueuse est reprise à l'acétate d'éthyle (20 mL). Les phases organiques rassemblées sont séchées sur MgSO₄ puis les solvants sont évaporés sous pression réduite. Le résidu obtenu est purifié par chromatographie sur gel de silice (dichlorométhane / méthanol 99.5 / 0.5) pour donner le produit **4** sous forme d'un solide jaune avec un rendement de 62%. F : 206 °C ; **IR** (ATR-Ge ν cm⁻¹) : 1685 (C=O amide), 1539 (C=C arom), 1360 (N=O₂), 1142 (C-O), 1028 (C-C) ; RMN ¹H (CDCl₃ ; 250 MHz) δ 8.37 (d, *J* = 8.1 Hz, 1H), 8.19 (d, *J* = 7.4 Hz, 1H), 7.70 (t, *J* = 7.8 Hz, 1H), 5.23 (dd, *J* = 3.3 Hz, *J* = 11.7 Hz, 1H), 4.55 (dd, *J* = 4.3 Hz, *J* = 13.4 Hz, 1H), 4.31 - 3.96 (m, 4H), 3.33 (td, *J* = 4.2 Hz, *J* = 13.0 Hz, 1H), 2.78 - 2.61 (m, 1H), 1.95 - 1.65 (m, 2H), 1.17 (t, *J* = 12.2 Hz, 1H) ; RMN ¹³C (CDCl₃ ; 63 MHz) δ 163.8 (Cq), 143.7 (Cq), 140.0 (Cq), 136.0 (Cq), 130.2 (CH), 130.0 (CH), 127.1 (CH), 107.30 (Cq), 65.0 (CH₂), 64.9 (CH₂), 58.4 (CH), 38.1 (CH₂), 37.0 (CH₂), 34.3 (CH₂) ; **SM** (IS) m/z : 291.5 [M+H]⁺.

### 10'-Amino-3',4'-dihydro-1'H-spiro[[1,3]dioxolane-2,2'-pyrido[2,1-a] isoindol]-6'(10b'H)-one (5).

A une solution du composé **4** (1.0 g, 3.4 mmol) dans de l'EtOH (20 mL) est ajouté du SnCl₂ anhydre (9.5 g, 51.0 mmol, 15.0 éq.) à température ambiante. Le mélange est agité une nuit puis l'éthanol est évaporé sous pression réduite sans chauffer. Le résidu solide est placé à 0°C une solution aqueuse de NaOH (2M) est ajoutée jusqu'à pH = 7. Les phases aqueuses sont extraites au dichlorométhane (3 x 20 mL). Les phases organiques rassemblées sont séchées sur MgSO₄ puis le solvant est évaporé sous pression réduite. Le résidu solide est purifié par chromatographie sur gel de silice (dichlorométhane / méthanol 99 / 1) pour donner le composé 5 sous forme de solide blanc avec un rendement de 82%. F : 76 °C ; IR (ATR-Ge ν cm⁻¹) : 1710 (C=O amide), 1542 (C=C arom), 1366 (C-N), 1143 (C-O), 1028 (C-C) ; RMN ¹H (CDCl₃ ; 250 MHz) δ 7.36 - 7.24 (m, 2H), 6.81 (dd, *J* = 1.8 Hz, 7.0 Hz, 1H), 4.61 - 4.41 (m, 2H), 4.14 - 3.99 (m, 4H), 3.74 (s, 2H), 3.23 (td, *J* = 3.9 Hz, *J* = 13.1 Hz, 1H), 2.45(m, 1H), 1.82 (m, 1H), 1.67 (td, *J* = 5.7 Hz,13.0 Hz, 1H), 1.38 (t, *J* = 12.5 Hz, 1H) ; RMN ¹³C (CDCl₃ ; 63 MHz) *δ* 166.8 (Cq), 141.2 (Cq), 133.48 (Cq), 129.6 (CH), 129.5 (Cq), 118.4 (CH), 114.5 (CH), 107.7 (Cq), 65.0 (CH₂), 64.8 (CH₂), 55.8 (CH), 39.1 (CH₂), 36.6 (CH₂), 34.1 (CH₂) ; SM (IS) m/z : 261 [M+H]⁺

### 10'-Nitro-3',4'-dihydro-1'H-spiro[[1,3]dithiolane-2,2'-pyrido[2,1-a]isoindol]-6'(10b'H)-one (6).

A une solution du composé **4** (1.0 g, 3.4 mmol) dans le dichlorométhane (40 mL), sont ajoutés, doucement et avec précaution, l'éthane dithiol (1.24 mL, 17 mmol, 5 éq.) le BF₃·Et₂O (2.15 mL, 17.0 mmol, 5.0 éq.). Après agitation pendant 24h à température ambiante, du CH₂Cl₂ (40 mL) et une solution aqueuse de NaOH 1M (40 mL) sont ajoutés. Après extraction, la phase organique est séchée sur MgSO₄ puis les solvants sont évaporés sous pression réduite. Le résidu est purifié par chromatographie sur gel de silice flash (acétate d'éthyle / éther de pétrole 60 / 40) pour donner le produit **6** sous forme d'une poudre beige avec un rendement de 86%. F : 185 °C ; IR (ATR-Ge ν cm⁻¹): 1689 (C=O amide), 1524 (C=C arom), 1416 (C-N), 1345 (N=O₂), 1079 (C-C) ; RMN ¹H (CDCl₃ ; 250 MHz) δ 8.36 (dd, *J* = 0.9 Hz, 8.2 Hz, 1H), 8.18 (dd, *J* = 0.6 Hz, *J* = 7.5 Hz, 1H), 7.69 (t, *J* = 7.8 Hz, 1H), 5.25 (dd, *J* = 3.1 Hz, *J* = 11.1 Hz, 1H), 4.57 (m, 1H), 3.56 - 3.36 (m, 4H), 3.36 - 3.19 (m, 1H), 3.10 - 2.97 (m, 1H), 2.31 - 1.97 (m, 2H), 1.51 (dd, *J* = 11.2 Hz, *J* = 12.9 Hz, 1H) ; RMN ¹³C (CDCl*₃* ; 63 MHz) *δ* 163.7 (Cq), 143.8 (Cq), 139.6 (Cq), 135.9 (Cq), 130.2 (CH), 130.1 (CH), 127.1 (CH), 65.5 (Cq), 59.5 (CH), 45.4 (CH₂), 40.6 (CH₂), 39.4 (CH₂), 39.3 (CH₂), 38.7 (CH₂) ; **SM** (IS) m/z : 323.0 [M+H]⁺, 340.0 [M+NH₄]⁺

### 10'-Amino-3',4'-dihydro-1'H-spiro[[1,3]dithiolane-2,2'-pyrido[2,1-a]isoindol]-6'(10b'H)one (7).

A une solution contenant le composé 6 (800 mg, 2.4 mmol) du composé 6 dans de l'EtOH (20 mL) sont ajoutés 6.8 g de SnCl₂ (36.0 mmol, 15.0 éq.). Le mélange est agité une nuit puis l'éthanol est évaporé sous pression réduite sans chauffer. Le résidu est refroidi à 0°C puis neutralisé par ajouts successifs d'une solution aqueuse de NaOH 2M. La phase aqueuse est extraite au dichlorométhane (3 x 20 mL), les phases organiques rassemblées sont ensuite séchées sur MgSO₄, filtrées et évaporées sous pression réduite. Le résidu est purifié par chromatographie sur gel de silice (dichlorométhane / méthanol 99 / 1) pour donner le composé 7 sous forme de solide jaune avec un rendement de 80%. F : 205 °C ; IR (ATR-Ge ν cm⁻¹) : 3236 (NH₂), 1675 (C=O amide), 1487 (C=C arom), 1287 (C-N), 1003 (C-C) ; RMN ¹H (CDCl₃ ; 400 MHz) δ 7.37 - 7.17 (m, 2H), 6.80 (dd, *J*= 1.3 Hz, 7.2 Hz, 1H), 4.60 - 4.41 (m, 2H), 3.81 (s, 2H), 3.50 - 3.31 (m, 4H), 3.23 (td, J = 3.2 Hz, J = 13.3 Hz, 1H), 2.92 - 2.78 (m, 1H), 2.24 - 2.13 (m, 1H), 2.02 (td, *J* = 5.1 Hz, *J* = 12.9 Hz, 1H), 1.77 - 1.63 (m, 1H); RMN ¹³C (CDCl*₃* ; 101 MHz) *δ* 166.7 (Cq), 141.4 (Cq), 133.3 (Cq), 129.7 (CH), 129.0 (Cq), 118.4 (CH), 114.3 (CH), 65.7 (Cq), 57.1 (CH), 45.7 (CH₂), 41.6 (CH₂), 39.66 (CH₂), 38.7 (CH₂), 38.4 (CH₂) ; SM (IS) m/z : 293.0 [M+H]⁺.

### 10-Nitro-1,3,4,10b-tétrahydropyrido[2,1-a]isoindole-2,6-dione (8).

A une solution de composé 4 (2.0 g, 6.91 mmol) dans de l'acétone (30 mL) est ajoutée une solution aqueuse d'acide chlorhydrique 10% (20 mL). Le mélange réactionnel est porté à reflux pendant 3h puis concentré à 50% par évaporation sous pression réduite. Après refroidissement dans un bain d'eau glacée, le précipité est filtré puis lavé avec de l'eau (10 mL) et séché sous pression réduite pour donner le composé 8 sous forme de solide jaune avec un rendement de 90%. F : 215 °C ; **IR** (ATR-Ge ν cm⁻¹) : 1692 (C=O cétone), 1529 (C=C arom), 1342 (NO₂), 1298 (C-N) ; RMN ¹H (CDCl₃ ; 400 MHz) *δ* 8.44 (d, *J* = 8.0 Hz, 1H), 8.25 (d, *J* = 7.4 Hz, 1H), 7.78 (t, *J* = 7.8 Hz, 1H), 5.39 (dd, *J* = 4.0 Hz, 11.6 Hz, 1H), 4.82 - 4.76 (m, 1H), 3.55 - 3.38 (m, 2H), 2.72 - 2.52 (m, 2H), 2.08 (dd, *J* = 11.7 Hz, 14.5 Hz, 1H) ; RMN ¹³C *(CDCl₃ ;* 101 MHz) *δ* 204.4 (Cq), 164.1 (Cq), 143.6 (Cq), 139.2 (Cq), 135.4 (Cq), 130.7 (CH), 130.6 (CH), 127.7 (CH), 58.7 (CH), 44.5 (CH₂), 39.5 (CH₂), 37.3 (CH₂) ; SM (IS) m/z : 293.5 [M+H]⁺

### 10-Amino-1,3,4,10b-tétrahydropyrido[2,1-a]isoindole-2,6-dione (9).

A une solution contenant le composé 8 (3.0 g, 12.2 mmol) dissous dans de l'EtOH (80 mL) sont ajoutés par portions 34.0 g de SnCl₂ (180 mmol, 15.0 éq.) puis 46 ml d'une solution aqueuse d'acide chlorhydrique 12N. Le milieu réactionnel est agité une nuit à température ambiante puis l'éthanol est évaporé sous pression réduite à basse température. Le résidu est placé à 0°C puis neutralisé avec une solution aqueuse de NaOH 2M. La phase aqueuse est extraite au dichlorométhane (3 x 50 mL) puis les phases organiques rassemblées sont séchées sur MgSO₄, filtrées et évaporées sous pression réduite. Le résidu est purifié par chromatographie sur gel de silice flash (dichlorométhane / méthanol 99 / 1) pour donner le composé 9 sous forme de solide blanc avec un rendement de 72%. IR (ATR-Ge ν cm⁻¹) : RMN ¹H (***CDCl₃***; 400 MHz) δ 7.34 - 7.27 (m, 2H), 6.85 (dd, *J* = 3.9 Hz, 4.9 Hz, 1H), 4.79 - 4.73 (m, 1H), 4.66 (dd, *J* = 3.9 Hz, 11.9 Hz, 1H), 3.90 (s, 2H), 3.40 - 3.32 (m, 1H), 3.21 - 3.16(m, 1H), 2.65 - 2.47 (m, 2H), 2.21 (dd, *J* = 12.2 Hz, 13.8 Hz, 1H) ; RMN ¹³C ***(CDCl₃**;* 101 MHz) *δ* 206.4 (Cq), 167.0 (Cq), 141.4 (Cq), 132.7 (Cq), 130.2 (CH), 128.5 (Cq), 118.9 (CH), 114.4 (CH), 56.8 (CH), 45.0 (CH₂), 40.0 (CH₂), 37.5 (CH₂) ; SM (IS) m/z :

### 10-Amino-1,2,3,4-tétrahydropyrido[2,1-a]isoindol-6(10bH)-one (10).

Dans un ballon contenant 9 mL d'éthylène glycol sont ajoutés sous agitation et avec précaution 160 mg de sodium (6.93 mmol, 3.0 éq.) à température ambiante puis 500 mg de la cétone 9 (2.31 mmol) et enfin 0.45 mL d'hydrazine monohydratée (9.24, mmol, 4 éq.). Le milieu réactionnel est chauffé pendant 4h à 190°C. Après disparition complète du produit de départ, le mélange est refroidi et du dichlorométhane (50 mL) est ajouté. La phase organique est lavée avec une solution aqueuse de NaOH 1 M, séchée sur MgSO₄, filtrée puis les solvants sont évaporés. Le produit brut est purifié par chromatographie sur gel de silice (dichlorométhane / méthanol 99.5 / 0.5) pour donner le composé 10 sous forme de solide jaune avec un rendement de 80%. **IR** (ATR-Ge ν cm⁻¹) : RMN ¹H (CDCl*₃* ; 250 MHz) *δ* 7.35 - 7.17 (m, 2H), 6.80 (dd, *J* = 1.4 Hz, 7.3 Hz, 1H), 4.49 (dd, *J* = 4.9 Hz, *J* = 13.3 Hz, 1H), 4.23 (dd, *J* = 3.6 Hz, *J* = 11.7 Hz, 1H), 3.79 (s, 2H), 2.96 (td, *J* = 3.5 Hz, *J* = 12.9 Hz, 1H), 2.50 (dd, *J* = 3.2 Hz, *J* = 13.4 Hz, 1H), 1.99 (m, 1H), 1.88 - 1.74 (m, 1H), 1.74 - 1.52 (m, 1H), 1.52 - 1.29 (m, 1H), 1.11 (m, 1H) ; RMN ¹³C (CDCl₃ ; 63 MHz) *δ* 166.6 (Cq), 141.3 (Cq), 133.6 (Cq), 130.2 (Cq), 129.3 (CH), 118.1 (CH), 114.2 (CH), 57.9 (CH), 39.9 (CH₂), 30.4 (CH₂), 25.5 (CH₂), 23.7 (CH₂) ; **SM** (IS) m/z : 203.0 [M+H]⁺.

### 10-Isothiocyanato-1,2,3,4-tétrahydropyrido[2,1-a]isoindol-6(10bH)-one (11).

A une solution contenant l'amine 10 (100 mg, 0.49 mmol) dans le chloroforme (20 mL) est ajoutée une solution de 289 mg de NaHCO₃ dans 20 ml d'eau. Le mélange est refroidi à 0°C puis 50 µl de thiophosgène (0.64 mmol, 1.3 éq.) sont ajoutés. La solution est agitée pendant 3h. Après extraction au chloroforme (3 x 20 mL), les phases organiques rassemblées sont séchées sur MgSO₄, filtrées et concentrées sous pression réduite. Le résidu est purifié par chromatographie sur gel de silice (dichlorométhane) pour donner le produit 11 sous forme d'huile avec un rendement quantitatif. **IR** (ATR-Ge ν cm⁻¹) : 2056 (N=C=S), 1687 (C=O amide), 1591-1475-1417 (C=C arom), 1284 (C-N) ; RMN ¹H *(CDCl₃;* 400 MHz) δ 7.77 (d, *J=* 7.5 Hz, 1H), 7.46 (t, *J =* 7.7 Hz, 1H), 7.37 (d, *J =* 7.9 Hz, 1H), 4.50 (dd, *J =* 5.0 Hz, 13.3 Hz, 1H), 4.40 (dd, *J =* 3.6 Hz, 12.4 Hz, 1H), 3.00 (td, *J =* 3.6 Hz, 12.4 Hz, 1H), 2.64 (dd, *J* = 3.1 Hz, 12.9 Hz, 1H), 2.05 (d, *J* = 13.7 Hz, 1H), 1.90 - 1.80 (m, 1H), 1.80 - 1.64 (m, 1H), 1.49 - 1.34 (m, 1H), 1.17 - 1.07 (m, 1H) ; RMN ¹³C *(CDCl₃;* 101 MHz) δ 164.9 (Cq), 140.8 (Cq), 138.2 (Cq), 134.8 (Cq), 129.7 (CH), 128.7 (CH), 126.5 (Cq), 122.8 (CH), 58.2 (CH), 40.0 (CH₂), 30.8 (CH₂), 25.3 (CH₂), 23.6 (CH₂).

### 10'-Isthiocyanato-3',4'-dihydro-1'H-spiro[[1,3]dithiolane-2,2'-pyrido[2,1-a]isoindol]-6'(10b'H) -one (12).

Une solution contenant l'amine 7 (900 mg, 3.0 mmol) dans le chloroforme (120 mL) et une solution de 1.78 g de NaHCO₃ dans 115 ml d'eau est refroidie à O°C. Le thiophosgène (0.3 mL, 3.9 mmol) est ajouté goutte à goutte puis la solution est agitée pendant 3h. Après extraction au chloroforme (3 x 20 mL), les phases organiques rassemblées sont séchées su MgSO₄, filtrées et concentrées sous pression réduite. Le résidu est purifié par chromatographie sur gel de silice (dichlorométhane) pour donner le produit 12 sous forme de poudre blanche avec un rendement de 56%. F : 150 °C ; **IR** (ATR-Ge ν cm⁻¹) : 2085 (N=C=S), 1690 (C=O amide), 1592-1474-1417 (C=C arom), 1258 (C-N) ; RMN ¹H (CDCl*₃* ; 250 MHz) *δ* 7.65 (dd, *J* = 0.8 Hz, *J* = 7.5 Hz, 1H), 7.38 (t, *J* = 7.7 Hz, 1H), 7.26 (dd, *J* = 1.0 Hz, J = 7.9 Hz, 1H), 4.63 (dd, *J* = 3.4 Hz, *J* = 11.6 Hz, 1H), 4.43 (m, 1H), 3.49 - 3.27 (m, 4H), 3.13 (td, *J* = 3.4 Hz, *J* = 13.2 Hz, 1H), 2.86 (m, 1H), 2.22 - 2.07 (m, 1H), 1.97 (td, *J* = 5.1 Hz, *J* = 12.9 Hz, 1H), 1.58 (dd, *J* = 11.7 Hz, *J* = 12.9 Hz, 1H) ; RMN ¹³C (CDCl₃ ; 63 MHz) *δ* 164.6 (Cq), 140.1 (Cq), 139.1 (Cq), 134.3 (Cq), 129.8 (CH), 128.3 (CH), 126.5 (Cq), 122.7 (CH), 65.2 (Cq), 57.0 (CH), 46.0 (CH₂), 40.2 (CH₂), 39.2 (CH₂), 38.9 (CH₂), 38.4 (CH₂).

### 2-Hydroxy-10-nitro-1,2,3,4-tétrahydropyrido[2,1-a]isoindol-6(10bH)-one (13).

On a ajouté lentement par portion NaBH₄ (155 mg, 4.08 mmol) à -20 °C dans un flacon, sous atmosphère inerte, à une solution du composé 8 (500 mg, 2,04 mmol) dans un mélange tétrahydrofurane/méthanol (10 mL/ 20 mL). La solution a été agitée à cette température pendant 1 h et ensuite à température ambiante pendant 4 h. On a ajouté de l'eau (10 mL) et la phase aqueuse a été extraite en premier avec du dichlorométhane (2 x 10 mL) et ensuite avec de l'acétate d'éthyle (10 mL). Les phases organiques combines ont été séchées, filtrées et concentres sous pression réduite. Le résidu brut a été purifié par chromatographie sur gel de silice flash (CH₂Cl₂/MeOH 95:5) pour obtenir le composé 13 avec un rendement de 86%, sous la forme d'un solide blanc; p.f. > 260°C; ¹H *RMN (DMSO- d₆, 400 MHz) δₚₚₘ*: 8.39 (d, *J =* 8.0 Hz, 1H), 8.11 (d, *J =* 8.0 Hz, 1H), 7.79 (dd, *J* = 8.0 Hz, 1H), 5.01-5.07 (m, 2H), 4.24 (dd, *J =* 5.0 Hz, *J* = 12.5 Hz, 1H), 3.89-3.98 (m, 1H), 3.08 (td, *J* = 5.0 Hz, *J* = 12.5 Hz, 1H), 2.64 (d, *J* = 13.2 Hz, 1H), 1.93 (d, *J* = 12.5 Hz, 1H), 1.04-1.21 (m, 1H), 0.76 (q, *J* = 12.5 Hz, 1H). *¹³C RMN (DMSO- d₆, 100 MHz) δₚₚₘ*: 162.5 (Cq), 143.2 (Cq), 139.6 (Cq), 135.0 (Cq), 130.2 (CH), 129.5 (CH), 126.9 (CH), 66.9 (CH), 57.8 (CH), 38.2 (CH₂), 36.6 (CH₂), 33.9 (CH₂). *IR* (ATR-Ge ν cm⁻¹) : 2 973 (O-H), 1683 (C=O cétone), 1566 (C=C arom), 1329 (NO₂), 1288 (C-N). MS (IS) m/z : 249.0 [M+H]⁺.

### 2-Méthoxy-10-nitro-1,2,3,4-tétrahydropyrido[2,1-a]isoindol-6(10bH)-one (14).

On a ajouté à la solution du composé 13 (100 mg, 0,4 mmol) dans du THF anhydre (5 mL), du Mel (0.25 mL, 4.0 mmol) et du Ag₂0 fraîchement préparé (37 mg, 1.6 mmol). La mélange a été agité à 50°C pendant 48 h. Après refroidissement à température ambiante, on a récupéré un précipité d'Ag₂O par filtration par aspiration, lavé avec du dichlorométhane (3 x 10 mL), et les filtrats organiques combinés ont été concentrés sous vide. Le résidu a été purifié par chromatographie sur gel de silice ((CH₂Cl₂/MeOH 99:1) pour obtenir le composé pur 14 en tant que solide blanc avev un rendement de 61%. p.f. 132-134°C; ¹H *RMN (*CDCl₃, 400 *MHz) δₚₚₘ* : 8.33 (d, *J =* 8.2 Hz, 1H), 8.15 (d, *J =* 7.5 Hz, 1H), 7.67 (dd, *J =* 7.5 Hz, *J =* 8.0 Hz, 1H), 4.96 (dd, *J =* 3.2 Hz, *J =* 11.6 Hz, 1H), 4.56 (dd, *J =* 5.0 Hz, *J =* 13.2 Hz, 1H), 3.64 (m, 1H), 3.42 (s, 3H), 2.95-3.08 (m, 2H), 2.17 (d, *J =* 13.2 Hz, 1H), 1.25-1.39 (m, 1H), 0.82 (q, *J* = 12.5 Hz, 1H). *¹³C RMN* (CDCl₃ , 100 *MHz) δₚₚₘ* : 136.5 (Cq), 143.6 (Cq), 139.6 (Cq), 135.7 (Cq), 130.1 (CH), 129.9 (CH), 127.0 (CH), 76.8 (CH), 58.6 (CH), 56.1 (CH₃), 37.3 (CH₂), 35.0 (CH₂), 30.9 (CH₂). *IR* (ATR-Ge ν cm⁻¹) : 1688 (C=O cétone), 1523 (C=C arom), 1349 (NO₂) 1285 (C-N). *MS* (IS) m/z : 285.0 [M+Na]⁺.

### 10-Amino-2-méthoxy-1,2,3,4-tétrahydropyrido[2,1-a]isoindol-6(10bH)-one (15).

On a ajouté du nickel de Raney W (ca 200 mg) fraîchement préparé à une solution agitée du composé **14** (1.0 g, 3.8 mmol) dans de l'éthanol absolu (30 mL). La suspension résultante a été hydrogénée à température ambiante pendant 16 h. Le mélange a été filtré sur Celite et lavé avec du dichlorométhane (3 x 10 mL). La phase organique a été concentrée sous vide pour donner 815 mg (92%) du composé **15** sous forme de solide jaune, p.f. 60-62°C; ¹H RMN (CDCl₃, 400 MHz) δₚₚₘ : 7.28 (m, 2H), 6.82 (d, J = 8.5 Hz, 1H), 4.56 (dd, J = 5.0 Hz, J = 11.7 Hz, 1H), 4.33 (dd, *J* = 5.0 Hz, *J* = 12.0 Hz, 1H), 3.84 (bl, 2H), 3.52-3.64 (m, 1H), 3.43 (m, 3H), 2.96 (td, *J* = 2.5 Hz, J = 12.0 Hz, 1H), 2.81 (d, *J* = 12.2 Hz, 1H), 2.18 (d, J = 12.2 Hz, 1H), 1.34-1.44 (m, 1H), 1.05 (q, *J =* 12.2 Hz, 1H). ¹³C RMN (CDCl₃, 100 MHz) δₚₚₘ : 166.5 (Cq), 141.1 (Cq), 133.1 (Cq), 129.3 (CH), 128.9 (Cq), 118.2 (CH), 114.1 (CH), 77.1 (CH), 56.1 (CH), 55.9 (CH₃), 36.9 (CH₂), 35.4 (CH₂), 30.8 (CH₂). IR (ATR-Ge ν cm⁻¹) : 3236 (NH₂), 1683 (C=O cétone), 1566 (C=C arom), 1288 (C-N). MS (IS) m/z : 233.0 [M+H]⁺.

### 2. Préparation des composés de l'invention

### 2.1. Procédure générale A

### Synthèse des urées (16)-(61) à partir d'isocyanates et des amines (5), (7), (10) ou (15).

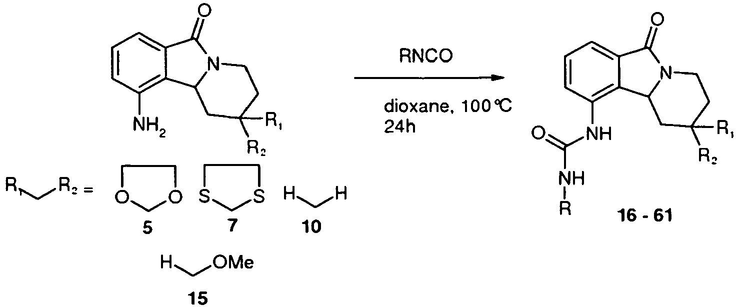

Les composés (16) à (61) ci-après sont des composés de formule (I), dans laquelle R₄ et R" sont H et A est un groupe CO, et R₁ et R₂ sont H, ou H et OMe, ou forment ensemble, avec l'atome de carbone sur lequel ils sont fixés, un groupe

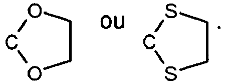

***Synthèse des isocyanates :*** Sous atmosphère inerte, l'acide carboxylique (0.37 mmol, 1.1 éq.) est dissous dans 7 ml de THF anhydre puis 66 µl (0.48 mmol) de triéthylamine sont ajoutés. Après 5 min d'agitation à -10°C, 53 µl de chloroformiate d'éthyle (0.55 mmol) sont additionnés. Le mélange est agité à cette température pendant 1 heure. L'analyse par TLC indique la conversion complète de la réaction. A ce stade, une solution aqueuse de NaN₃ (40 mg, 0.63 mmol) est ajoutée. L'agitation est maintenue pendant une heure à -10°C. Le mélange réactionnel est filtré, le filtrat est concentré sous pression réduite et la phase aqueuse est extraite avec l'acétate d'éthyle (2 x 20 mL). Les phases organiques rassemblées sont séchées sur MgSO₄, filtrées puis concentrées sous pression réduite pour donner un azoture d'acyle correspondant qui est transformé par réarrangement de Curtius dans le toluène (20 mL) au reflux en isocyanate correspondant.

***Synthèse des dérivés d'urées** :* A une solution de l'isocyanate fraîchement préparé dans 7 ml de dioxane anhydre, l'amine (0.34 mmol, 5, 7, 10 ou 15) est ajoutée en une portion puis le mélange réactionnel est chauffé à 100°C pendant 24h. Après refroidissement, le solvant est évaporé sous pression réduite et le produit brut est purifié par chromatographie sur gel de silice pour donner les urées attendues.

### 1-(6'-oxo-3',4',6',10b'-tétrahydro-1'H-spiro[[1,3]dithiolane-2,2'-pyrido[2,1-a]isoindole]-1 0'-yl)-3-(pyridin-2-yl)urée (16).

Le composé 16 est obtenu sous forme de solide blanc selon la procédure générale A avec un rendement de 96% après purification sur gel de silice (CH₂Cl₂ /MeOH : 99 /1). F : 242 °C ; IR (ATR-Ge ν cm⁻¹) : 1703-1677 (C=O amide), 1553 (N-H amide), 1510- 1483-1418 (C=C arom), 1310 (C-N), 1153 (C-C) ; RMN ¹H *(*DMSO-d6 ; 400 MHz) *δ* 1.34 (s, 1H), 9.98 (s, 1H), 8.47 (d, *J* = 3.9 Hz, 1H), 8.21 (d, *J* = 7.9 Hz, 1H), 7.84 - 7.74 (m, 1H), 7.48 (t, *J* = 7.7 Hz, 1H), 7.42 (d, *J* = 6.9 Hz, 1H), 7.23 (d, *J* = 8.3 Hz, 1H), 7.10 - 7.04 (m, 1H), 4.80 (dd, *J* = 3.3 Hz, *J* = 11.5 Hz, 1H), 4.34 (dd, *J* = 3.2 Hz, *J* = 13.7 Hz, 1H), 3.49 - 3.23 (m, 4H), 3.17 (td, *J* = 3.2 Hz, *J* = 13.3 Hz, 1H), 2.97 (d, *J* = 11.8 Hz, 1H), 2.14 (d, *J* = 13.3 Hz, 1H), 1.96 (td, *J* = 5.0 Hz, *J* = 13.0 Hz, 1H), 1.67 (t, *J* = 12.2 Hz, 1H) ; RMN ¹³C *(*DMSO-*d6* ; 101 MHz) *δ* 164.8 (Cq), 152.9 (Cq), 152.2 (Cq), 146.4 (CH), 139.0 (CH), 134.1 (Cq), 133.8 (Cq), 132.6 (Cq), 129.0 (CH), 123.6 (CH), 117.7 (CH), 117.3 (CH), 112.1 (CH), 65.5 (Cq), 56.8 (CH), 44.0 (CH₂), 40.6 (CH₂), 38.8 (CH₂), 38.2 (CH₂), 37.7 (CH₂) ; SM (IS) m/z : 413.5 [M+H]⁺

### 1-(6'-Oxo-3',4',6',10b'-tétrahydro-1'H-spiro[[1,3]dithiolane-2,2'-pyrido[2,1-a]isoindole]-10'-yl)-3-(pyrazin-2-yl)urée (17).

Le composé 17 est obtenu sous forme de solide blanc selon la procédure générale A avec un rendement de 90% après purification sur gel de silice (CH₂Cl₂ /MeOH : 99 /1). F : 236 °C ; IR (ATR-Ge ν cm⁻¹) : 1676 (C=O amide), 1551 (N-H amide), 1505- 1483-1421 (C=C arom), 1298 (C-N), 1138 (C-C) ; RMN ¹H (DMSO-d6 ; 400 MHz) δ 10.19 (s, 1H), 10.13 (s, 1H), 8.83 (d, *J* = 1.1 Hz, 1H), 8.40 (dd, *J* = 1.5 Hz, *J* = 2.6 Hz, 1H), 8.29 (d, *J* = 2.7 Hz, 1H), 8.08 (dd, *J* = 0.9 Hz, *J* = 7.8 Hz, 1H), 7.54 - 7.42 (m, 2H), 4.79 (dd, *J* = 3.4 Hz, *J* = 11.6 Hz, 1H), 4.33 (dd, *J* = 3.1 Hz, *J* = 13.7 Hz, 1H), 3.48 - 3.28 (m, 4H), 3.16 (td, *J* = 3.1 Hz, *J* = 13.1 Hz, 1H), 2.89 (d, *J* = 11.5 Hz, 1H), 2.14 (d, *J* = 13.2 Hz, 1H), 1.96 (td, *J* = 5.0 Hz, *J* = 12.9 Hz, 1H), 1.63 (t, *J* = 12.3 Hz, 1H) ; RMN ¹³C *(*DMSO-*d6* ; 101 MHz) *δ* 164.68 (Cq), 151.7 (Cq), 149.2 (Cq), 140.8 (CH), 137.6 (CH), 135.6 (CH), 134.7 (Cq), 133.3 (Cq), 132.7 (Cq), 129.1 (CH), 124.0 (CH), 118.3 (CH), 65.6 (Cq), 56.9 (CH), 44.0 (CH₂), 40.5 (CH₂), 38.7 (CH₂), 38.2 (CH₂), 37.7 (CH₂) ; SM (IS) m/z : 414.0 [M+H]⁺.

### 1-(5-Bromopyridin-2-yl)-3-(6-oxo-1,2,3,4,6,10b-hexahydropyrido[2,1-a] isoindol-10-yl)urée (18).

Le composé 18 est obtenu sous forme de solide beige selon la procédure générale A avec un rendement de 66 % après purification sur gel de silice (CH₂Cl₂ /MeOH : 99 /1). F : 225 °C ; IR (ATR-Ge ν cm⁻¹) : 1684 (C=O amide), 1560 (N-H amide), 1498- 1465-1445 (C=C arom), 1362 (C-N), 1298- 1232 (C-C), 749 (C-H arom) ; RMN ¹H *(*DMSO-*d6* ; 400 MHz) δ 10.25 (s, 1H), 10.00 (s, 1H), 8.40 (d, *J* = 2.3 Hz, 1H), 8.14 (dd, *J* = 0.7 Hz, *J* = 7.9 Hz, 1H), 8.00 (dd, *J* = 2.5 Hz, *J* = 8.9 Hz, 1H), 7.50 (d, *J* = 8.9 Hz, 1H), 7.45 (t, *J* = 7.7 Hz, 1H), 7.39 (dd, *J* = 0.8 Hz, *J* = 7.4 Hz, 1H), 4.59 (dd, *J* = 3.2 Hz, *J* = 11.5 Hz, 1H), 4.25 (dd, *J* = 4.2 Hz, *J* = 13.0 Hz, 1H), 3.00 - 3.11 (m, 1H), 2.63 (dd, *J* = 2.7 Hz, *J* = 12.6 Hz, 1H), 1.90 (d, *J* = 12.6 Hz, 1H), 1.82 - 1.62 (m, 2H), 1.33 - 1.20 (m, 1H), 0.93 - 0.77 (m, 1H) ; RMN ¹³C *(*DMSO-*d6*; 101 MHz) *δ* 164.5 (Cq), 151.8 (Cq), 151.7 (Cq), 147.1 (CH), 141.2 (CH), 135.2 (Cq), 133.7 (Cq), 132.9 (Cq), 128.8 (CH), 122.5 (CH), 117.6 (CH), 113.9 (CH), 111.7 (Cq), 57.2 (CH), 45.6 (CH₂), 30.0 (CH₂), 25.0 (CH₂), 22.9 (CH₂) ; SM (IS) m/z : 402 [M+H]⁺

### 1-(6-Bromopyridin-2-yl)-3-(6-oxo-1,2,3,4,6,10b-hexahydropyrido[2,1-a] isoindol-10-yl) urée (19).

Le composé 19 est obtenu sous forme de solide beige selon la procédure générale A avec un rendement de 70 % après purification sur gel de silice (CH₂Cl₂ /MeOH : 99 /1). F : 218 - 220 °C ; IR (ATR-Ge ν cm⁻¹) : 1678 (C=O amide), 1538 (N-H amide), 1458- 1429-1390 (C=C arom), 1337 (C-N), 1281- 1251 (C-C), 748 (C-Br) ; RMN ¹H *(*DMSO-*d6* ; 400 MHz) *δ* 10.04 (s, 1H), 9.28 (s, 1H), 8.10 (dd, *J* = 0.7 Hz, *J* = 7.8 Hz, 1H), 7.75 - 7.65 (m, 2H), 7.46 (t, *J* = 7.7 Hz, 1H), 7.43 - 7.38 (m, 1H), 7.27 (dd, *J* = 1.4 Hz, *J* = 6.8 Hz, 1H), 4.56 (dd, *J* = 3.4 Hz, *J* = 11.5 Hz, 1H), 4.26 (dd, *J* = 3.6 Hz, *J* = 13.0 Hz, 1H), 3.02 (td, *J* = 3.6 Hz, *J* = 12.9 Hz, 1H), 2.66 - 2.55 (m, 1H), 1.89 (d, *J* = 13.0 Hz, 1H), 1.81 - 1.61 (m, 2H), 1.34 - 1.20 (m, 1H), 0.87 (qd, *J* = 3.1 Hz, *J* = 12.7 Hz, 1H) ; RMN ¹³C *(*DMSO-*d6* ; 101 MHz*) δ* 164.5 (Cq), 152.9 (Cq), 151.4 (Cq), 141.5 (CH), 138.1 (Cq), 135.2 (Cq), 133.5 (Cq), 132.9 (Cq), 128.8 (CH), 122.9 (CH), 121.2 (CH), 117.7 (CH), 110.7 (CH), 57.2 (CH), 39.0 (CH₂), 29.8 (CH₂), 25.0 (CH₂), 23.0 (CH₂) ; SM (IS) m/z : 402 [M+H]⁺.

### 1-(6-Oxo-1,2,3,4,6,10b-hexahydropyrido[2,1-a]isoindol-10-yl)-3-(quinolin-2-yl)urée (20).

Le composé 20 est obtenu sous forme de solide blanc selon la procédure générale A avec un rendement de 67 % après purification sur gel de silice (CH₂Cl₂ /MeOH : 99 /1). F : 260 °C ; IR (ATR-Ge ν cm⁻¹) : 1691 (C=O amide), 1563 (N-H amide), 1432-1424 (C=C arom), 1256 - 1040 (C-C), 826- 753 (C-H arom) ; RMN ¹H *(*CDCl*₃* ; 400 MHz) *δ*12.32 (s, 1H), 10.24 (s, 1H), 8.35 (d, *J* = 8.0 Hz, 1H), 7.94 (d, *J* = 8.8 Hz, 1H), 7.71 (dd, *J* = 6.2 Hz, *J* = 15.8 Hz, 4H), 7.54 (t, *J* = 7.7 Hz, 1H), 7.48 - 7.44 (m, 1H), 7.10 (d, *J* = 8.8 Hz, 1H), 4.56 (dd, *J* = 4.0 Hz, *J* = 13.2 Hz, 1H), 4.42 (dd, J = 3.0 Hz, *J* = 11.7 Hz, 1H), 2.98 (td, *J* = 3.0 Hz, *J* = 12.9 Hz, 1H), 2.70 (d, *J* = 10.5 Hz, 1H), 1.82 (t, *J* = 15.1 Hz, 2H), 1.59 (q, *J* = 13.2 Hz, 1H), 1.47 - 1.31 (m, 1H), 1.05 (qd, *J* = 4.0 Hz, *J* = 12.0 Hz, 1H) ; RMN ¹³C *(*CDCl₃ ; 101 MHz) *δ* 166.0 (Cq), 154.3 (Cq), 152.3 (Cq), 145.0 (Cq), 139.1 (CH), 135.9 (Cq), 133.7 (Cq), 133.4 (Cq), 130.4 (CH), 129.2 (CH), 128.2 (CH), 126.2 (CH), 125.0 (CH), 125.0 (Cq), 124.5 (CH), 119.5 (CH), 114.0 (CH), 58.4 (CH), 40.0 (CH₂), 29.8 (CH₂), 25.4 (CH₂), 23.9 (CH₂) ; SM (IS) m/z : 373.0 [M+H]⁺

### 1-(6-Bromopyridin-2-yl)-3-(6'-oxo-3',4',6',10b'-tétrahydro-1'H-spiro[[1,3] dithiolane-2,2'-pyrido[2,1-a]isoindole]-10'-yl)urée (21).

Le composé **21** est obtenu sous forme de solide jaune selon la procédure générale A avec un rendement de 57 % après purification sur gel de silice (CH₂Cl₂ /MeOH : 99 /1). F : 170°C ; IR (ATR-Ge ν cm⁻¹) : 1654 (C=O amide), 1565 (N-H amide), 1530- 1486- 1430 (C=C arom), 786 (C-Br) ; RMN ¹H *(*CDCl₃ ; 400 MHz) *δ* 10.89 (s, 1H), 9.93 (s, 1H), 7.74 (dd, *J* = 7.6 Hz, *J* = 13.8 Hz, 2H), 7.52 (td, *J* = 5.7 Hz, *J* = 7.8 Hz, 2H), 7.15 (d, *J* = 7.7 Hz, 2H), 4.98 (dd, *J* = 3.3 Hz, *J* = 11.5 Hz, 1H), 4.56 (dd, *J* = 3.7 Hz, *J* = 13.8 Hz, 1H), 3.32 - 3.22 (m, 4H), 3.22 - 3.15 (m, 1H), 2.86 (d, *J* = 12.0 Hz, 1H), 2.19 (d, *J* = 13.5 Hz, 1H), 2.04 (td, *J* = 5.0 Hz, *J* = 13.4 Hz, 1H), 1.67 (t, *J* = 12.0 Hz, 1H) ; RMN ¹³C *(*CDCl*₃* ; 101 MHz) *δ* 166.1 (Cq), 153.6 (Cq), 153.1 (Cq), 140.8 (CH), 138.2 (Cq), 137.3 (Cq), 133.7 (Cq), 132.4 (Cq), 129.4 (CH), 127.3 (CH), 121.4 (CH), 120.9 (CH), 111.2 (CH), 65.6 (Cq), 58.2 (CH), 45.2 (CH₂), 40.9 (CH₂), 39.0 (CH₂), 38.9 (CH₂), 38.6 (CH₂) ; SM (IS) m/z : décomposition.

### 1-(3-Méthylpyridin-2-yl)-3-(6-oxo-1,2,3,4,6,10b-hexahydropyrido[2,1-a] isoindol-10-yl) urée (22).

Le composé **22** est obtenu sous forme de solide blanc selon la procédure générale A avec un rendement de 58 % après purification sur gel de silice (CH₂Cl₂ /MeOH : 99 /1). F : 235°C ; IR (ATR-Ge ν cm⁻¹) : 1681 (C=O amide), 1565 (N-H amide), 1482- 1459-1434- 1416 (C=C arom), 1286 (C-N), 1235 (C-C), 749 (C-H arom) ; RMN ¹H *(*CDCl₃ ; 400 MHz*) δ* 12.29 (s, 1H), 8.26 (d, *J* = 7.9 Hz, 1H), 8.09 (dd, *J* = 1.1 Hz, *J* = 5.0 Hz, 1H), 7.60 (dd, *J* = 0.6 Hz, *J* = 7.4 Hz, 1H), 7.53 (dd, *J* = 0.7 Hz, *J* = 7.3 Hz, 1H), 7.49 - 7.41 (m, 2H), 6.95 (dd, *J* = 5.1 Hz, *J* = 7.4 Hz, 1H), 4.54 (dd, *J* = 4.9 Hz, *J* = 13.3 Hz, 1H), 4.46 (dd, *J* = 3.5 Hz, *J* = 11.7 Hz, 1H), 3.02 (td, *J* = 3.5 Hz, *J* = 13.0 Hz, 1H), 2.81 (dd, *J* = 3.1 Hz, *J* = 13.0 Hz, 1H), 2.34 (s, 3H), 2.04 (d, *J* = 13.4 Hz, 1H), 1.83 (d, *J* = 13.2 Hz, 1H), 1.71 (qt, *J* = 3.2 Hz, *J* = 13.3 Hz, 1H), 1.54 - 1.36 (m, 1H), 1.08 (qd, *J* = 3.1 Hz, *J* = 13.0 Hz, 1H) ; RMN ¹³C (CDCl₃ ; 101 MHz) *δ* 166.1 (Cq), 152.8 (Cq), 151.3 (Cq), 143.0 (CH), 139.8 (CH), 135.5 (Cq), 133.5 (Cq), 133.4 (Cq), 129.2 (CH), 123.6 (CH), 120.0 (Cq), 119.0 (CH), 117.8 (CH), 58.5 (CH), 39.9 (CH₂), 30.3 (CH₂), 25.4 (CH₂), 24.1 (CH₂), 17.1 (CH₃) ; SM (IS) m/z : 337.0 [M+H]⁺.

### 1-(6-Fluoropyridin-2-yl)-3-(6-oxo-1,2,3,4,6,10b-hexahydropyrido[2,1-a] isoindol-10-yl) urée (23).

Le composé 23 est obtenu sous forme de solide blanc selon la procédure générale A avec un rendement de 70 % après purification sur gel de silice (CH₂Cl₂ /MeOH : 99 /1). F : 254°C ; IR (ATR-Ge ν cm⁻¹) : 1687 (C=O amide), 1585 (N-H amide), 1561- 1471-1420 (C=C arom), 1286 (C-F) ; RMN ¹H *(*DMSO-*d6* ; 250 MHz) *δ* 9.92 (s, 1H), 9.33 (s, 1H), 8.14 (dd, *J* = 1.2 Hz, *J* = 7.6 Hz, 1H), 7.94 (q, *J* = 8.2 Hz, 1H), 7.57 (dd, *J* = 2.1 Hz, *J* = 8.0 Hz, 1H), 7.51 - 7.36 (m, 2H), 6.77 (dd, *J* = 2.0 Hz, *J* = 7.9 Hz, 1H), 4.51 (dd, *J* = 3.3 Hz, *J* = 11.5 Hz, 1H), 4.26 (dd, *J* = 3.5 Hz, *J* = 13.0 Hz, 1H), 3.02 (td, *J* = 3.5 Hz, 1 *J* = 2.7 Hz, 1H), 2.75 - 2.57 (m, 1H), 1.90 (d, *J* = 13.2 Hz, 1H), 1.76 (d, *J* = 14.1 Hz, 1H), 1.72 - 1.52 (m, 1H), 1.40 - 1.12 (m, 1H), 1.03 - 0.70 (m, 1H) ; RMN ¹³C *(*DMSO-*d6* ; 63 MHz *) δ* 164.5 (Cq), 151.5 (Cq), 151.3 (Cq), 151.1 (Cq), 144.0 (CH), 135.1 (Cq), 133.6 (Cq), 132.9 (Cq), 128.8 (CH), 122.5 (CH), 117.6 (CH), 108.7 (CH), 101.3 (CH), 57.3 (CH), 39.1 (CH₂), 29.9 (CH₂), 24.9 (CH₂), 23.0 (CH₂) ; SM (IS) m/z : 341.0 [M+H]⁺, 363.0 [M+Na]⁺, 681.5 [2M+H]⁺.

### 1-(5-Méthylisoxazol-3-yl)-3-(6-oxo-1,2,3,4,6,10b-hexahydropyrido[2,1-a] isoindol-10-yl) urée (24).

Le composé 24 est obtenu sous forme de solide jaune selon la procédure générale A avec un rendement de 72 % après purification sur gel de silice (CH₂Cl₂ /MeOH : 99 /1). F : 156°C ; IR (ATR-Ge ν cm⁻¹) : 1680 (C=O amide), 1624 (C=N), 1599 (N-H amide), 1539- 1484-1428 (C=C arom), 1286 (C-O), 752 (C-H arom) ; RMN ¹H *(*CDCl*₃* ; 400 MHz) *δ* 9.53 (s, 1H), 9.33 (s, 1H), 7.99 (d, *J* = 7.9 Hz, 1H), 7.64 (d, *J* = 7.3 Hz, 1H), 7.44 (t, *J* = 7.8 Hz, 1H), 6.12 (s, 1H), 4.49 (td, *J* = 4.0 Hz, *J* = 11.8 Hz, 2H), 2.98 (td, *J* = 3.2 Hz, *J* = 13.0 Hz, 1H), 2.70 (d, *J* = 11.1 Hz, 1H), 2.41 (s, 3H), 1.94 (d, *J* = 13.3 Hz, 1H), 1.80 (d, *J* = 13.1 Hz, 1H), 1.70 - 1.53 (m, 1H), 1.46 - 1.29 (m, 1H), 1.03 (qd, *J* = 3.1 Hz, *J* = 12.8 Hz, 1H) ; RMN ¹³C *(*CDCl₃ ; 101 MHz) *δ* 169.87 (Cq), 166.22 (Cq), 159.04 (Cq), 152.78 (Cq), 136.75 (Cq), 133.55 (Cq), 133.00 (Cq), 129.28 (CH), 124.80 (CH), 119.95 (CH), 95.36 (CH), 58.73 (CH), 40.13 (CH₂), 30.31 (CH₂), 25.43 (CH₂), 23.68 (CH₂), 12.62 (CH₃) ; SM (IS) m/z : 327.0 [M+H]⁺, 349.0 [M+Na]⁺, 653.5 [2M+H]⁺.

### 1-(6-Oxo-1,2,3,4,6,10b-hexahydropyrido[2,1-a]isoindol-10-yl)-3-(5-phényl-isoxazol-3-yl)urée (25).

Le composé 25 est obtenu sous forme de solide blanc selon la procédure générale A avec un rendement de 87 % après purification sur gel de silice (CH₂Cl₂ /MeOH : 99 /1). F : 248°C ; IR (ATR-Ge ν cm⁻¹) : 1708 (C=O amide), 1680 (C=N), 1627 (N-H amide), 1535- 1521-1485 (C=C arom), 1366 (C-N), 1288 (C-O), 746 (C-H arom) ; RMN ¹H *(*DMSO-*d6* ; 400 MHz) *δ* 10.04 (s, 1H), 8.58 (s, 1H), 8.04 (d, *J* = 7.7 Hz, 1H), 7.88 (dd, *J* = 1.7 Hz, *J* = 7.6 Hz, 2H), 7.59 - 7.39 (m, 5H), 7.27 (s, 1H), 4.52 (dd, *J* = 3.3 Hz, *J* = 11.5 Hz, 1H), 4.26 (dd, *J* = 3.6 Hz, *J* = 13.0 Hz, 1H), 3.02 (td, *J* = 3.6 Hz, *J* = 12.8 Hz, 1H), 2.61 (dd, *J* = 2.9 Hz, *J* = 12.5 Hz, 1H), 1.91 (d, *J* = 13.1 Hz, 1H), 1.76 (d, *J* = 12.5 Hz, 1H), 1.71 - 1.54 (m, 1H), 1.34 - 1.17 (m, 1H), 0.86 (qd, *J* = 2.9 Hz, *J* = 12.7 Hz, 1H) ; RMN ¹³C *(*DMSO-*d6* ; 101 MHz) *δ* 168.5 (Cq), 164.4 (Cq), 159.1 (Cq), 151.2 (Cq), 135.5 (Cq), 133.6 (Cq), 133.0 (Cq), 130.5 (CH), 129.2 (2 x CH), 128.7 (CH), 126.8 (Cq), 125.4 (2 x CH), 123.0 (CH), 117.8 (CH), 93.7 (CH), 57.3 (CH), 39.0 (CH₂), 29.8 (CH₂), 25.0 (CH₂), 23.0 (CH₂) ; SM (IS) m/z : 389.0 [M+H]⁺, 411.0 [M+Na]⁺, 777.0 [2M+H]⁺.

### 1-(6-Oxo-1,2,3,4,6,10b-hexahydropyrido[2,1-a]isoindol-10-yl)-3-(thiazol-4-yl)urée (26).

Le composé 26 est obtenu sous forme de solide jaune selon la procédure générale A avec un rendement de 62 % après purification sur gel de silice (CH₂Cl₂ /MeOH : 99 /1). F : 178-180°C ; IR (ATR-Ge ν cm⁻¹) : 3305- 3224 (N-H amide), 1651 (C=O amide), 1529- 1485-1427 (C=C arom), 1288 (C-N), 1200 (C-C), 723 (C-H arom) ; RMN ¹H (CDCl₃ ; 400 MHz) *δ* 9.68 (s, 1H), 8.64 (d, *J*= 1.5 Hz, 1H), 7.96 (d, *J* = 7.8 Hz, 1H), 7.61 (d, *J* = 7.4 Hz, 1H), 7.41 (t, *J* = 7.8 Hz, 1H), 7.11 (s, 1H), 4.57 - 4.38 (m, 2H), 2.97 - 2.91 (m, 1H), 2.65 (d, *J* = 10.9 Hz, 1H), 1.88 (d, *J* = 12.2 Hz, 1H), 1.76 (d, *J* = 12.3 Hz, 1H), 1.60 - 1.43 (m, 1H), 1.43 - 1.28 (m, 1H), 1.10 - 0.92 (m, 1H) ; RMN ¹³C *(*CDCl₃ ; 101 MHz) *δ* 166.4 (Cq), 153.0 (Cq), 150.8 (CH), 149.9 (Cq), 136.6 (Cq), 133.5 (Cq), 133.3 (Cq), 129.2 (CH), 124.9 (CH), 119.4 (CH), 97.7 (CH), 58.91 (CH), 40.1 (CH₂), 30.3 (CH₂), 25.4 (CH₂), 23.6 (CH₂) ; SM (IS) m/z : 329.0 [M+H]⁺, 351.0 [M+Na]⁺, 657.5 [2M+H]⁺.

### 1-(4-Méthylpyridin-2-yl)-3-(6-oxo-1,2,3,4,6,10b-hexahydropyrido[2,1-a] isoindol-10-yl) urée (27).

Le composé **27** est obtenu sous forme de solide blanc selon la procédure générale A avec un rendement de 90 % après purification sur gel de silice (CH₂Cl₂ /MeOH : 99 /1). F : 178-180°C ; IR (ATR-Ge ν cm⁻¹) : 3305-3224 (N-H amide), 1651 (C=O amide), 1529- 1485-1427 (C=C arom), 1288 (C-N), 1200 (C-C), 723 (C-H arom) ; RMN ¹H *(*CDCl*₃* ; 400 MHz) δ 9.68 (s, 1H), 8.64 (d, *J* = 1.5 Hz, 1H), 7.96 (d, *J* = 7.8 Hz, 1 H), 7.61 (d, *J* = 7.4 Hz, 1 H), 7.41 (t, *J* = 7.8 Hz, 1 H), 7.11 (s, 1H), 4.57 - 4.38 (m, 2H), 2.97 - 2.91 (m, 1H), 2.65 (d, *J* = 10.9 Hz, 1H), 1.88 (d, *J* = 12.2 Hz, 1H), 1.76 (d, *J* = 12.3 Hz, 1H), 1.60 - 1.43 (m, 1H), 1.43 - 1.28 (m, 1H), 1.10 - 0.92 (m, 1H) ; RMN ¹³C *(*CDCl₃ ; 101 MHz) *δ* : 166.4 (Cq), 153.0 (Cq), 150.8 (CH), 149.9 (Cq), 136.6 (Cq), 133.5 (Cq), 133.3 (Cq), 129.2 (CH), 124.9 (CH), 119.4 (CH), 97.7 (CH), 58.9 (CH), 40.1 (CH₂), 30.3 (CH₂), 25.4 (CH₂), 23.6 (CH₂) ; SM (IS) m/z : 329.0 [M+H]⁺, 351.0 [M+Na]⁺, 657.5 [2M+H]⁺.

### 1-(6'-Oxo-3',4',6',10b'-tétrahydro-1'H-spiro[[1,3]dioxolane-2,2'-pyrido[2,1-a]isoindole]-10'-yl)-3-(pyridin-2-yl)urée (28).

Le composé 28 est obtenu sous forme de solide blanc selon la procédure générale A avec un rendement de 78 % après purification sur gel de silice (CH₂Cl₂ /MeOH : 99 /1). F : 178-180°C ; IR (ATR-Ge ν cm⁻¹) : 3305-3224 (N-H amide), 1651 (C=O amide), 1529- 1485-1427 (C=C arom), 1288 (C-N), 1200 (C-C), 723 (C-H arom) ; RMN ¹H (CDCl₃ ; 400 MHz) *δ* 9.68 (s, 1H), 8.64 (d, *J*= 1.5 Hz, 1H), 7.96 (d, *J* = 7.8 Hz, 1H), 7.61 (d, *J* = 7.4 Hz, 1H), 7.41 (t, *J* = 7.8 Hz, 1H), 7.11 (s, 1H), 4.57 - 4.38 (m, 2H), 2.97 - 2.91 (m, 1H), 2.65 (d, *J* = 10.9 Hz, 1H), 1.88 (d, *J* = 12.2 Hz, 1H), 1.76 (d, *J* = 12.3 Hz, 1H), 1.60 - 1.43 (m, 1H), 1.43 - 1.28 (m, 1H), 1.10 - 0.92 (m, 1H) ; RMN ¹³C (CDCl*₃* ; 101 MHz) *δ* 166.4 (Cq), 153.0 (Cq), 150.8 (CH), 149.9 (Cq), 136.6 (Cq), 133.5 (Cq), 133.3 (Cq), 129.2 (CH), 124.9 (CH), 119.4 (CH), 97.7 (CH), 58.9 (CH), 40.1 (CH₂), 30.3 (CH₂), 25.4 (CH₂), 23.6 (CH₂) ; SM (IS) m/z : 329.0 [M+H]⁺, 351.0 [M+Na]⁺, 657.5 [2M+H]⁺.

### 1-(6'-Oxo-3',4',6',10b'-tétrahydro-1'H-spiro[[1,3]dioxolane-2,2'-pyrido[2,1-a]isoindole]-10'-yl)-3-(pyrazin-2-yl)urée (29).

Le composé 29 est obtenu sous forme de solide marron clair selon la procédure générale A avec un rendement de 68 % après purification sur gel de silice (CH₂Cl₂ / MeOH : 99 /1). F > 260°C ; IR (ATR-Ge ν cm⁻¹) : 1698 (C=O amide), 1570 (N-H amide), 1549- 1504-1478 (C=C arom), 1298 (C-O), 1244 (C-N), 1073 (C-C) ; RMN ¹H *(*DMSO-*d6* ; 400 MHz) *δ* 10.12 (s, 1H), 10.03 (s, 1H), 8.88 (s, 1H), 8.34 - 8.25 (m, 2H), 8.13 (d, *J* = 7.8 Hz, 1H), 7.49 (t, *J* = 7.7 Hz, 1H), 7.43 (d, *J* = 6.9 Hz, 1H), 4.78 (dd, *J* = 3.6 Hz, *J* = 12.0 Hz, 1H), 4.28 (dd, *J* = 4.0 Hz, *J* = 13.3 Hz, 1H), 4.14 - 4.00 (m, 2H), 4.00 - 3.83 (m, 2H), 3.15 (td, *J* = 4.0 Hz, *J* = 13.1 Hz, 1H), 2.55 (d, *J* = 11.5 Hz, 1H), 1.83 (d, *J* = 13.1 Hz, 1H), 1.51 (td, *J* = 5.7 Hz, 13.0 Hz, 1H), 1.23 (t, *J* = 12.3 Hz, 1H) ; RMN ¹³C *(*DMSO-*d6* ; 101 MHz) *δ* 164.7 (Cq), 151.6 (Cq), 149.2 (Cq), 140.7 (CH), 137.8 (CH), 135.5 (CH), 134.8 (Cq), 133.4 (Cq), 132.8 (Cq), 129.0 (CH), 123.2 (CH), 118.0 (CH), 106.8 (Cq), 64.2 (CH₂), 63.9 (CH₂), 55.5 (CH), 38.0 (CH₂), 36.0 (CH₂), 33.1 (CH₂) ; SM (IS) m/z : 382.0 [M+H]⁺, 404.0 [M+Na]⁺, 763.5 [2M+H]⁺.

### 1-(6-Oxo-1,2,3,4,6,10b-hexahydropyrido[2,1-a]isoindol-10-yl)-3-(pyridin-2-yl)urée (30).

Le composé 30 est obtenu sous forme de solide blanc selon la procédure générale A avec un rendement de 90 % après purification sur gel de silice (CH₂Cl₂ /MeOH 97 / 3 + 1% Et₃N). F : 142°C ; IR (ATR-Ge ν cm⁻¹) : 3515 (NH amide), 1670 (C=O amide), 1603 (N-H amide), 1576-1478-1417 (C=C arom), 1315 (C-N) ; RMN ¹H *(*DMSO-*d6* ; 400 MHz) δ 11.16 (s, 1H), 10.00 (s, 1H), 8.29 (d, *J* = 4.0 Hz, 1H), 8.23 (d, *J* = 8.0 Hz, 1H), 7.85 - 7.74 (m, 1H), 7.45 (t, *J* = 7.7 Hz, 1H), 7.38 (dd, *J* = 0.8 Hz, *J* = 7.4 Hz, 1H), 7.33 (d, *J* = 8.3 Hz, 1H), 7.11 - 7.03 (m, 1H), 4.59 (dd, *J* = 3.3 Hz, *J* = 11.5 Hz, 1H), 4.26 (dd, *J* = 4.4 Hz, *J* = 13.0 Hz, 1H), 3.12 - 2.97 (m, 1H), 2.72 (dd, *J* = 3.0 Hz, *J* = 12.6 Hz, 1H), 1.91 (d, *J* = 13.3 Hz, 1H), 1.83 - 1.64 (m, 2H), 1.36-1.22 (m, 1H), 0.95 - 0.77 (qd, 1H, *J* = 13.0 Hz, *J* = 3.0 Hz) ; RMN ¹³C *(*DMSO-*d6* ; 101 MHz*) δ* 164.6 (Cq), 152.7 (Cq), 152.1 (Cq), 145.9 (CH), 139.2 (CH), 134.8 (Cq), 133.9 (Cq), 132.8 (Cq), 128.8 (CH), 122.2 (CH), 117.6 (CH), 117.3 (CH), 112.2 (CH), 57.3 (CH), 39.0 (CH₂), 30.0 (CH₂), 25.0 (CH₂), 23.0 (CH₂) ; SM (IS) m/z : 323.5 [M+H]⁺.

### 1-(5-Bromopyridin-2-yl)-3-(6'-oxo-3',4',6',10b'-tétrahydro-1'H-spiro[[1,3] dithiolane-2,2'-pyrido [2,1-a]isoindole]-10'-yl)urée (31).

Le composé **31** est obtenu sous forme de solide jaune selon la procédure générale A avec un rendement de 82 % après purification sur gel de silice (CH₂Cl₂ /MeOH : 99 /1). F : 240°C ; IR (ATR-Ge ν cm⁻¹) : 1700 (C=O amide), 1551 (N-H amide), 1481-1430 (C=C arom), 1367 (C-N), 1240- 1048 (C-C), 759 (C-Br) ; RMN ¹H *(*DMSO-*d6* ; 400 MHz) δ 10.69 (s, 1H), 10.10 (s, 1H), 8.57 (d, *J* = 2.4 Hz, 1H), 8.17 (d, *J* = 7.8 Hz, 1H), 8.01 (dd, *J* = 2.5 Hz, 8.9 Hz, 1H), 7.49 (t, *J* = 7.7 Hz, 1H), 7.43 (d, *J* = 6.8 Hz, 1H), 7.33 (d, *J* = 8.9 Hz, 1H), 4.78 (dd, *J* = 3.3 Hz, *J* = 11.6 Hz, 1H), 4.34 (dd, *J* = 3.2 Hz, *J* = 13.5 Hz, 1H), 3.50 - 3.27 (m, 4H), 3.17 (td, *J* = 3.2 Hz, *J* = 13.3 Hz, 1H), 2.92 (d, *J* = 11.7 Hz, 1H), 2.15 (d, *J* = 13.3 Hz, 1H), 1.95 (td, *J* = 5.0 Hz, *J* = 12.9 Hz, 1H), 1.66 (t, *J* = 12.2 Hz, 1H) ; RMN ¹³C *(*DMSO-*d6*; 101 MHz) *δ* 164.7 (Cq), 151.8 (2 x Cq), 147.1 (CH), 141.3 (CH), 134.2 (Cq), 133.6 (Cq), 132.3 (Cq), 129.1 (CH), 123.6 (CH), 117.9 (CH), 114.0 (CH), 111.4 (Cq), 65.7 (Cq), 56.8 (CH), 43.9 (CH₂), 40.7 (CH₂), 38.7 (CH₂), 38.1 (CH₂), 37.7 (CH₂) ; SM (IS) m/z : 492.5 [M+H]⁺.

### 1-(6-Oxo-1,2,3,4,6,10b-hexahydropyrido[2,1-a]isoindol-10-yl)-3-(pyrimidin-4-yl)urée (32).

Le composé 32 est obtenu sous forme de solide beige selon la procédure générale A avec un rendement de 76 % après purification sur gel de silice (CH₂Cl₂ /MeOH : 99 /1). F : 218°C ; IR (ATR-Ge ν cm⁻¹) : 1685 (C=O amide), 1559 (N-H amide), 1478-1435-1417- 1389 (C=C arom), 1310 (C-N) ; RMN ¹H *(*DMSO-*d6* ; 400 MHz) δ 10.21 (s, 1H), 10.14 (s, 1H), 8.84 (s, 1H), 8.58 (d, *J* = 5.6 Hz, 1H), 8.12 (d, *J* = 7.6 Hz, 1H), 7.54 (d, *J* = 5.4 Hz, 1H), 7.5 - 7.4 (m, 2H), 4.57 (d, *J* = 8.8 Hz, 1H), 4.26 (d, *J* = 9.6 Hz, 1H), 3.03 (t, *J* = 11.8 Hz, 1H), 2.64 (d, *J* = 10.9 Hz, 1H), 1.91 (d, J = 11.9 Hz, 1H), 1.80 - 1.61 (m, 2H), 1.38 - 1.12 (m, 1H), 0.91 - 0.82 (m, 1H) ; RMN ¹³C *(*DMSO-*d6* ; 101 MHz) δ 164.4 (Cq), 158.0 (Cq), 157.6 (CH), 157.3 (CH), 151.4 (Cq), 135.4 (Cq), 133.2 (Cq), 133.0 (Cq), 128.9 (CH), 122.8 (CH), 118.0 (CH), 108.6 (CH), 57.3 (CH), 39.0 (CH₂), 29.9 (CH₂), 25.0 (CH₂), 22.9 (CH₂) ; SM (IS) m/z : 324.0 [M+H]⁺, 647.5 [2M+H]⁺.

### 1-(6-Oxo-1,2,3,4,6,10b-hexahydropyrido[2,1-a]isoindol-10-yl)-3-(perchloropyridin-2-yl)urée (33).

Le composé **33** est obtenu sous forme de solide beige selon la procédure générale A avec un rendement de 60 % après purification sur gel de silice (CH₂Cl₂ /MeOH : 99 /1). F : 246°C ; IR (ATR-Ge ν cm⁻¹) : 1683 (C=O amide), 1598 (N-H amide), 1529-1461-1430 (C=C arom), 1278 (C-N), 1221 (C-C), 750-732 (C-Cl) ; RMN ¹H *(*DMSO-*d6* ; 400 MHz) δ 9.60 (d, *J* = 2.6 Hz, 2H), 7.97 (dd, *J* = 1.2 Hz, *J* = 7.3 Hz, 1H), 7.49 - 7.44 (m, 2H), 4.58 (dd, *J* = 3.3 Hz, *J* = 11.5 Hz, 1H), 4.25 (dd, *J* = 3.8 Hz, *J* = 12.6 Hz, 1H), 3.02 (td, *J* = 3.8 Hz, *J* = 12.0 Hz, 1H), 2.61 (d, *J* = 10.2 Hz, 1H), 1.88 (d, *J* = 13.0 Hz, 1H), 1.75 (d, *J* = 12.3 Hz, 1H), 1.64 (q, *J* = 12.8 Hz, 1H), 1.29 - 1.15 (m, 1H), 0.92 - 0.84 (m, 1H) ; RMN ¹³C *(*DMSO-*d6* ; 101 MHz) δ 164.4 (Cq), 150.4 (Cq), 147.3 (Cq), 143.9 (Cq), 143.0 (Cq), 136.2 (Cq), 133.1 (Cq), 133.0 (Cq), 128.7 (CH), 124.0 (CH), 122.3 (Cq), 119.2 (Cq), 118.4 (CH), 57.3 (CH), 39.1 (CH₂), 29.7 (CH₂), 25.0 (CH₂), 23.0 (CH₂) ; SM (IS) m/z : 461.0 [M+H]⁺, 483.0 [M+Na]⁺.

### 1-(Benzo[d]thiazol-2-yl)-3-(6-oxo-1,2,3,4,6,10b-hexahydropyrido[2,1-a]isoindol-10-yl)urée (34).

Le composé 34 est obtenu sous forme de solide jaune pâle selon la procédure générale A avec un rendement de 50 % après purification sur gel de silice (CH₂Cl₂ /MeOH : 99 /1). F : 260°C ; IR (ATR-Ge ν cm⁻¹) : 1688 (C=O amide), 1597 (N-H amide), 1561-1524-1482 (C=C arom), 1286 (C-N) ; RMN ¹H *(*DMSO-*d6* ; 400 MHz) δ 11.19 (s, 1H), 9.07 (s, 1H), 8.05 (d, *J* = 7.0 Hz, 1H), 7.93 (d, *J* = 7.7 Hz, 1H), 7.69 (d, *J* = 6.6 Hz, 1H), 7.51 - 7.39 (m, 3H), 7.26 (t, *J* = 7.5 Hz, 1H), 4.57 (d, *J* = 9.4 Hz, 1H), 4.26 (d, *J* = 9.2 Hz, 1H), 3.07 - 3.01 (m, 1H), 2.61 (d, *J* = 11.5 Hz, 1H), 1.90 (d, *J* = 12.8 Hz, 1H), 1.76 (d, *J* = 12.4 Hz, 1H), 1.64 (q, *J* = 13.0 Hz, 1H), 1.32 - 1.15 (m, 1H), 0.88 (q, *J* = 12.0 Hz, 1H) ; RMN ¹³C *(*DMSO-*d6* ; 101 MHz) δ 164.4 (Cq), 135.8 (2 x Cq), 133.2 (2 x Cq), 133.1 (Cq), 128.8 (CH), 126.0 (CH), 123.3 (CH + Cq), 123.0 (CH), 121.6 (CH), 120.0 (CH), 118.3 (CH), 57.3 (CH), 54.8 (Cq), 39.0 (CH₂), 29.8 (CH₂), 24.9 (CH₂), 23.0 (CH₂) ; SM (IS) m/z : 379.5 [M+H]⁺.

### 1-(2-Méthylthiazol-4-yl)-3-(6-oxo-1,2,3,4,6,10b-hexahydropyrido[2,1-a]isoindol-10-yl)urée (35).

Le composé 35 est obtenu sous forme de solide jaune pâle selon la procédure générale A avec un rendement de 42 % après purification sur gel de silice (CH₂Cl₂ /MeOH : 99 /1). F : 146°C ; IR (ATR-Ge ν cm⁻¹) : 1691 (C=O amide), 1650 (N-H amide), 1532- 1484-1429 (C=C arom), 1286 (C-N), 1202 (C-C) ; RMN ¹H *(*CDCl₃ ; 400 MHz) δ 9.30 (s, 2H), 8.03 (d, *J* = 6.5 Hz, 1H), 7.62 (d, *J* = 7.3 Hz, 1H), 7.41 (t, *J* = 7.8 Hz, 1H), 6.70 (s, 1H), 4.51 (dd, *J* = 4.0 Hz, *J* = 13.2 Hz, 1H), 4.45 (dd, *J* = 3.5 Hz, *J* = 11.6 Hz, 1H), 2.95 (t, *J* = 12.0 Hz, 1H), 2.71 (s, 4H), 1.92 (d, *J* = 12.6 Hz, 1H), 1.78 (d, *J* = 12.6 Hz, 1H), 1.62 - 1.48 (m, 1H), 1.47 - 1.31 (m, 1H), 1.04 (q, *J* = 12.0 Hz, 1H) ; RMN ¹³C *(*CDCl₃; 101 MHz) δ 166.3 (Cq), 164.6 (Cq), 152.9 (Cq), 147.9 (Cq), 136.2 (Cq), 133.5 (Cq), 133.4 (Cq), 129.2 (CH), 124.7 (CH), 119.4 (CH), 96.6 (CH), 58.69 (CH), 40.1 (CH₂), 30.3 (CH₂), 25.4 (CH₂), 23.9 (CH₂), 19.3 (CH₃) ; SM (IS) m/z : 343.0 [M+H]⁺, 685.5 [2M+H]⁺.

### 1-(6-Méthylpyridin-2-yl)-3-(6-oxo-1,2,3,4,6,10b-hexahydropyrido[2,1-a]isoindol-10-yl)urée (36).

Le composé 36 est obtenu sous forme de solide blanc selon la procédure générale A avec un rendement de 73 % après purification sur gel de silice (CH₂Cl₂ /MeOH : 99 /1). F : 210°C ; IR (ATR-Ge ν cm⁻¹) : 1685 (C=O amide), 1572 (N-H amide), 1510-1464-1438 (C=C arom), 1283 (C-N), 757 (C-H arom) ; RMN ¹H *(*CDCl₃ ; 400 MHz) δ 1.95 (s, 1H), 9.56 (s, 1H), 8.13 (d, *J* = 8.0 Hz, 1H), 7.67 (d, *J* = 7.5 Hz, 1H), 7.56 (t, *J* = 7.8 Hz, 1H), 7.49 (t, *J* = 7.8 Hz, 1H), 6.84 - 6.76 (m, 2H), 4.65 - 4.46 (m, 2H), 3.00 (td, *J* = 3.5 Hz, *J* = 13.1 Hz, 1H), 2.71 (dd, *J* = 3.2 Hz, 13.0 Hz, 1H), 1.96 (d, *J* = 13.3 Hz, 1H), 1.81 (d, *J* = 13.0 Hz, 1H), 1.63 (q, *J* = 13.2 Hz, 1H), 1.51 - 1.34 (m, 1H), 1.10 (qd, *J* = 3.2 Hz, *J* = 13.0 Hz, 1H) ; RMN ¹³C *(*CDCl₃ ; 101 MHz) δ 166.1 (Cq), 155.0 (Cq), 154.2 (Cq), 152.6 (Cq), 139.2 (CH), 136.4 (Cq), 133.6 (Cq), 133.3 (Cq), 129.1 (CH), 125.2 (CH), 119.6 (CH), 117.0 (CH), 109.6 (CH), 58.6 (CH), 40.0 (CH₂), 29.9 (CH₂), 25.4 (CH₂), 24.5 (CH₃), 23.9 (CH₂) ; SM (IS) m/z : 337.5 [M+H]⁺.

### 1-(6-Oxo-1,2,3,4,6,10b-hexahydropyrido[2,1-a]isoindol-10-yl)-3-(2-(pyridin-4-yl)thiazol-4-yl)urée (37).

Le composé 37 est obtenu sous forme de solide orange pâle selon la procédure générale A avec un rendement de 60 % après purification sur gel de silice (CH₂Cl₂ / MeOH : 99 /1). F : 152°C ; IR (ATR-Ge ν cm⁻¹) : 1649 (C=O amide), 1561 (N-H amide), 1521- 1485-1430 (C=C arom), 1201 (C-C) ; RMN ¹H *(*DMSO-*d6*; 400 MHz) δ 10.10 (s, 1H), 8.72 (dd, *J* = 1.5 Hz, *J* = 4.6 Hz, 2H), 8.48 (s, 1H), 8.11 (d, *J* = 7.2 Hz, 1H), 7.84 (dd, *J* = 1.6 Hz, *J* = 4.5 Hz, 2H), 7.55 (s, 1H), 7.45 (t, *J* = 7.7 Hz, 1H), 7.38 (dd, *J* = 0.8 Hz, *J* = 7.4 Hz, 1H), 4.50 (dd, *J* = 3.3 Hz, *J* = 11.5 Hz, 1H), 4.26 (dd, *J* = 3.7 Hz, *J* = 13.0 Hz, 1H), 3.03 (td, *J* = 3.7 Hz, *J* = 12.9 Hz, 1H), 2.62 (dd, *J* = 2.9 Hz, *J* = 12.6 Hz, 1H), 1.90 (d, *J* = 13.1 Hz, 1H), 1.76 (d, *J* = 12.6 Hz, 1H), 1.62 (q, *J* = 13.0 Hz, 1H), 1.34 - 1.15 (m, 1H), 0.85 (qd, *J* = 2.9 Hz, *J* = 12.7 Hz, 1H) ; RMN ¹³C *(*DMSO-*d6* ; 101 MHz) δ 164.5 (Cq), 161.4 (Cq), 151.6 (Cq), 150.8 (2 x CH), 149.6 (Cq), 139.2 (Cq), 134.9 (Cq), 134.00 (Cq), 132.9 (Cq), 128.7 (CH), 122.3 (CH), 119.4 (2 x CH), 117.3 (CH), 100.4 (CH), 57.3 (CH), 39.0 (CH₂), 29.9 (CH₂), 25.0 (CH₂), 23.0 (CH₂) ; SM (IS) m/z : 406.5 [M+H]⁺.

### 1-(6-Oxo-1,2,3,4,6,10b-hexahydropyrido[2,1-a]isoindol-10-yl)-3-(pyrazin-2-yl)urée (38).

Le composé **38** est obtenu sous forme de solide beige selon la procédure générale A avec un rendement de 70 % après purification sur gel de silice (CH₂Cl₂ /MeOH 96 / 4). F : 215°C ; IR (ATR-Ge ν cm⁻¹) : 1699-1683 (C=O amide), 1671 (C=N), 1651 (N-H amide), 1558-1540-1520-1507 (C=C arom) ; RMN ¹H *(*DMSO-*d6* ; 400 MHz) δ 10.05 (s, 1H), 9.81 (s, 1H), 8.93 (d, *J* = 1.3 Hz, 1H), 8.36 - 8.31 (m, 1H), 8.29 (d, *J* = 2.7 Hz, 1H), 8.13 (dd, *J* = 1.0 Hz, *J* = 7.9 Hz, 1H), 7.47 (t, *J* = 7.7 Hz, 1H), 7.42 (dd, *J* = 1.0 Hz, *J* = 7.4 Hz, 1H), 4.56 (dd, *J* = 3.4 Hz, *J* = 11.5 Hz, 1H), 4.26 (dd, *J* = 3.8 Hz, *J* = 13.0 Hz, 1H), 3.03 (td, *J* = 3.8 Hz, *J* = 12.8 Hz, 1H), 2.63 (dd, *J* = 3.0 Hz, *J* = 12.6 Hz, 1H), 1.91 (d, *J* = 13.1 Hz, 1H), 1.76 (d, *J* = 15.0 Hz, 1H), 1.72 - 1.64 (m, 1H), 1.33 - 1.16 (m, 1H), 0.88 (qd, *J* = 3.0 Hz, *J* = 12.7 Hz, 1H) ; RMN ¹³C *(*DMSO-*d6* ; 101 MHz) δ 164.4 (Cq), 151.6 (Cq), 149.1 (Cq), 141.0 (CH), 137.8 (CH), 135.4 (CH), 135.3 (Cq), 133.5 (Cq), 132.9 (Cq), 128.8 (CH), 122.8 (CH), 117.8 (CH), 57.3 (CH), 39.0 (CH₂), 29.9 (CH₂), 25.01 (CH₂), 23.0 (CH₂) ; SM (IS) m/z : 324.0 [M+H]⁺

### 1-(5-méthylpyrazin-2-yl)-3-(6-oxo-1,2,3,4,6,10b-hexahydropyrido[2,1-a] isoindol-10-yl)urée (39).

Le composé 39 est obtenu selon la procédure A à partir de l'amine 10 sous forme de solide blanc avec 80 % de rendement après chromatographie sous gel de silice flash (CH₂Cl₂ / MeOH : 99 /1). F : 220°C ; IR (ATR-Ge ν cm⁻¹) : 1687 (C=O amide), 1560 (N-H amide), 1503-1483-1416 (C=C arom), 1342 (C-N), 1155 (C-C) ; RMN ¹H *(*DMSO-*d6* ; 400 MHz) δ 9.92 (s, 1H), 9.74 (s, 1H), 8.82 (s, 1H), 8.20 (s, 1H), 8.13 (d, *J* = 7.8Hz, 1H), 7.46 (t, *J* = 7.7 Hz, 1H), 7.40 (d, *J* = 7.2 Hz, 1H), 4.54 (dd, *J* = 3.1 Hz, 11.5 Hz, 1H), 4.26 (dd, *J* = 4.1 Hz, 13.0 Hz, 1H), 3.09 - 2.95 (m, 1H), 2.63 (d, *J* = 10.1 Hz, 1H), 2.44 (s, 3H), 1.91 (d, *J* = 13.2 Hz, 1H), 1.82 - 1.60 (m, 2H), 1.33 - 1.15 (m, 1H), 0.94 - 0.77 (m, 1H) ; RMN ¹³C *(*DMSO-*d6* ; 101 MHz) δ 164.5 (Cq), 151.7 (Cq), 146.9 (Cq), 146.2 (Cq), 139.9 (CH), 135.1 (Cq), 134.0 (CH), 133.6 (Cq), 132.9 (Cq), 128.8 (CH), 122.6 (CH), 117.6 (CH), 57.3 (CH), 39.0 (CH₂), 29.9 (CH₂), 25.0 (CH₂), 23.0 (CH₂), 20.1 (CH₃). SM (IS) m/z : 338.5 [M+H]⁺, 675.5 [2M+H]⁺

### 1-(6'-Oxo-3',4',6',10b'-tétrahydro-1'H-spiro[[1,3]dithiolane-2,2'-pyrido[2,1-a]isoindole]-10'-yl)-3-(1-Méthyl-1H-pyrazol-3-yl)urée (40).

Le composé **40** a été obtenu selon la procédure **A** après purification par chromatographie flash (CH₂Cl₂ / MeOH : 99 /1) sous la forme d'un solide jaune. Rendement: 68%. p.f. 135°C ; IR (ATR-Ge ν cm⁻¹) : 3256 (N-H amide), 2923 (Cₛₚ₃-H), 1676 (C=O amide), 1530-1485 (C=C arom), 1287 (C-N) ; ¹H RMN (CDCl₃ ; 400 MHz) δ 10.08 (s, 1H), 8.82 (s, 1H), 7.89 (d, *J* = 8.0 Hz, 1H), 7.61 (d, *J* = 7.4 Hz, 1H), 7.42 (t, *J* = 7.7 Hz, 1H), 7.23 (d, *J* = 2.2 Hz, 1H), 5.91 (s, 1H), 4.81 (dd, *J* = 11.4 Hz, 3.1 Hz, 1H), 4.52 (dd, *J* = 13.8 Hz, 3.8 Hz, 1H), 3.87 (s, 3H), 3.33 - 3.16 (m, 5H), 2.95 (d, *J* = 12.5 Hz, 1H), 2.17 (d, *J* = 12.1 Hz, 1H), 2.01 (td, *J* = 13.0 Hz, 5.0 Hz, 1H), 1.62 (t, *J* = 12.0 Hz, 1H) ; ¹³C RMN (CDCl₃ ; 101 MHz) δ 166.3 (Cq), 153.5 (Cq), 148.5 (Cq), 136.0 (Cq), 133.3 (Cq), 133.3 (Cq), 131.4 (CH), 129.3 (CH), 126.3 (CH), 119.8 (CH), 94.65 (CH), 65.8 (Cq), 57.8 (CH), 45.0 (CH₂), 41.0 (CH₂), 39.2 (CH₃), 38.9 (CH₂), 38.9 (CH₂), 38.4 (CH₂) ; HRMS : calculé pour C₁₉H₂₁N₅O₂NaS₂ 438,1034 ; trouvé 438,1025

### 1-(1-Méthyl-1H-pyrazol-3-yl)-3-(6-oxo-1,2,3,4,6,10b-hexahydro-pyrido[2,1-a]isoindol-10-yl)-urée (41).

Le composé **41** a été obtenu selon la procédure **A** après purification par chromatographie flash (CH₂Cl₂ / MeOH : 99 /1) sous la forme d'un solide blanc. Rendement : 62%. p.f. 233°C ; IR (ATR-Ge ν cm⁻¹) : 3083 (Cₛₚ₂-H arom), 2937 (Cₛₚ₃-H), 1686 (C=O amide), 1598-1481 (C=C arom), 1284 (C-N) ; ¹H RMN (DMSO-*d6* ; 400 MHz) δ 9.42 (s, 1H), 9.21 (s, 1H), 8.18 (d, *J* = 7.3 Hz, 1H), 7.59 (s, 1H), 7.43 (t, *J* = 7.7 Hz, 1H), 7.35 (d, *J* = 7.2 Hz, 1H), 6.07 (s, 1H), 4.52 (d, *J* = 8.9 Hz, 1H), 4.26 (d, *J* = 9.5 Hz, 1H), 3.78 (s, 3H), 3.03 (t, *J* = 11.6 Hz, 1H), 2.71 (d, *J* = 11.1 Hz, 1H), 1.92 (d, *J* = 13.1 Hz, 1H), 1.76 (d, *J* = 12.5 Hz, 1H), 1.74 - 1.55 (m, 1H), 1.25 (d, *J* = 12.6 Hz, 1H), 0.87 (dd, *J* = 23.0 Hz, 11.8 Hz, 1H) ; ¹³C RMN (DMSO-*d6*; 101 MHz) δ 164.6 (Cq), 151.7 (Cq), 147.7 (Cq), 134.5 (Cq), 134.4 (Cq), 132.8 (Cq), 131.7 (CH), 128.7 (CH), 122.1 (CH), 117.0 (CH), 94.3 (CH), 57.3 (CH), 39.0 (CH₂), 38.3 (CH₃), 29.8 (CH₂), 25.0 (CH₂), 23.1 (CH₂) ; HRMS : calc. pour C₁₇H₁₉N₅O₂Na 348,1436 ; trouvé 348,1431.

### 1-(6-Oxo-1,2,3,4,6,10b-hexahydro-pyrido[2,1-a]isoindol-10-yl)-3-pyrimidin-2-yl-urée (42).

Le composé **42** a été obtenu selon la procédure **A** après purification par chromatographie flash (CH₂Cl₂ / MeOH : 99 /1) sous la forme d'un solide beige. Rendement: 87 %. p.f. 232°C ; IR (ATR-Ge ν cm⁻¹) : 3063 (Cₛₚ₂-H arom), 2913 (Cₛₚ₃-H), 1691 (C=O amide), 1582-1484 (C=C arom), 1291 (C-N); ¹H RMN *(*DMSO-*d6* ; 400 MHz) δ 11.60 (s, 1H), 10.42 (s, 1H), 8.70 (d, *J* = 4.9 Hz, 2H), 8.24 (d, *J* = 7.8 Hz, 1H), 7.48 (t, *J* = 7.7 Hz, 1H), 7.42 (d, *J* = 7.2 Hz, 1H), 7.20 (t, *J* = 4.9 Hz, 1H), 4.64 (dd, *J* = 11.5 Hz, 3.2 Hz, 1H), 4.27 (dd, *J* = 12.8 Hz, 4.5 Hz, 1H), 3.11 - 2.96 (m, 1H), 2.68 (d, *J* = 10.2 Hz, 1H), 1.92 (d, *J* = 13.1 Hz, 1H), 1.75 (t, *J* = 13.2 Hz, 2H), 1.25 (dd, *J* = 13.3 Hz, 9.3 Hz, 1H), 0.90 (dt, *J* = 14.5 Hz, 12.0 Hz, 1H) ; ¹³C RMN (DMSO-*d6* ; 101 MHz) δ 164.5 (Cq), 158.2 (2CH), 157.7 (Cq), 151.4 (Cq), 135.2 (Cq), 133.6 (Cq), 132.9 (Cq), 128.9 (CH), 122.4 (CH), 117.7 (CH), 115.3 (CH), 57.2 (CH), 39.0 (CH₂), 30.0 (CH₂), 25.0 (CH₂), 23.0 (CH₂) ; HRMS : calc. pour C₁₇H₁₈N₅O₂ 324,1461 ; trouvé 324,1449.

### 1-(6-Méthyl-pyrazin-2-yl)-3-(6-oxo-1,2,3,4,6,10b-hexahydro-pyrido[2,1-a]isoindol-10-yl)-urée (43).

Le composé **43** a été obtenu selon la procédure **A** après purification par chromatographie flash (CH₂Cl₂ / MeOH : 99 /1) sous la forme d'un solide beige. Rendement : 82%. p.f. 142°C ; IR (ATR-Ge ν cm⁻¹) : 3207 (N-H amide), 2948 (Cₛₚ₃-H), 1665 (C=O amide), 1594-1487 (C=C arom), 1280 (C-N) ; ¹H RMN (DMSO-*d6* ; 400 MHz) δ 10.00 (s, 1H), 9.61 (s, 1H), 8.72 (s, 1H), 8.27 - 8.05 (m, 2H), 7.54 - 7.33 (m, 2H), 4.55 (dd, *J* = 11.5 Hz, 3.1 Hz, 1H), 4.26 (dd, *J* = 12.7 Hz, 3.7 Hz, 1H), 3.10 - 2.93 (m, 1H), 2.60 (d, *J* = 10.5 Hz, 1H), 2.46 (s, 3H), 1.89 (d, *J* = 13.1 Hz, 1H), 1.75 (d, *J* = 12.5 Hz, 1H), 1.62 (dd, *J* = 26.1 Hz, 13.1 Hz, 1H), 1.25 (dd, *J* = 17.4 Hz, 8.4 Hz, 1H), 0.88 (dd, 3*J* = 23.4 Hz, 11.1 Hz, 1H) ; ¹³C RMN (DMSO-*d6* ; 101 MHz) δ 164.5 (Cq), 151.7 (Cq), 150.3 (Cq), 148.3 (Cq), 137.2 (CH), 135.3 (Cq), 133.5 (Cq), 132.9 (Cq), 131.9 (CH), 128.8 (CH), 123.1 (CH), 117.8 (CH), 57.3 (CH), 39.1 (CH₂), 29.8 (CH₂), 25.0 (CH₂), 23.1 (CH₂), 20.7 (CH₃) ; HRMS : calc. pour C₁₈H₂₀N₅O₂ 338,1617 ; trouvé 338,1621.

### 1-(3-Méthyl-pyrazin-2-yl)-3-(6-oxo-1,2,3,4,6,1.0b-hexahydro-pyrido[2,1-a]isoindol-10-yl)-urée (44).

Le composé **44** a été obtenu selon la procédure **A** après purification par chromatographie flash (CH₂Cl₂ / MeOH : 99 /1) sous la forme d'un solide blanc. Rendement : 70% p.f. 228°C ; IR (ATR-Ge ν cm⁻¹) : 3255 (N-H amide), 2935 (Cₛₚ₃-H), 1677 (C=O amide), 1597-1481 (C=C arom), 1280 (C-N) ; ¹H RMN (CDCl₃, 400 MHz) δ 11.53 (s, 1H), 8.19 (d, *J* = 2.8 Hz, 1H), 8.14 (d, *J* = 8.0 Hz, 1H), 8.01 (d, *J* = 2.7 Hz, 1H), 7.69 - 7.58 (m, 2H), 7.47 (t, *J* = 7.8 Hz, 1H), 4.54 (dd, *J* = 13.3 Hz, 4.8 Hz, 1H), 4.46 (dd, *J* = 11.7 Hz, 3.5 Hz, 1H), 3.01 (td, *J* = 13.0 Hz, 3.5 Hz, 1H), 2.74 - 2.57 (m, 4H), 2.03 (d, *J* = 13.4 Hz, 1H), 1.84 (d, *J* = 13.3 Hz, 1H), 1.77 - 1.63 (m, 1H), 1.52 - 1.35 (m, 1H), 1.10 (qd, J = 13.0 Hz, 3.3 Hz, 1H) ; ¹³C RMN *(*CDCl₃ ; 101 MHz) δ 166.0 (Cq), 152.3 (Cq), 147.4 (Cq), 143.2 (Cq), 137.1 (CH), 136.8 (CH), 136.1 (Cq), 133.7 (Cq), 132.8 (Cq), 129.4 (CH), 124.3 (CH), 120.0 (CH), 58.5 (CH), 40.0 (CH₂), 30.5 (CH₂), 25.4 (CH₂), 24.1 (CH₂), 20.6 (CH₃). HRMS : calculé pour C₁₈H₂₀N₅O₂ 338,1617 ; trouvé 338,1629.

### 1-(3-Méthylpyridin-2-yl)-3-(6'-oxo-3',4',6',10b'-tétrahydro-1'H-spiro[[1,3] dioxolane-2,2'-pyrido [2,1-i]isoindole]-10'-yl)urée (45).

Le composé **45** a été obtenu selon la procédure **A** avec un rendement de 72%, sous la forme d'un solide blanc. p.f. > 260°C ; IR (ATR-Ge ν cm⁻¹) : 1679 (C=O amide), 1566 (N-H amide), 1480-1415 (C=C arom), 1291 (C-N), 1135 (C-C). ¹H RMN (DMSO- *d*₆, 400 MHz) *δₚₚₘ:* 12.4 (s, 1H), 8.85 (s, 1H), 8.33 (d, *J* = 7.5 Hz, 1H), 8.23 (d, *J* = 7.5 Hz, 1H), 7.69 (d, *J* = 7.0 Hz, 1H), 7.39-7.50 (m, 2H), 7.09 (dd, *J* = 7.0 Hz , *J* = 8.0 Hz, 1H), 4.84 (dd, *J* = 3.3 Hz, *J* = 11.5 Hz, 1H), 4.30 (dd, *J* = 3.2 Hz, *J* = 13.7 Hz, 1H), 3.81 - 4.07 (m, 4H), 3.17 (td, *J* = 3.2 Hz, *J* = 13.3 Hz, 1H), 2.62 (d, *J* = 11.5 Hz, 1H), 3.32 (s, 3H), 1.86 (d, *J* = 12.5 Hz, 1H), 1.53 (td, *J* = 5.0 Hz, *J* = 12.5 Hz, 1H), 1.35 (t, *J* = 12.5 Hz, 1H). ¹³C RMN (DMSO- *d*₆, 100 MHz) : 166.2 (Cq), 152.7 (Cq), 151.6 (Cq), 145.5 (CH), 143.9 (Cq), 143.3 (Cq), 137.2 (CH), 134.5 (Cq), 133.0 (Cq), 129.9 (CH), 129.2 (CH), 121.7 (CH), 112.7 (CH), 107.3 (Cq), 64.6 (CH₂), 64.3 (CH₂), 55.7 (CH), 37.8 (CH₂), 36.4 (CH₂), 34.4 (CH₂), 17.5 (CH₃). HRMS : calc. pour C₂₁ H₂₃ N₄ O₄ : 395,1719 ; trouvé : 395,1731.

### 1-(6-Méthylpyridin-2-yl)-3-(6'-oxo-3',4',6',10b'-tétrahydro-1'H-spiro[[1,3] dioxolane-2,2'-pyrido[2,1-a]isoindole]-10'-yl)urée (46).

Le composé **46** a été obtenu selon la procédure **A** avec un rendement de 75%, sous la forme d'un solide blanc. p.f. 240-242°C; IR (ATR-Ge ν cm⁻¹) : 3180 (N-H amine), 1685 (C=O amide), 1584-1560-1510 (C=C arom), 1327 (C-N), 1287 (C-C), 751 (C-H arom). ¹H RMN (CDCl₃ , 400 MHz) *δₚₚₘ*: 12.14 (s, 1H), 9.16 (s, 1H), 7.99 (d, *J =* 7.5 Hz, 1H), 7.67 (d, *J =* 7.5 Hz, 1H), 7.47-7.59 (m, 2H), 6.82 (d, *J =* 7.5 Hz, 1H), 6.73 (d, *J =* 7.5 Hz, 1H), 4.92 (dd, *J =* 2.5 Hz, *J =* 12.5 Hz, 1H), 4.52 (dd, *J* = 5.0 Hz, *J* = 12.5 Hz, 1H), 3.77 - 3.97 (m, 4H), 3.27 (td, *J* = 2.5 Hz, *J* = 12.5 Hz, 1H), 2.60 (s, 3H), 2.53 (d, *J* = 12.5 Hz, 1H), 1.79 (d, *J* = 12.5 Hz, 1H), 1.64 (td, *J* = 5.0 Hz, *J =* 12.5 Hz, 1H), 1.33 (t, *J =* 12.5 Hz, 1H). ¹³C RMN (CDCl₃ , 100 MHz) : 166.0 (Cq), 155.4 (Cq), 153.9 (Cq), 152.4 (Cq), 139.1 (CH), 136.1 (Cq), 133.2 (Cq), 132.8 (Cq), 129.1 (CH), 125.8 (CH), 119.7 (CH), 116.7 (CH), 109.3 (CH), 107.4 (Cq), 64.5 (CH₂), 64.4 (CH₂), 56.4 (CH), 38.1 (CH₂), 36.5 (CH₂), 34.0 (CH₂), 23.8 (CH₃). HRMS : calc. pour C₂₁ H₂₃ N₄ O₄ : 395,1719 ; trouvé : 395,1733.

### 1-(6-Bromopyridin-2-yl)-3-(6'-oxo-3',4',6',10b'-tétrahydro-1'H-spiro[[1,3] dioxolane-2,2'-pyrido[2,1-a]isoindole]-10'-yl)urée (47).

Le composé **47** a été obtenu selon la procédure **A** avec un rendement de 60%, sous la forme d'un solide blanc. p.f. 184-186°C; IR (ATR-Ge ν cm⁻¹) : 2977 (N-H amine), 1685 (C=O amide), 1565- 1511 -1434 (C=C arom), 1326 (C-N), 1289 (C-C), 752 (C-H arom) ¹H RMN (CDCl₃ , 400 MHz) *δₚₚₘ*: 10.97 (s, 1H), 9.88 (s, 1H), 7.87 (d, *J* = 7.5 Hz, 1H), 7.72 (d, *J =* 7.5 Hz, 1H), 7.47-7.55 (m, 2H), 7.15 (d, *J* = 7.5 Hz, 1H), 7.03 (d, *J =* 10.0 Hz, 1H), 4.99 (dd, *J =* 2.5 Hz, *J =* 12.5 Hz, 1H), 4.53 (dd, *J* = 2.5 Hz, *J* = 12.5 Hz, 1H), 3.86 - 3.96 (m, 4H), 3.27 (td, *J* = 2.5 Hz, *J* = 12.5 Hz, 1H), 2.54 (d, *J* = 15.0 Hz, 1H), 1.79 (d, *J* = 12.5 Hz, 1H), 1.68-1.95 (m, 2H), 1.34 (t, *J* = 12.5 Hz, 1H). ¹³C RMN (CDCl₃ , 100 MHz) : 165.9 (Cq), 153.4 (Cq), 152.9 (Cq), 140.7 (CH), 137.9 (Cq), 136.9 (Cq), 133.5 (Cq), 132.1 (Cq), 129.1 (CH), 126.4 (CH), 121.1 (CH), 120.4 (CH), 110.9 (CH), 107.3 (Cq), 64.7 (CH₂), 64.6 (CH₂), 56.5 (CH), 38.2 (CH₂), 36.6 (CH₂), 34.2 (CH₂). HRMS : calc. pour C₂₀ H₂₀ N₄ O₄ Br : 459,0668 ; trouvé 459,0685.

### 1-(4-Méthoxyquinolin-2-yl)-3-(6'-oxo-3',4',6',10b'-tétrahydro-1'H-spiro[[1,3] dioxolane-2,2'-pyrido[2,1-a]isoindole]-10'-yl)urée (48).

Le composé **48** a été obtenu selon la procédure **A** avec un rendement de 72%, sous la forme d'un solide jaune. p.f. 258-260°C; IR (ATR-Ge ν cm⁻¹) : 2967 (N-H amine), 1689 (C=O amide), 1621-1585-1414 (C=C arom), 1332 (C-N), 1289 (C-C), 751 (C-H arom). ¹H RMN (CDCl₃ , 400 MHz) δₚₚₘ : 12.51 (s, 1H), 8.95 (s, 1H), 8.23 (d, *J =* 7.5 Hz, 1H), 8.10 (d, *J =* 7.5 Hz, 1H), 7.95 (d, *J =* 7.5 Hz, 1H), 7.67-7.73 (m, 2H), 7.38-7.49 (m, 2H), 6.47 (s, 1H), 5.01 (dd, *J =* 2.5 Hz, *J =* 12.5 Hz, 1H), 4.56 (dd, *J* = 2.5 Hz, *J* = 12.5 Hz, 1H), 4,02 (s, 3H), 3.57 - 3.79 (m, 3H), 3.27-3.39 (m, 2H), 2.72 (d, *J =* 15.0 Hz, 1H), 1.79 (d, *J =* 12.5 Hz, 1H), 1.70 (td, *J =* 5.0 Hz, *J =* 12.5 Hz, 1H), 1.38 (t, *J =* 12.5 Hz, 1H). ¹³C RMN (CDCl₃ , 100 MHz) : 166.0(Cq), 163.9 (Cq), 154.3 (Cq), 153.3(Cq), 145.8 (Cq), 135.6 (Cq), 133.3 (Cq), 133.0 (Cq), 130.5 (CH), 128.8 (CH), 126.5 (CH), 125.2 (CH), 124.1 (CH), 121.8 (CH), 119.6 (CH), 118.8 (Cq), 107.2 (Cq), 91.5 (CH), 64.6 (CH₂), 64.5 (CH₂), 56.5 (CH), 55.7 (CH₃), 38.3 (CH₂), 36.5 (CH₂), 34.1 (CH₂). HRMS : calc. pour C₂₅ H₂₅ N₄ O₅ : 461,1825 ; trouvé 461,1844.

### 1-(4-Méthoxyquinolin-2-yl)-3-(6'-oxo-3',4',6',10b'-tétrahydro-1'H-spiro[[1,3] dithiolane-2,2'-pyrido[2,1-a]isoindole]-10'-yl)urée (49).

Le composé **49** a été obtenu selon la procédure **A** avec un rendement de 80%, sous la forme d'un solide jaune. p.f. 250-252°C ; IR (ATR-Ge ν cm⁻¹) : 2975 (N-H amine), 1685 (C=O amide), 1621-1585-1414 (C=C arom), 1332 (C-N), 1289 (C-C), 751 (C-H arom). ¹H RMN (CDCl₃ , 400 MHz) *δₚₚₘ* : 12.56 (s, 1H), 10.01 (s, 1H), 8.08 (d, *J =* 7.5 Hz, 1H), 8.00 (dd, *J =* 7.5 Hz, *J =* 8.0 Hz, 2H), 7.72 (d, *J =* 7.5 Hz, 1H), 7.64 (dd, *J* = 7.5 Hz, *J* = 8.0 Hz, 1H), 7.49 (d, *J* = 7.5 Hz, 1H), 7.39 (dd, *J* = 7.5 Hz, *J =* 8.0 Hz, 1H), 6.48 (s, 1H), 5.07 (dd, *J =* 2.5 Hz, *J =* 12.5 Hz, 1H), 4.58 (dd, *J =* 2.5 Hz, *J =* 12.5 Hz, 1H), 4.00 (s, 3H), 3.21 (td, *J =* 5.0 Hz, *J =* 12.5 Hz, 1H), 2.78-3.13 (m, 4H), 2.47-2.57 (m, 1H), 2.19 (d, *J =* 12.5 Hz, 1H), 2.06 (td, *J =* 5.0 Hz, *J =* 12.5 Hz, 1H), 1.68 (t, *J* = 12.5 Hz, 1H). ¹³C RMN (CDCl₃ , 100 MHz) : 166.0 (Cq), 163.9 (Cq), 154.3 (Cq), 153.3 (Cq), 145.8 (Cq), 136.3 (Cq), 133.4 (Cq), 132.8 (Cq), 130.4 (CH), 128.7 (CH), 127.0 (CH), 126.8 (CH), 124.2 (CH), 121.8 (CH), 120.2 (CH), 118.7 (Cq), 91.5 (CH), 65.3 (Cq), 57.7 (CH₂), 55.7 (CH₃), 45.4 (CH₂), 40.1 (CH₂), 39.0 (CH₂), 38.3 (CH₂), 38.1 (CH₂). HRMS : calc. pour C₂₅ H₂₅ N₄ O₃ S₂: 493,1368 ; trouvé: 493,1384.

### 1-(6-Méthylpyridin-2-yl)-3-(6'-oxo-3',4',6',10b'-tétrahydro-1'H-spiro[[1,3] dithiolane-2,2'-pyrido[2,1-a]isoindole]-10'-yl)urée (50).

Le composé **50** a été obtenu selon la procédure **A** avec un rendement de 78%, sous la forme d'un solide blanc. p.f. 154-156°C ; IR (ATR-Ge ν cm⁻¹) : 2922 (N-H amine), 1689 (C=O amide), 1622-1596-1429 (C=C arom), 1355 (C-N), 1290 (C-C), 741 (C-H arom). ¹H RMN (CDCl₃ , 400 MHz) δₚₚₘ : 12.05 (s, 1H), 9.48 (s, 1H), 7.75 (d, *J* = 8.0 Hz, 1H), 7.68 (d, *J =* 8.0 Hz, 1H), 7.58-7.65 (m, 1H), 7.47 (dd, *J =* 7.5 Hz, *J* = 8.0 Hz, 1H), 6.84 (d, *J =* 8.0 Hz, 2H), 4.92 (dd, *J =* 2.5 Hz, *J =* 12.5 Hz, 1H), 4.52 (dd, *J =* 2.5 Hz, *J =* 12.5 Hz, 1H), 3.14-3.29 (m, 5H), 3.88 (d, *J =* 12.5 Hz, 1H), 2.60 (s, 3H), 2.18 (d, *J =* 12.5 Hz, 1H), 2.03 (td, *J =* 5.0 Hz, *J =* 12.5 Hz, 1H), 1.61 (t, *J* = 12.5 Hz, 1H). ¹³C RMN (CDCl₃ , 100 MHz) : 165.8 (Cq), 154.0 (Cq), 152.1 (Cq), 139.7 (Cq), 136.8 (CH), 133.4 (CH), 132.4(Cq), 129.1 (2 CH), 127.0 (Cq), 120.4 (CH), 116.8 (CH), 109.7 (Cq), 65.5 (Cq), 57.8 (CH) , 45.1 (CH₂), 40.5 (CH₂), 38.8 (CH₂), 38.6 (CH₂), 38.3 (CH₂), 24.8 (CH₃). HRMS : calc. pour C₂₁H₂₃N₄O₂S₂ : 427,1262 ; trouvé 427,1278.

### 1-(5-Méthylpyrazin-2-yl)-3-(6'-oxo-3',4',6',10b'-tétrahydro-1'H-spiro[[1,31 dithiolane-2,2'-pyrido [2,1-a]isoindole]-10'-yl)urée (51).

Le composé **51** a été obtenu selon la procédure **A** avec un rendement de 85%, sous la forme d'un solide blanc. p.f. > 260°C ; IR (ATR-Ge ν cm⁻¹) : 2977 (N-H amine), 1688 (C=O amide), 1621-1586-1414 (C=C arom), 1332 (C-N), 1288 (C-C), 753 (C-H arom). ¹H RMN (DMSO- d₆, 400 MHz) *δₚₚₘ*: 10.03 (s, 2H), 8.73 (s, 1H), 8.26 (s, 1H), 8.07 (d, *J =* 7.5 Hz, 1H), 7.42-7.52 (m, 2H), 4.75 (dd, *J =* 2.8 Hz, *J =* 12.2 Hz, 1H), 4.32 (dd, *J =* 2.8 Hz, *J =* 12.2 Hz, 1H), 3.38-3.43 (m, 1H), 3.27-3.34 (m, 3H), 3.14 (t, *J =* 12.5 Hz, 1H), 2.86 (d, *J =* 12.5 Hz, 1H), 2.42 (s, 3H), 2.12 (d, *J* = 12.8 Hz, 1H), 1.93 (td, *J* = 5.0 Hz, J = 12.5 Hz, 1H), 1.62 (t, *J* = 12.5 Hz, 1H). ¹³C RMN (DMSO- d₆, 100 MHz) : 164.5 (Cq), 151.7 (Cq), 146.8 (Cq), 145.8 (Cq), 139.5 (CH), 134.5 (Cq), 134.2 (CH), 133.4 (Cq), 132.6 (Cq), 128.9 (CH), 123.8 (Cq), 118.0 (CH), 65.5 (Cq), 56.8 (CH), 43.9 (CH₂), 40.4 (CH₂), 38.5 (CH₂), 38.1 (CH₂), 37.7 (CH₂), 20.0 (CH₃). *HRMS* : calc. pour C₂₀H₂₂N₅O₂S₂: 428,1215 ; trouvé 428,1229.

### 1-(4-Méthoxyquinolin-2-yl)-3-(6-oxo-1,2,3,4,6,10b-hexahydropyrido[2,1-a] isoindol-10-yl) urée (52).

Le composé **52** a été obtenu selon la procédure **A** avec un rendement de 80%, sous la forme d'un solide blanc. p.f. > 260°C ; IR (ATR-Ge ν cm⁻¹) : 1692 (C=O amide), 1588 (N-H amide), 1521-1480-1416 (C=C arom), 1286 (C-O). ¹H RMN (DMSO- *d*₆, 400 MHz) *δₚₚₘ*: 11.98 (s, 1H), 10.15 (s, 1H), 8.28 (d, *J =* 7.5 Hz, 1H), 8.05 (d, *J =* 8.2 Hz, 1H), 7.90 (d, *J =* 8.2 Hz, 1H), 7.77 (dd, *J =* 7.5 Hz, *J =* 8.2 Hz, 1H), 7.43-7.54 (m, 3H), 6.85 (s, 1H), 4.85 (dd, *J =* 2.5 Hz, *J =* 12.5 Hz, 1H), 4.30 (dd, *J =* 2.8 Hz, *J =* 12.5 Hz, 1H), 4.03 (s, 3H), 3.13 (td, *J =* 2.8 Hz, *J =* 12.5 Hz, 1H), 2.66 (d, *J* = 12.5 Hz, 1H), 1.77 (d, *J* = 12.5 Hz, 2H), 1.60-1.66 (m, 1H), 1.22-1.28 (m, 1H), 0.95 (q, *J =* 12.5 Hz, 1H). ¹³C RMN (DMSO- *d*₆, 100 MHz) : 165.1 (Cq), 163.6 (Cq), 154.3 (Cq), 152.8 (Cq), 146.4 (Cq), 136.2 (Cq), 134.2 (Cq), 133.6 (Cq), 131.0 (CH), 129.1 (CH), 126.4 (CH), 124.4 (CH), 124.3 (CH), 122.0 (CH), 118.7 (Cq), 118.3 (CH), 92.6 (CH), 57.9 (CH), 56.5 (CH₃), 39.6 (CH₂), 30.2 (CH₂), 25.5 (CH₂), 23.5 (CH₂). HRMS : calc. pour C₂₃ H₂₃ N₄ O₃ : 403,1770 ; trouvé 403,1763

### 1-(2-Methoxy-6-oxo-1,2,3,4,6,10b-hexahydropyrido[2,1-a]isoindol-10-yl)-3-(pyridin-2-yl)urée (53).

Le composé **53** a été obtenu selon la procédure **A** avec un rendement de 81%, sous la forme d'un solide jaune. p.f. 166-168°C ; IR (ATR-Ge ν cm⁻¹) : 1688 (C=O amide), 1602 (C=O cétone), 1579 (N-H amide), 1478- 1418 (C=C arom), 1312 (C-N), 1291 (C-C), 751 (C-H arom) ; ¹H *RMN* (CDCl₃ , *400 MHz) δₚₚₘ*: 11.98 (s, 1H), 8.66 (s, 1H), 7.26 (d, *J =* 7.5 Hz, 1H), 8.15 (d, *J =* 8.0 Hz, 1H), 7.70 (dd, *J =* 7.5 Hz, *J =* 8.0 Hz, 1H), 7.64 (d, *J =* 7.2 Hz, 1 H), 7.49 (dd, *J =* 7.5 Hz, *J =* 8.0 Hz, 1H), 7.01 (dd, *J =* 7.5 Hz, *J =* 8.0 Hz, 1H), 6.90 (d, *J =* 8.0 Hz, 1H), 4.60 (dd, *J =* 3.2 Hz, *J =* 12.5 Hz, 2H), 3.58-3.66 (m, 1H), 3.36 (s, 3H), 3.00-3.09 (m, 2H), 2.21 (d, *J =* 12.5 Hz, 1H), 1.13-1.42 (m, 1H), 1.04 (t, *J =* 12.5 Hz, 1H). *¹³C RMN (*CDCl₃ , *100 MHz) δₚₚₘ*: 166.2 (Cq), 153.5 (Cq), 152.9 (Cq), 145.7 (CH), 139.4 (CH), 135.2 (Cq), 133.4 (Cq), 133.3 (Cq), 129.5 (CH), 124.7 (CH), 119.7 (Cq), 117.9 (CH), 112.6 (CH), 77.6 (CH), 57.1 (CH), 56.1 (CH₃), 45.5 (CH₂), 35.7 (CH₂), 30.9 (CH₂). HRMS : calc. pour C₁₉ H₂₁ N₄ O₃ : 353,1614 ; trouvé 353,1603

### 1-(2-Méthoxy-6-oxo-1,2,3,4,6,10b-hexahydropyrido[2,1-a]isoindol-10-yl)-3-(pyridin-2-yl) urée (54).

Le composé **54** a été obtenu selon la procédure **A** avec un rendement de 78%, sous la forme d'un solide jaune. p.f. 192-194°C; IR (ATR-Ge ν cm⁻¹) : 1684 (C=O amide), 1595 (C=O cétone), 1557 (N-H amide), 1486- 1421 (C=C arom), 1306 (C-N), 1084 (C-C), 753 (C-H arom). ¹H RMN (DMSO- d₆, 400 MHz) δₚₚₘ : 10.08 (s, 1H), 9.79 (s, 1H), 8.95 (s, 1H), 8.30-8.35 (m, 2H), 8.14 (d, *J* = 8.0 Hz, 1H), 7.48 (dd, *J* = 7.8 Hz, *J* = 8.0 Hz, 1H), 7.41 (d, *J* = 8.0 Hz, 1H), 4.66 (dd, *J* = 3.2 Hz, *J* = 12.5 Hz, 1H), 4.28 (dd, *J =* 4.0 Hz, *J =* 13.5 Hz, 1H), 3.64-3.69 (m, 1H), 3.28 (s, 3H), 3.08 (td, *J* = 3.2 Hz, *J*= 12.5 Hz, 1H), 2.91 (d, *J=* 13.5 Hz, 1H), 2.13 (d, *J=* 12.5 Hz, 1H), 1.07-1.18 (m, 1H), 0.74 (q, *J =* 12.5 Hz, 1H). ¹³C RMN (DMSO- d₆, 100 MHz) δₚₚₘ: 164.5 (Cq), 151.6 (Cq), 149.1 (Cq), 141.1 (CH), 137.9 (CH), 135.4 (CH), 134.5 (Cq), 133.5 (Cq), 132.8 (Cq), 129.0 (CH), 123.0 (CH), 117.9 (CH), 76.2 (CH), 55.8 (CH), 55.1 (CH₃), 36.4 (CH₂), 35.2 (CH₂), 30.6 (CH₂). HRMS: calc. pour C₁₈ H₁₉ N₅ O₃ Na : 376,1386 ; trouvé 376,1370.

### 1-(2-Méthoxy-6-oxo-1,2,3,4,6,10b-hexahydropyrido[2,1-a]isoindol-10-yl)-3-(4-méthoxyqui-nolin-2-yl)urée (55).

Le composé **55** a été obtenu selon la procédure **A** avec un rendement de 80%, sous la forme d'un solide blanc. p.f. > 260°C. IR (ATR-Ge ν cm⁻¹) : 1694 (C=O amide), 1610 (C=O cétone), 1556 (N-H amide), 1479-1416 (C=C arom), 1338 (C-N), 1295 (C-C), 759 (C-H arom). ¹H RMN (DMSO-d₆, 400 MHz) δₚₚₘ : 11.99 (s, 1H), 10.15 (s, 1H), 8.30 (d, *J =* 8.0 Hz, 1H), 8.02 (d, *J =* 8.0 Hz, 1H), 7.92 (d, *J =* 8.0 Hz, 1H), 7.77 (dd, *J =* 7.5 Hz, *J =* 8.0 Hz, 1H), 7.41-7.51 (m, 3H), 6.84 (s, 1H), 4.92 (dd, *J* = 3.2 Hz, *J =* 12.0 Hz, 1H), 4.30 (dd, *J* = 4.0 Hz, *J* = 13.5 Hz, 1H), 4.01 (s, 3H), 3.59 (d, *J =* 12.0 Hz, 1H), 3.15 (td, *J =* 3.2 Hz, *J =* 12.5 Hz, 1H), 2.91 (d, *J =* 13.5 Hz, 1H), 2.82 (s, 3H), 2.09 (d, *J =* 12.5 Hz, 1H), 1.05-1.15 (m, 1H), 0.76 (q, *J =* 12.5 Hz, 1H). ¹³C RMN (DMSO-*d*₆, 100 MHz) δₚₚₘ : 164.6 (Cq), 162.9 (Cq), 153.7 (Cq), 152.3 (Cq), 145.6 (Cq), 134.3 (Cq), 133.7 (Cq), 132.8 (Cq), 130.7 (CH), 128.9 (CH), 126.3 (CH), 124.1 (CH), 123.5 (Cq), 121.5 (CH), 118.0 (Cq), 117.8 (CH), 91.9 (CH), 76.3 (CH), 56.0 (CH), 55.8 (CH₃), 54.7 (CH₃), 36.4 (CH₂), 34.4 (CH₂), 30.7 (CH₂). HRMS : calc. pour C₂₄ H₂₅ N₄ O₄ : 433,1876 ; trouvé 433,1884.

### 1-(2-Méthoxy-6-oxo-1,2,3,4,6,10b-hexahydropyrido[2,1-a]isoindol-10-yl)-3-(6-methylpyri-din-2-yl)urée (56).

Le composé **56** a été obtenu selon la procédure **A** avec un rendement de 75%, sous la forme d'un solide blanc. p.f. 167-169°C. IR (ATR-Ge ν cm⁻¹) : 1689 (C=O amide), 1589 (C=O cétone), 1556 (N-H amide), 1436 (C=C arom), 1283 (C-N), 751 (C-H arom). ¹H RMN (CDCl₃ , 400 MHz) δₚₚₘ : 11.98 (s, 1H), 8.93 (s, 1H), 7.99 (d, *J =* 8.0 Hz, 1H), 7.66 (d, *J =* 8.0 Hz, 1H), 7.55 (dd, *J =* 7.5 Hz, *J =* 8.0 Hz, 1H), 7.49 (dd, *J* = 7.5. Hz, *J* = 8.0 Hz, 1H), 6.83 (d, *J* = 7.5 Hz, 1H), 6.70 (d, *J* = 8.0 Hz, 1H), 4.69 (dd, *J =* 3.2 Hz, *J =* 12.0 Hz, 1H), 4.58 (dd, *J =* 4.5 Hz, *J =* 13.5 Hz, 1H), 3.52-3.59 (m, 1H), 3.30 (s, 3H), 3.02 (td, *J =* 3.2 Hz, *J =* 12.5 Hz, 1H), 2.93 (d, *J* = 12.5 Hz, 1H), 2.56 (s, 3H), 2.19 (d, *J* = 12.5 Hz, 1H), 1.27-1.38 (m, 1H), 1.03 (q, *J* = 12.5 Hz, 1H). ¹³C RMN (CDCl₃ , 100 MHz) δₚₚₘ : 166.2 (Cq), 155.1 (Cq), 153.9 (Cq), 152.5 (Cq), 139.4 (CH), 136.0 (Cq), 133.5 (Cq), 133.1 (Cq), 129.4 (CH), 125.9 (CH), 120.1 (CH), 117.1 (CH), 109.5 (CH), 77.4 (CH), 57.3 (CH), 56.0 (CH₃), 37.3 (CH₂), 35.3 (CH₂), 30.8 (CH₂), 24.6 (CH₃). HRMS : calc. pour C₂₀ H₂₃ N₄ O₃: 367,1770 ; trouvé 367,1776.

### 1-(2-Méthoxy-6-oxo-1,2,3,4,6,10b-hexahydropyrido[2,1-a]isoindol-10-yl)-3-(3-méthylpyridin-2-yl)urée (57).

Le composé **57** a été obtenu selon la procédure **A** avec un rendement de 73%, sous la forme d'un solide jaune. p.f. 222-224°C. IR (ATR-Ge ν cm⁻¹) : 1692 (C=O amide), 1592 (C=O cétone), 1554 (N-H amide), 1484- 1421 (C=C arom), 1298 (C-N), 1189 (C-C), 751 (C-H arom). ¹H RMN (CDCl₃, 400 MHz) *δₚₚₘ*: 12.24 (s, 1H), 8.13-8.16 (m, 2H), 7.61 (d, *J =* 7.5 Hz, 1H), 7.53 (d, *J =* 7.5 Hz, 1H), 7.46 (dd, *J =* 7.5 Hz, *J =* 8.0 Hz, 1H), 7.10 (s, 1H), 6.95 (dd, *J =* 7.5 Hz, *J =* 8.0 Hz, 1H), 4.58 (dd, *J =* 3.2 Hz, *J =* 12.0 Hz, 2H), 3.60-3.65 (m, 1H), 3.36 (s, 3H), 3.03 (td, *J =* 3.2 Hz, *J* = 12.5 Hz, 2H), 2.31 (s, 3H), 2.20 (d, *J* = 12.5 Hz, 1H), 1.30-1.40 (m, 1H), 1.02 (q, *J* = 12.5 Hz, 1H). ¹³C RMN (CDCl₃, 100 MHz) (δ_{ppm :} 166.2 (Cq), 152.7 (Cq), 151.2 (Cq), 143.2 (CH), 139.9 (CH), 135.1 (Cq), 133.3 (Cq), 133.2 (Cq), 129.5 (CH), 124.6 (CH), 119.8 (Cq), 119.6 (CH), 117.9 (CH), 77.6 (CH), 57.1 (CH), 56.1 (CH₃), 37.2 (CH₂), 35.7 (CH₂), 30.9 (CH₂), 17.1 (CH₃). HRMS : calc. pour C₂₀ H₂₃ N₄ O₃: 367,1770 ; trouvé 367,1778.

### 1-(2-Méthoxy-6-oxo-1,2,3,4,6,10b-hexahydropyrido[2,1-a]isoindol-10-yl)-3-(4-méthylpyridin-2-yl)urée (58).

Le composé a été obtenu selon la procédure **A** avec un rendement de 76%, sous la forme d'un solide jaune. p.f. 218-220°C. IR (ATR-Ge ν cm⁻¹) : 1690 (C=O amide), 1619 (C=O cétone), 1573 (N-H amide), 1481- 1439 (C=C arom), 1310 (C-N), 1293 (C-C), 752 (C-H arom). ¹H RMN (DMSO- d₆, 400 MHz) δₚₚₘ: 11.41 (s, 1H), 9.88 (s, 1H), 8.26 (d, *J =* 8.0 Hz, 1H), 8.18 (d, *J =* 7.5 Hz, 1H), 7.45 (dd, *J =* 7.5 Hz, *J* = 8.0 Hz, 1H), 7.36 (d, *J* = 7.5 Hz, 1H), 7.08 (s, 1H), 6.92 (d, *J* = 7.5 Hz, 1H), 4.69 (dd, *J =* 3.2 Hz, *J =* 12.0 Hz, 1H), 4.28 (dd, *J =* 3.2 Hz, *J =* 12.0 Hz, 1H), 3.68-3.77 (m, 1H), 3.24 (s, 3H), 3.08 (td, *J* = 3.2 Hz, *J* = 12.5 Hz, 2H), 2.29 (s, 3H), 2.13 (d, *J =* 12.0 Hz, 1H), 1.07-1.18 (m, 1H), 0.75 (q, *J =* 12.0 Hz, 1H). ¹³C RMN (DMSO- d₆, 100 MHz) δₚₚₘ: 164.7 (Cq), 152.9 (Cq), 152.2 (Cq), 150.0 (Cq), 145.6 (CH), 134.0 (Cq), 133.9 (Cq), 132.7 (Cq), 129.1 (CH), 122.3 (CH), 118.9 (CH), 117.4 (CH), 112.1 (CH), 76.3 (CH), 55.8 (CH), 55.2 (CH₃), 36.5 (CH₂), 35.2 (CH₂), 30.7 (CH₂), 20.8 (CH₃). HRMS : calc. pour C₂₀ H₂₃ N₄ O₃ : 367,1770 ; trouvé 367,1761.

### 1-(2-Méthoxy-6-oxo-1,2,3,4,6,10b-hexahydropyrido[2,1-a]isoindol-10-yl)-3-(5-méthylpyrazin-2-yl)urée (59).

Le composé **59** a été obtenu selon la procédure **A** avec un rendement de 76%, sous la forme d'un solide jaune. p.f. 140-142°C. IR (ATR-Ge ν cm⁻¹) : 1688 (C=O amide), 1598 (C=O cétone), 1554 (N-H amide), 1484- 1438 (C=C arom), 1344 (C-N), 1291 (C-C), 753 (C-H arom). ¹H RMN (CDCl₃ , 400 MHz) *δₚₚₘ*: 11.16 (s, 1H), 9.80 (s, 1H), 8.42 (s, 1H), 8.11 (d, *J* = 8.0 Hz, 1H), 8.02 (s, 1H), 7.66 (d, *J =* 7.5 Hz, 1H), 7.49 (dd, *J* = 7.5 Hz, *J* = 8.0 Hz, 1H), 4.53-4.62 (m, 2H), 3.58-3.64 (m, 1H), 3.38 (s, 3H), 2.96-3.06 (m, 2H), 2.54 (s, 3H), 2.22 (d, *J =* 12.5 Hz, 1H), 1.30-1.40 (m, 1H), 1.06 (q, *J =* 12.0 Hz, 1H). ¹³C RMN (CDCl₃ , 100 MHz) *δₚₚₘ*: 165.8 (Cq), 153.4 (Cq), 146.7 (Cq), 146.6 (Cq), 137.7 (CH), 135.1 (CH), 135.0 (Cq), 133.3 (Cq), 132.6 (Cq), 129.5 (CH), 124.6 (CH), 120.0 (CH), 77.3 (CH), 56.9 (CH), 55.9 (CH₃), 37.0 (CH₂), 35.7 (CH₂), 30.5 (CH₂), 20.6 (CH₃). HRMS : calc. pour C₁₉ H₂₂ N₅ O₃ : 368,1723 ; trouvé 368,1722.

### 1-(2-Méthoxy-6-oxo-1,2,3,4,6,10b-hexahydropyrido[2,1-a]isoindol-10-yl)-3-(1-méthyl-1H-pyrazol-3-yl)urée (60).

Le composé **60** a été obtenu selon la procédure **A** avec un rendement de 81%, sous la forme d'un solide jaune. p.f. 196-198 °C; IR (ATR-Ge ν cm⁻¹) : 1686 (C=O amide), 1625 (C=O cétone), 1538 (N-H amide), 1482- 1421 (C=C arom), 1316 (C-N), 1284 (C-C), 751 (C-H arom). *¹H RMN* (CDCl₃ , *400 MHz) δₚₚₘ*: 10.06 (s, 1H), 8.17 (s, 1H), 8.05 (d, *J =* 8.0 Hz, 1H), 7.62 (d, *J =* 7.5 Hz, 1H), 7.45 (dd, *J =* 7.5 Hz, *J =* 8.0 Hz, 1H), 7.27 (s, 1H), 5.85 (s, 1H), 4.56-4.60 (m, 2H), 3.87 (s, 3H), 3.52-3.60 (m, 1H), 3.36 (s, 3H), 3.97-3.08 (m, 2H), 2.21 (d, *J =* 12.5 Hz, 1H), 1.27-1.38 (m, 1H), 1.06 (q, *J =* 12.5 Hz, 1H). *¹³C RMN (*CDCl₃ , 100 *MHz) δₚₚₘ* : 166.0 (Cq), 152.8 (Cq), 148.2 (Cq), 135.2 (Cq), 133.3 (Cq), 133.2 (Cq), 131.4 (CH), 129.2 (CH), 125.0 (CH), 119.5 (CH), 94.1 (Cq), 77.2 (CH), 56.9 (CH), 55.7 (CH₃), 38.7 (CH₃), 37.0 (CH₂), 35.7 (CH₂), 30.3 (CH₂). HRMS : calc. pour C₁₈ H₂₁ N₅ O₃ Na : 378,1542 ; trouvé 378,1542.

### 1-(5-(Méthoxyméthoxy)quinolin-2-yl)-3-(6'-oxo-3',4',6',10b'-tetrahydro-1'H-spiro[[1,3]dioxolane-2,2'-pyrido[2,1-a]isoindole]-10'-yl)urée

Le composé **61** a été obtenu selon la procédure **A** avec un rendement de 78%, sous la forme d'un solide blanc. p.f. 192-194°C. IR (ATR-Ge ν cm⁻¹) : 1690 (C=O amide), 1601 (C=O cétone), 1525 (N-H amide), 1422 (C=C arom), 1332 (C-N), 1298 (C-C), 778 (C-H arom). ¹H RMN (CDCl₃ , 400 MHz) δₚₚₘ: 12.44 (s, 1H), 10.26 (s, 1H), 8.28 (d, *J =* 8.0 Hz, 1H), 8.03 (d, *J =* 8.0 Hz, 1H), 7.91 (d, *J =* 8.0 Hz, 1H), 7.58-7.65 (m, 2H), 7.41 (dd, *J =* 7.6 Hz, *J =* 8.0 Hz, 1H), 7.33 (dd, *J =* 7.6 Hz, *J* = 7.6 Hz, 1H), 6.73 (s, 1H), 5.34-5.39 (m, 2H), 4.92 (dd, *J =* 4.0 Hz, *J =* 12.8 Hz, 1H), 4.47 (dd, *J =* 4.8 Hz, *J =* 13.6 Hz, 1H), 3.50-3.68 (m, 3H), 3.49 (s, 3H), 3.21-3.27 (m, 2H), 2.63 (d, *J* = 11.6 Hz, 1H), 1.70 (d, *J* = 13.6 Hz, 1H), 1.61 (td, *J* = 4.8 Hz, 12.8 Hz, 1H), 1.27 (t, *J* = 12.8 Hz, 1H). ¹³C RMN (CDCl₃ , 100 MHz) δₚₚₘ: 166.1 (Cq), 161.3 (Cq), 154.4 (Cq), 153.4 (Cq), 146.0 (Cq), 135.3 (Cq), 133.1 (2 Cq), 130.4 (CH), 129.0 (2 CH), 126.7 (CH), 125.1 (CH), 124.1 (CH), 121.8 (CH), 119.3 (CH), 118.8 (Cq), 107.2 (Cq), 94.6 (CH₂), 64.6 (CH₂), 64.5 (CH₂), 56.9 (CH₃), 56.5 (CH), 38.3 (CH₂), 36.5 (CH₂), 34.3 (CH₂). MS (IS) m/z : 491,0 [M+H]⁺.

### 2.2. Procédure générale B

### Synthèse des dérivés de thiourées (62)-(74) à partir d'isothiocyanates (11) ou (12).

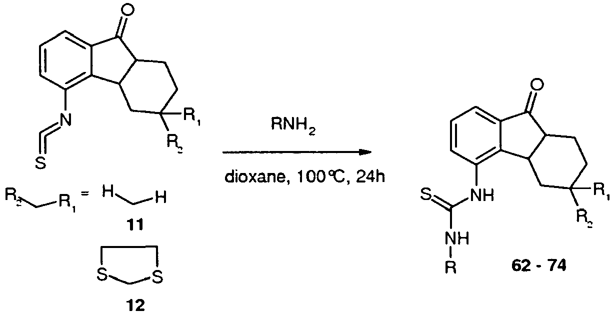

Dans un ballon de 25 ml, l'isothiocyanate (0.6 mmol, **11** ou **12**) est dissous dans 10 ml de dioxane anhydre puis l'amine (0.6 mmol, 1 éq.) est ajoutée. Le mélange réactionnel est chauffé à 100°C sous agitation pendant 24h puis est refroidi avant d'éliminer le solvant sous pression réduite. Le brut réactionnel est purifié par chromatographie sur gel de silice pour donner les thiourées attendues.

### 1-(6-Oxo-1,2,3,4,6,10b-hexahydropyrido[2,1-a]isoindol-10-yl)-3-(pyridin-2-yl)thiourée (62)

Le composé **62** est obtenu sous forme de solide blanc selon la procédure générale **B** avec un rendement de 90 % après purification sur gel de silice (CH₂Cl₂ /MeOH : 99 /1). F : 195°C ; IR (ATR-Ge ν cm⁻¹) : 3223 (N-H amine), 1679 (C=O amide), 1597- 1565- 1526 (C=C arom), 1346 (C-N), 1286 (C-C), 776 (C-H arom) ; RMN ¹H (CDCl₃ ; 400 MHz) *δ* 13.63 (s, 1H), 9.67 (s, 1H), 8.21 (dd, *J =* 1.2 Hz, *J* = 5.1 Hz, 1H), 7.85 (dd, *J* = 7.6 Hz, *J* = 15.5 Hz, 2H), 7.76 - 7.68 (m, 1H), 7.53 (t, *J* = 7.7 Hz, 1H), 7.10 - 7.00 (m, 2H), 4.61 (dd, *J* = 3.6 Hz, *J* = 11.7 Hz, 1H), 4.52 (dd, *J* = 4.1 Hz, *J =* 13.3 Hz, 1H), 2.99 (td, *J* = 4.1 Hz, *J* = 13.0 Hz, 1H), 2.47 (dd, *J* = 3.1 Hz, *J =* 12.9 Hz, 1H), 1.92 (d, *J =* 13.5 Hz, 1H), 1.79 (d, *J =* 13.1 Hz, 1H), 1.68 - 1.53 (m, 1H), 1.34 - 1.46 (m, 1H), 1.22 - 1.09 (m, 1H) ; RMN ¹³C (CDCl*₃* ; 101 MHz) *δ* 179.7 (Cq), 165.8 (Cq), 153.3 (Cq), 145.6 (CH), 141.0 (Cq), 139.5 (CH), 134.1 (Cq), 133.7 (Cq), 129.7 (CH), 129.0 (CH), 122.6 (CH), 118.8 (CH), 112.9 (CH), 58.8 (CH), 39.9 (CH₂), 30.6 (CH₂), 25.4 (CH₂), 23.9 (CH₂) ; SM (IS) m/z : 339.5 [M+H]⁺, 677.5 [2M+H]⁺.

### 1-(6-Oxo-1,2,3,4,6,10b-hexahydropyrido[2,1-a]isoindol-10-yl)-3-(pyrazin-2-yl)thiourée (63).

Le composé **63** est obtenu sous forme de solide marron clair selon la procédure générale B avec un rendement de 62 % après purification sur gel de silice (CH₂Cl₂ / MeOH : 99 /1). F *:* 204°C ; IR (ATR-Ge ν cm⁻¹) : 3010 (N-H amine), 1686 (C=O amide), 1591- 1560- 1514 (C=C arom), 1416 (C-N), 1136 (C-C) ; RMN ¹H (DMSO-*d6* ; 400 MHz) *δ* 12.80 (s, 1H), 11.40 (s, 1H), 8.68 (d, *J =* 1.0 Hz, 1H), 8.40 - 8.29 (m, 2H), 7.93 (d, *J* = 7.7 Hz, 1H), 7.62 (d, *J* = 6.9 Hz, 1H), 7.54 (t, *J* = 7.7 Hz, 1H), 4.65 (dd, *J* = 3.5 Hz, *J* = 11.6 Hz, 1H), 4.24 (dd, *J* = 3.7 Hz, *J* = 13.0 Hz, 1H), 3.00 (td, *J* = 3.7 Hz, *J* = 12.9 Hz, 1H), 2.34 (dd, *J* = 3.0 Hz, *J* = 12.7 Hz, 1H), 1.82 (d, *J =* 12.8 Hz, 1H), 1.73 (d, *J =* 13.0 Hz, 1H), 1.58 (q, *J =* 13.1 Hz, 1H), 1.31 - 1.19 (m, 1H), 0.97 (qd, *J* = 3.0 Hz, *J* = 12.7 Hz, 1H) ; RMN ¹³C (DMSO-*d6* ; 101 MHz) *δ* 179.3 (Cq), 164.3 (Cq), 149.3 (Cq), 140.7 (Cq), 139.4 (CH), 138.1 (CH), 136.5 (CH), 133.8 (Cq), 133.2 (Cq), 129.3 (CH), 128.6 (CH), 121.1 (CH), 57.7 (CH), 39.0 (CH₂), 30.1 (CH₂), 24.9 (CH₂), 23.0 (CH₂) ; SM (IS) m/z : 340.5 [M+H]⁺, 679.5 [2M+H]⁺.

### 1-(6-Oxo-1,2,3,4,6,10b-hexahydropyrido[2,1-a]isoindol-10-yl)-3-(1H-pyrazol-3-yl)thiourée (64).

Le composé **64** est obtenu sous forme de solide beige selon la procédure générale B avec un rendement de 56 % après purification sur gel de silice (CH₂Cl₂ /MeOH : 99 /1). F : 208°C ; IR (ATR-Ge ν cm⁻¹) : 3190 (NH amine), 1659 (C=O amide), 1579- 1522- 1454-1424 (C=C arom), 1258- 1016 (C-C), 762 (C-H arom) ; RMN ¹H *(*DMSO-*d6* ; 400 MHz) *δ* 12.70 (s, 1H), 11.64 (s, 1H), 10.91 (s, 1H), 7.93 (t, *J =* 8.0 Hz, 1H), 7.76 (d, *J =* 2.3 Hz, 1H), 7.59 (d, *J =* 6.8 Hz, 1H), 7.51 (t, *J =* 7.7 Hz, 1H), 6.05 (s, 1H), 4.60 (dd, *J* = 3.5 Hz, *J* = 11.6 Hz, 1H), 4.24 (dd, *J* = 3.8 Hz, *J* = 13.0 Hz, 1H), 2.98 (td, *J* = 3.8 Hz, *J* = 12.9 Hz, 1H), 2.39 (d, *J* = 12.4 Hz, 1H), 1.82 (d, *J* = 13.4 Hz, 1H), 1.73 (d, *J* = 12.7 Hz, 1H), 1.60 (q, *J* = 13.1 Hz, 1H), 1.31 -1.18 (m, 1H), 0.95 (qd, *J* = 3.2 Hz, *J* = 12.7 Hz, 1H) ; RMN ¹³C *(*DMSO-*d6* ; 101 MHz) δ 164.7 (Cq), 149.4 (Cq), 140.8 (Cq), 140.4 (Cq), 134.3 (Cq), 133.2 (Cq), 130.0 (CH), 129.6 (CH), 128.7 (CH), 121.0 (CH), 94.4 (CH), 58.0 (CH), 39.2 (CH₂), 30.2 (CH₂), 25.1 (CH₂), 23.0 (CH₂) ; SM (IS) m/z : 328.5 [M+H]⁺.

### 1-(6-Oxo-1,2,3,4,6,10b-hexahydropyrido[2,1-a]isoindol-10-yl)-3-(pyrimidin-2-yl)thiourée (65).

Le composé 65 est obtenu sous forme de solide beige selon la procédure générale B avec un rendement de 48 % après purification sur gel de silice (CH₂Cl₂ /MeOH : 99 /1). F : 212°C ; IR (ATR-Ge ν cm⁻¹) : 3145 (N-H amine), 1698 (C=O amide), 1580- 1545-1522 (C=C arom), 1406 (C-N), 1198- 1158 (C-C) ; RMN ¹H *(*DMSO-*d6* ; 400 MHz) *δ* 13.15 (s, 1H), 11.30 (s, 1H), 8.74 (d, *J* = 4.9 Hz, 2H), 7.93 (d, *J* = 7.7 Hz, 1H), 7.62 (d, *J* = 7.0 Hz, 1H), 7.54 (t, *J* = 7.7 Hz, 1H), 7.25 (t, *J* = 4.9 Hz, 1H), 4.65 (dd, *J =* 3.5 Hz, *J =* 11.6 Hz, 1H), 4.24 (dd, *J =* 3.7 Hz, *J =* 12.8 Hz, 1H), 3.00 (td, *J* = 3.7 Hz, *J* = 12.8 Hz, 1H), 2.33 (dd, *J* = 3.0 Hz, *J* = 12.6 Hz, 1H), 1.82 (d, *J =* 13.2 Hz, 1H), 1.73 (d, *J =* 13.0 Hz, 1H), 1.58 (q, *J =* 13.0 Hz, 1H), 1.30 - 1.14 (m, 1H), 0.97 (qd, *J =* 3.0 Hz, *J* = 12.9 Hz, 1H) ; RMN ¹³C *(*DMSO-*d6* ; 101 MHz) *δ* 179.1 (Cq), 164.3 (Cq), 158.2 (2 x CH), 157.4 (Cq), 140.6 (Cq), 134.0 (Cq), 133.1 (Cq), 129.2 (CH), 128.5 (CH), 121.4 (CH), 116.1 (CH), 57.6 (CH), 39.0 (CH₂), 30.1 (CH₂), 24.9 (CH₂), 22.9 (CH₂) ; SM (IS) m/z : 340.5 [M+H]⁺, 679.5 [2M+H]⁺.

### 1-(5-Bromopyridin-2-yl)-3-(6-oxo-1,2,3,4,6,10b-hexahydropyrido[2,1-a] isoindol-10-yl)thiourée (66).

Le composé **66** est obtenu sous forme de solide blanc selon la procédure générale B avec un rendement de 75 % après purification sur gel de silice (CH₂Cl₂ /MeOH : 99 /1). F > 260°C ; IR (ATR-Ge ν cm⁻¹) : 3286 (NH amine), 1671 (C=O amide), 1592- 1543-1510-1468 (C=C arom), 1170 (C-N), 662 (C-Br) ; RMN ¹H *(*DMSO-*d6* ; 400 MHz) δ 13.21 (s, 1H), 11.22 (s, 1H), 8.47 (d, *J* = 2.2 Hz, 1H), 8.09 (dd, *J* = 2.4 Hz, *J* = 8.9 Hz, 1H), 7.99 (d, *J* = 7.7 Hz, 1H), 7.60 (d, *J* = 7.3 Hz, 1H), 7.53 (t, *J* = 7.6 Hz, 1H), 7.27 (d, *J* = 8.9 Hz, 1H), 4.66 (dd, *J* = 3.2 Hz, *J* = 11.6 Hz, 1H), 4.24 (dd, *J* = 4.0 Hz, *J* = 12.0 Hz, 1H), 3.02 (td, *J* = 4.0 Hz, *J* = 12.0 Hz, 1H), 2.32 (d, *J =* 9.9 Hz, 1H), 1.82 (d, *J =* 13.2 Hz, 1H), 1.73 (d, *J* = 12.7 Hz, 1H), 1.59 (q, *J =* 13.3 Hz, 1H), 1.30 - 1.17 (m, 1H), 1.03 - 0.90 (m, 1H) ; RMN ¹³C *(*DMSO-*d6* ; 101 MHz*) δ* 178.7 (Cq), 164.3 (Cq), 152.1 (Cq), 146.0 (CH), 141.9 (CH), 140.3 (Cq), 133.9 (Cq), 133.1 (Cq), 128.9 (CH), 128.4 (CH), 120.8 (CH), 114.8 (CH), 112.5 (Cq), 57.5 (CH), 38.9 (CH₂), 30.0 (CH₂), 24.9 (CH₂), 22.9 (CH₂) ; SM (IS) m/z : 418.5 [M+H]⁺.

### 1-(6-Bromopyridin-2-yl)-3-(6-oxo-1,2,3,4,6,10b-hexahydropyrido[2,1-a] isoindol-10-yl)thiourée (67).

Le composé 67 est obtenu sous forme de solide beige selon la procédure générale B avec un rendement de 68 % après purification sur gel de silice (CH₂Cl₂ /MeOH : 99 /1). F : 195°C ; IR (ATR-Ge ν cm⁻¹) : 3225 (N-H amine), 1678 (C=O amide), 1578- 1447- 1433-1409 (C=C arom), 1148- 1127 (C-C), 679 (C-Br) ; RMN ¹H *(*DMSO-*d6* ; 400 MHz) *δ* 12.47 (s, 1H), 11.29 (s, 1H), 7.97 (d, *J =* 7.7 Hz, 1H), 7.80 (t, *J =* 8.0 Hz, 1H), 7.62 (d, *J =* 7.0 Hz, 1H), 7.54 (t, *J =* 7.7 Hz, 1H), 7.39 (d, *J =* 7.6 Hz, 1H), 7.34 (d, *J =* 8.2 Hz, 1H), 4.68 (dd, *J =* 3.6 Hz, *J =* 11.7 Hz, 1H), 4.24 (dd, *J* = 3.7 Hz, *J* = 12.9 Hz, 1H), 3.00 (td, *J* = 3.7 Hz, *J* = 12.9 Hz, 1H), 2.45 (dd, *J* = 3.1 Hz, *J* = 12.8 Hz, 1H), 1.85 (d, *J* = 13.0 Hz, 1H), 1.74 (d, *J* = 12.7 Hz, 1H), 1.70 - 1.55 (m, 1H), 1.32 - 1.15 (m, 1H), 1.02 (qd, *J* = 3.1 Hz, *J* = 12.6 Hz, 1H) ; RMN ¹³C *(*DMSO-*d6* ; 101 MHz*) δ* 178.7 (Cq), 164.2 (Cq), 153.0 (Cq), 142.0 (CH), 140.0 (Cq), 136.5 (Cq), 133.5 (Cq), 133.2 (Cq), 129.5 (CH), 128.4 (CH), 122.0 (CH), 121.0 (CH), 112.0 (CH), 57.4 (CH), 39.0 (CH₂), 30.2 (CH₂), 24.9 (CH₂), 22.9 (CH₂) ; SM (IS) m/z : 418.5 [M+H]⁺.

### 1,3-Bis(6'-oxo-3',4',6',10b'-tétrahydro-1'H-spiro[[1,3]dithiolane-2,2'-pyrido [2,1-a]isoindole]-10'-yl)thiourée (68).

Le composé **68** est obtenu sous forme de solide blanc selon la procédure générale B avec un rendement de 56 % après purification sur gel de silice (CH₂Cl₂ /MeOH : 99 /1). F : 188°C ; IR (ATR-Ge ν cm⁻¹): 3195 (N-H amine), 1687 (C=O amide), 1521- 1487- 1454-1429 (C=C arom), 1204 (C-C), 762 (C-H arom) ; RMN ¹H *(*DMSO*-d6;* 250 MHz) *δ* 9.89 (s, 1H), 9.76 (s, 1H), 7.74 - 7.40 (m, 6H), 4.78 (d, *J* = 11.3 Hz, 2H), 4.31 (d, *J =* 13.3 Hz, 2H), 3.5 - 3.35 (m, 8H), 3.23 - 3.01 (m, 2H), 2.82 - 2.62 (m, 2H), 2.14 (d, *J =* 12.5 Hz, 2H), 1.97 (td, *J =* 4.4 Hz, *J =* 12.6 Hz, 2H), 1.76 - 1.55 (m, 2H) ; RMN ¹³C *(*DMSO-*d6* ; 101 MHz) *δ* 181.1 (Cq), 180.9 (Cq), 164.5 (2 x Cq), 141.4 (Cq), 140.8 (Cq), 134.3 (Cq), 134.8 (Cq), 133.1 (Cq), 131.3 (CH), 130.5 (CH), 129.1 (2 x CH), 121.6 (CH), 121.3 (CH), 65.6 (2 x Cq), 57.4 (CH), 57.2 (CH), 44.86 (CH₂), 44.6 (CH₂), 40.0 (2 x CH₂), 38.5 (2 x CH₂), 38.4 (CH₂), 38.3 (CH₂), 37.9 (CH₂), 37.8 (CH₂) ; SM (IS) m/z : 627.5 [M+H]⁺, 649.0 [M+Na]⁺.

### 1-(6'-Oxo-3',4',6',10b'-tétrahydro-1'H-spiro[[1,3]dithiolane-2,2'-pyrido[2,1-a]isoindole]-10'-yl)-3-(pyrazin-2-yl)thiourée (69).

Le composé **69** est obtenu sous forme de solide jaune selon la procédure générale B avec un rendement de 62 % après purification sur gel de silice (CH₂Cl₂ /MeOH : 99 /1). F : 128°C ; IR (ATR-Ge ν cm⁻¹) : 3203 (N-H amine), 1686 (C=O amide), 1599- 1567- 1514-1484 (C=C arom), 1403 (C-N), 1140 (C-C) ; RMN ¹H (CDCl₃ ; 400 MHz) *δ* 12.94 (s, 1H), 9.79 (s, 1H), 8.57 (d, *J =* 1.1 Hz, 1H), 8.34 (d, *J* = 2.8 Hz, 1H), 8.30 (dd, *J* = 1.4 Hz, *J* = 2.8 Hz , 1H), 8.12 (d, *J* = 7.8 Hz, 1H), 7.82 (d, *J* = 7.4 Hz, 1H), 7.55 (t, *J* = 7.7 Hz, 1H), 4.86 (dd, *J* = 3.4 Hz, *J* = 11.7 Hz, 1H), 4.64 - 4.53 (m, 1H), 3.35 - 3.16 (m, 4H), 3.16 - 3.08 (m, 1H), 2.83 (m, 1H), 2.23 - 2.16 (m, 1H), 2.05 (td, *J* = 5.0 Hz, *J* = 12.8 Hz, 1H), 1.80 (t, *J* = 12.0 Hz, 1H) ; RMN ¹³C *(*CDCl₃; 101 MHz*) δ* 179.6 (Cq), 165.8 (Cq), 149.1 (Cq), 139.2 (CH), 138.7 (Cq), 138.6 (CH), 136.3 (CH), 133.7 (Cq), 133.2 (Cq), 129.4 (CH), 129.3 (CH), 122.7 (CH), 65.6 (Cq), 57.9 (CH), 45.6 (CH₂), 41.3 (CH₂), 39.3 (CH₂), 38.8 (CH₂), 38.5 (CH₂) ; SM (IS) m/z : 430.0 [M+H]⁺

### 1-(6'-Oxo-3',4',6',10b'-tétrahydro-1'H-spiro[[1,3]dithiolane-2,2'-pyrido[2,1-a]isoindole]-10'-yl)-3-(pyridin-2-yl)thiourée (70).

Le composé **70** est obtenu sous forme de solide beige selon la procédure générale B avec un rendement de 45 % après purification sur gel de silice (CH₂Cl₂ /MeOH : 99 /1). F : 142°C ; IR (ATR-Ge ν cm⁻¹) : 3226 (N-H amine), 1677 (C=O amide), 1598- 1530- 1475-1421 (C=C arom), 1144- 1000 (C-C) ; RMN ¹H *(*CDCl₃ ; 400 MHz) *δ* 13.73 (s, 1H), 8.92 (s, 1H), 8.38 (dd, *J =* 1.2, *J* = 5.1vHz, 1H), 8.17 (d, *J* = 7.9 Hz, 1H), 7.81 - 7.71 (m, 2H), 7.54 (t, *J* = 7.8 Hz, 1H), 7.06 (dd, *J* = 5.2 Hz, *J* = 6.7 Hz, 1H), 6.90 (d, *J* = 8.3 Hz, 1H), 4.88 (dd, *J* = 3.5 Hz, *J* = 11.6 Hz, 1H), 4.60 - 4.50 (m, 1H), 3.47 - 3.12 (m, 4H), 3.09 - 3.00 (m, 1H), 2.91 (m, 1H), 2.20 (d, *J* = 13.4 Hz, 1H), 2.09 - 2.01 (m, 1H), 1.79 - 1.73 (m, 1H) ; RMN ¹³C (CDCl₃; 101 MHz) *δ* 179.5 (Cq), 165.9 (Cq), 153.1 (Cq), 146.2 (CH), 139.6 (CH), 138.6 (Cq), 133.7 (Cq), 133.7 (Cq), 129.3 (CH), 129.1 (CH), 122.3 (CH), 118.7 (CH), 112.5 (CH), 65.7 (Cq), 57.8 (CH), 45.7 (CH₂), 41.1 (CH₂), 39.1 (CH₂), 38.9 (CH₂), 38.5 (CH₂) ; SM (IS) m/z : 429.0 [M+H]⁺

### 1-(6'-Oxo-3',4',6',10b'-tétrahydro-1'H-spiro[[1,3]dithiolane-2,2'-pyrido[2,1-a]isoindole]-10'-yl)-3-(1H-pyrazol-3-yl)thiourée (71).

Le composé **71** est obtenu sous forme de solide blanc selon la procédure générale B avec un rendement de 55 % après purification sur gel de silice (CH₂Cl₂ /MeOH : 99 /1). F : 185°C ; IR (ATR-Ge ν cm⁻¹) : 3190 (NH amine), 1669 (C=O amide), 1574, 1525-1485 (C=C arom), 1199 (C-C), 999 (C-N), 755 (C-H arom) ; RMN ¹H *(*DMSO-*d6* ; 400 MHz*) δ* 12.64 (s, 1H), 11.54 (s, 1H), 10.92 (s, 1H), 7.80 (d, *J =* 7.7 Hz, 1H), 7.75 (s, 1H), 7.61 (d, *J =* 7.4 Hz, 1H), 7.53 (t, *J =* 7.6 Hz, 1H), 6.06 (s, 1H), 4.82 (dd, *J* = 3.3 Hz, *J* = 11.5 Hz, 1H), 4.31 (dd, *J* = 3.9 Hz, *J* = 13.6 Hz, 1H), 3.32 - 3.00 (m, 5H), 2.63 (d, *J =* 12.0 Hz, 1H), 2.09 (d, *J =* 13.3 Hz, 1H), 1.97 (td, *J =* 5.0 Hz, *J =* 12.9 Hz, 1H), 1.60 (t, *J* = 12.3 Hz, 1H) ; RMN ¹³C *(*DMSO-*d6* ; 101 MHz) *δ* 177.2 (Cq), 164.5 (Cq), 149.2 (Cq), 139.9 (Cq), 134.2 (Cq), 132.8 (Cq), 130.3 (CH), 129.8 (CH), 128.8 (CH), 121.0 (CH), 94.4 (CH), 65.4 (Cq), 57.1 (CH), 44.4 (CH₂), 39.6 (CH₂), 38.3 (CH₂), 38.0 (CH₂), 37.5 (CH₂) ; SM (IS) m/z : 418.0 [M+H]⁺.

### 1-(3-bromopyridin-2-yl)-3-(6-oxo-1,2,3,4,6,10b-hexahydropyrido[2,1-a] isoindol-10-yl)thiourée (72).

Le composé **72** est obtenu sous forme de solide jaune selon la procédure générale B avec un rendement de 70 % après purification sur gel de silice (CH₂Cl₂ /MeOH : 99 /1). F : 180°C ; IR (ATR-Ge ν cm⁻¹) : 3403 (N-H amine), 1686 (C=O amide), 1566- 1503- 1417 (C=C arom), 1342 (C-N), 1286 (C-C), 757 (C-H arom) ; RMN ¹H *(CDCl₃ ;* 400 MHz) *δ* 13.25 (s, 1H), 8.84 (s, 1H), 8.20 (dd, *J* = 1.5 Hz, 4.9 Hz, 1H), 7.99 (dd, *J =* 1.6 Hz, 7.9 Hz, 1H), 7.83 (dd, *J =* 7.5 Hz, 13.9 Hz, 2H), 7.52 (t, *J* = 7.7 Hz, 1H), 6.99 (dd, *J* = 4.9 Hz, 7.9 Hz, 1H), 4.59 (dd, *J* = 3.7 Hz, 11.8 Hz, 1H), 4.49 (dd, *J =* 4.1 Hz, 13.1 Hz, 1H), 2.98 (td, *J =* 4.1 Hz, 13.1 Hz, 1H), 2.42 (dd, *J* = 3.3 Hz, 12.9 Hz, 1H), 1.93 (d, *J* = 13.5 Hz, 1H), 1.80 (d, *J* = 13.2 Hz, 1H), 1.68 - 1.56 (m, 1H), 1.48 - 1.31 (m, 1H), 1.14 (qd, *J* = 3.3 Hz, 12.9 Hz, 1H) ; RMN ¹³C *(CDCl₃*; 101 MHz) *δ* 179.4 (Cq), 165.6 (Cq), 149.7 (Cq), 144.6 (CH), 142.5 (CH), 140.8 (Cq), 134.1 (Cq), 133.4 (Cq), 129.3 (CH), 129.0 (CH), 122.7 (CH), 119.5 (CH), 107.0 (Cq), 58.7 (CH), 39.8 (CH₂), 30.5 (CH₂), 25.3 (CH₂), 23.8 (CH₂) ; SM (IS) m/z : 418.5 [M+H]⁺

### 1-(5-méthylpyridin-2-yl)-3-(6-oxo-1 ,2,3,4,6,10b-hexahydropyrido[2,1-a] isoindol-10-yl)thiourée (73).

Le composé 73 est obtenu selon la procédure B à partir de l'isothiocyanate **11** sous forme de solide blanc avec 60 % de rendement après chromatographie sous gel de silice flash (CH₂Cl₂ / MeOH : 99 /1). F : 232°C ; IR (ATR-Ge ν cm⁻¹) : 3297 (N-H amine), 1673 (C=O amide), 1540-1506-1488 (C=C arom), 1170 (C-N) ; RMN ¹H *(*DMSO-*d6* ; 400 MHz) δ 13.74 (s, 1H), 11.02 (s, 1H), 8.16 (s, 1H), 8.10 (d, *J* = 7.6 Hz, 1H), 7.71 (dd, *J =* 2.1 Hz, 8.5 Hz, 1H), 7.58 (d, *J =* 6.9 Hz, 1H), 7.52 (t, *J =* 7.6 Hz, 1H), 7.20 (d, *J* = 8.5 Hz, 1H), 4.65 (dd, *J* = 3.5 Hz, 11.6 Hz, 1H), 4.24 (dd, *J* = 3.5 Hz, 12.7 Hz, 1H), 3.02 (td, *J =* 3.5 Hz, 12.8 Hz, 1H), 2.44 - 2.32 (m, 1H), 2.27 (s, 3H), 1.83 (d, *J =* 12.9 Hz, 1H), 1.73 (d, *J =* 12.7 Hz, 1H), 1.67 - 1.49 (m, 1H), 1.31 - 1.14 (m, 1H), 1.01 - 0.96 (m, 1H) ; RMN ¹³C *(*DMSO-*d6* ; 101 MHz) δ 178.6 (Cq), 164.3 (Cq), 151.4 (Cq), 144.6 (CH), 140.3 (CH), 139.9 (Cq), 134.1 (Cq), 133.0 (Cq), 128.5 (CH), 128.4 (CH), 127.6 (Cq), 120.5 (CH), 112.5 (CH), 57.6 (CH), 38.9 (CH₂), 30.0 (CH₂), 24.9 (CH₂), 22.9 (CH₂), 17.2 (CH₃). SM (IS) m/z : 353.0 [M+H]⁺

### 1-(3-Bromopyridin-2-yl)-3-(6'-oxo-3',4',6',10b'-tétrahydro-1'H-spiro[[1,3] dithiolane-2,2'-pyrido [2,1-a]isoindole]-10'-yl)thiourée (74).

Le composé **74** a été obtenu selon la procédure **B** avec un rendement de 70%, sous la forme d'un solide blanc. p.f. 215°C. IR (ATR-Ge ν cm⁻¹) : 3380 (N-H amine), 1686 (C=O amide), 1554- 1503 (C=C arom), 1336 (C-N), 1287 (C-C), 754 (C-H arom). ¹H RMN (CDCl₃ , 400 MHz) : 13.4 (s, 1H), 8.83 (s, 1 H), 8.35 (dd, *J =* 2.5 Hz, *J* = 7.5 Hz, 1H), 8.16 (d, *J* = 7.5 Hz, 1H), 7.98 (dd, *J* = 2.5 Hz, *J* = 7.5 Hz, 1H), 7.77 (d, *J* = 7.5 Hz, 1H), 7.53 (t, *J* = 7.5 Hz, 1H), 6.97 (dd, *J* = 5.0 Hz, *J* = 7.5 Hz, 1H), 4.85 (dd, *J =* 5.0 Hz, *J =* 12.5 Hz, 1H), 4.53 (dd, *J =* 2.5 Hz, *J =* 12.5 Hz, 1H), 3.05 - 3.34 (m, 5H), 2.86 (d, *J* = 12.5 Hz, 1H), 2.20 (d, *J* = 12.5 Hz, 1H), 2.01 (td, *J* = 5.0 Hz, *J =* 12.5 Hz, 1H), 1.77 (t, *J =* 12.5 Hz, 1H). ¹³C RMN (CDCl₃ , 100 MHz) : 178.9 (Cq), 165.5 (Cq), 149.6 (Cq), 144.8 (CH), 142.4 (CH), 138.2 (Cq), 133.4 (Cq), 133.3 (Cq), 128.9 (CH), 128.8 (CH), 122.2 (CH), 119.1 (CH), 106.8 (Cq), 65.5 (Cq), 57.6 (CH), 45.4 (CH₂), 41.0 (CH₂), 39.0 (CH₂), 38.6 (CH₂), 38.2 (CH₂). HRMS : calc. pour C₂₀ H₂₀ N₄ O S₃ Br : 506,9983 ; trouvé 506,9998.

### 2.3. Procédure générale C

### Synthèse des dérivés des urées (75) et (76) à partir des thiourées (72) ou (73).

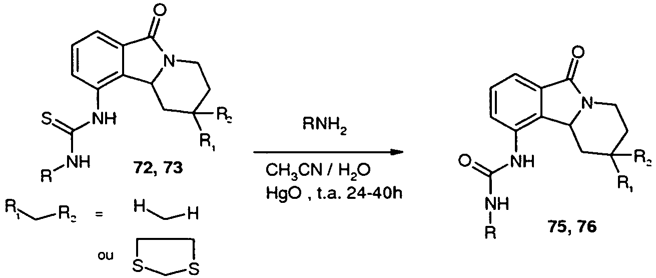

A une solution de thiourée (0.3 mmol) dans un mélange eau / acétonitrile 1/1 (20 mL), 390 mg d'oxide de mercure (II) (1.8 mmol, 6.0 éq.) sont ajoutés. Le mélange réactionnel est agité à température ambiante dans l'obscurité pendant 24 à 40h puis filtré sur célite. Le filtrat est évaporé sous pression réduite et purifié par chromatographie sur gel de silice. Pour donner les urées attendues.

### 1-(3-Bromopyridin-2-yl)-3-(6-oxo-1,2,3,4,6,10b-hexahydropyrido [2,1-a]isoindol-10-yl)urée (75).

Le composé **75** est obtenu sous forme de solide jaune pâle selon la procédure générale C à partir de **72** avec un rendement de 62% après purification sur gel de silice (CH₂Cl₂ / MeOH : 99 /1). F : 220°C ; IR (ATR-Ge ν cm⁻¹) : 1678 (C=O amide), 1567 (N-H amide), 1478-1432-1391 (C=C arom), 1283 (C-N), 1203- 1025 (C-C), 739 (C-Br) ; RMN ¹H (CDCl₃ ; 400 MHz) δ 11.60 (s, 1H), 8.15 - 8.05 (m, 2H), 7.87 (dd, *J* = 1.5 Hz, *J* = 7.9 Hz, 1H), 7.60 - 7.49 (m, 2H), 7.39 (t, *J* = 7.8 Hz, 1H), 6.87 (dd, *J* = 4.9 Hz, *J* = 7.9 Hz, 1H), 4.46 (dd, *J* = 4.9 Hz, *J* = 13.3 Hz, 1H), 4.39 (dd, *J* = 3.5 Hz, *J* = 11.7 Hz, 1H), 2.94 (td, *J* = 3.5 Hz, *J* = 13.0 Hz, 1H), 2.64 (dd, *J* = 3.1 Hz, *J* = 13.0 Hz, 1H), 1.96 (d, *J =* 13.3 Hz, 1H), 1.76 (d, *J* = 13.2 Hz, 1H), 1.69 - 1.55 (m, 1H), 1.43 - 1.29 (m, 1H), 1.02 (qd, *J* = 3.1 Hz, *J* = 13.0 Hz, 1H) ; RMN ¹³C *(*CDCl₃ ; 101 MHz) δ 166.0 (Cq), 151.5 (Cq), 149.6 (Cq), 144.5 (CH), 142.3 (CH), 135.9 (Cq), 133.6 (Cq), 132.9 (Cq), 129.3 (CH), 124.2 (CH), 119.7 (CH), 118.7 (CH), 107.4 (Cq), 58.5 (CH), 39.9 (CH₂), 30.4 (CH₂), 25.4 (CH₂), 24.1 (CH₂) ; SM (IS) m/z : 402.5 [M+H]⁺.

### 1-(5-méthylpyridin-2-yl)-3-(6-oxo-1,2,3,4,6,10b-hexahydropyrido [2,1-a]isoindol-10-yl)urée (76).

Le composé 76 est obtenu selon la procédure C à partir du dérivé 73 sous forme de solide blanc après chromatographie sous gel de silice flash (CH₂Cl₂ /MeOH : 99 /1). IR (ATR-Ge ν cm⁻¹) : RMN ¹H *(CDCl₃;* 400 MHz) δ 11.86 (s, 1H), 8.22 (d, *J* = 8.1 Hz, 1H), 8.03 (s, 1H), 7.63 (d, *J* = 7.4 Hz, 1H), 7.60 - 7.39 (m, 3H), 6.73 (s, 1H), 4.55 (dd, *J =* 4.6 Hz, 13.2 Hz, 1H), 4.49 (dd, *J =* 3.4 Hz, 12.3 Hz, 1H), 3.03 (td, *J =* 3.5 Hz, 12.3 Hz, 1H), 2.81 (d, *J =* 10.0 Hz, 1H), 2.33 (s, 3H), 2.05 (d, *J* = 13.8 Hz, 1H), 1.84 (d, *J =* 14.6 Hz, 1H), 1.78 - 1.68 (m, 1H), 1.50 - 1.41 (m, 1H), 1.16 - 1.02 (m, 1H) ; SM (IS) m/z : 337.5 [M+H]⁺, 673.5 [2M+H]⁺

### 2.4. Procédure générale D

### Synthèse des urées (77) à (96) à partir des amines (5), (7) ou (10).

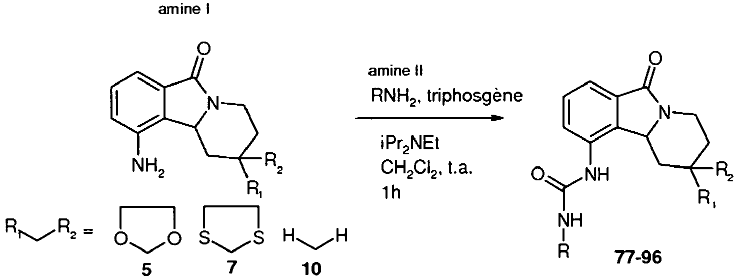

A une solution de triphosgène (29 mg, 0.36 éq.) dans du dichlorométhane (2 mL) est ajouté doucement pendant une heure à l'aide d'une pompe à seringue une solution de l'amine **I** (0.27 mmol) et 84 µl d'*i*-Pr₂NEt (0.59 mmol ; 2.2 éq.) dans du dichlorométhane (3 mL). Le mélange réactionnel est agité 10 min, puis une solution de l'amine **II** (0.32 mmol, 1.2 éq.) et de l'*i*-Pr₂NEt (84 µl, 0.59 mmol ; 2.2 éq.) dans du dichlorométhane (3 mL) est ajoutée. Le mélange réactionnel est agité pendant 1 h puis du dichlorométhane (4 mL) et une solution aqueuse de NaHCO₃ saturée (5 mL) sont additionnés. Après décantation, les phases organiques sont lavées avec une solution aqueuse de NaCl saturée (5 mL). La phase organique est séchée sur MgSO₄ filtrées puis évaporée sous pression réduite pour donner l'urée attendue qui sera purifiée par chromatographie sur gel de silice flash.

### 1-(6'-Oxo-3',4',6',10b'-tétrahydro-1'H-spiro[[1,3]dithiolane-2,2'-pyrido[2,1-a]isoindole]-10'-yl)-3-(1H-pyrazol-3-yl)urée (77).

Le composé **77** est obtenu sous forme de solide beige pâle à partir de l'amine **7** selon la procédure générale D après chromatographie sur gel de silice (dichlorométhane / méthanol 98 /2). F : 184°C ; IR (ATR-Ge ν cm⁻¹) : 1731-1691 (C=O amide), 1620- 1606 (N-H amide), 1575- 1528-1488 (C=C arom), 1386- 1242 (C-N), 1042 (C-C) ; RMN ¹H *(*DMSO-*d6* ; 400 MHz*) δ* 9.88 (s, 1H), 8.05 (d, *J =* 2.8 Hz, 1H), 7.66 (dd, *J* = 1.0 Hz, 7.6 Hz, 1H), 7.59 - 7.48 (m, 2H), 5.90 (d, *J* = 2.8 Hz, 1H), 5.40 (s, 2H), 4.89 (dd, *J* = 3.3 Hz, *J* = 11.5 Hz, 1H), 4.30 (dd, *J* = 3.5 Hz, *J* = 13.7 Hz, 1H), 3.46 - 3.22 (m, 4H), 3.12 (td, *J* = 3.5 Hz, *J* = 13.2 Hz, 1H), 2.59 (d, *J* = 11.9 Hz, 1H), 2.11 (d, *J* = 13.4 Hz, 1H), 1.96 (td, *J* = 5.1 Hz, *J* = 12.9 Hz, 1H), 1.55 (t, 12.0 Hz, 1H) ; RMN ¹³C *(*DMSO-*d6* ; 101 MHz) δ 164.4 (Cq), 158.0 (Cq), 147.7 (Cq), 138.1 (Cq), 132.8 (Cq), 132.3 (Cq), 129.9 (CH), 128.8 (CH), 127.5 (CH), 120.0 (CH), 99.6 (CH), 65.5 (Cq), 57.2 (CH), 43.9 (CH₂), 39.7 (CH₂), 38.2 (CH₂), 38.0 (CH₂), 37.6 (CH₂) ; SM (IS) m/z : 402.0 [M+H]⁺.

### 3-Amino-N-(6-oxo-1,2,3,4,6,10b-hexahydropyrido[2,1-a]isoindol-10-yl)-1H-pyrazole-1-carboxamide (78).

Le composé **78** est obtenu avec un rendement de 63% sous forme de solide beige pâle à partir de l'amine **10** selon la procédure générale D après chromatographie sur gel de silice (dichlorométhane / méthanol 98 /2). F : 166°C ; IR (ATR-Ge ν cm⁻¹) : 3320 (NH amine), 1685 (C=O amide), 1572 (N-H amide), 1520-1482-1430 (C=C arom), 1384 (C-N), 1286-1240-1046 (C-C), 758 (C-H arom) ; RMN ¹H (CDCl₃ ; 400 MHz) *δ* 8.77 (s, 1H), 8.05 (d, *J* = 2.8 Hz, 1H), 7.95 (d, *J* = 8.0 Hz, 1H), 7.69 (d, *J* = 7.5 Hz, 1H), 7.48 (t, *J* = 7.8 Hz, 1H), 5.91 (d, *J* = 2.9 Hz, 1H), 4.61 - 4.42 (m, 2H), 4.12 (s, 2H), 2.99 (td, *J* = 3.4 Hz, *J* = 13.0 Hz, 1H), 2.49 (dd, *J* = 3.3 Hz, *J =* 12.9 Hz, 1H), 1.99 (d, *J* = 13.6 Hz, 1H), 1.82 (d, *J* = 13.2 Hz, 1H), 1.70 (qt, *J* = 3.3 Hz, *J* = 13.3 Hz, 1H), 1.49 - 1.32 (m, 1H), 1.12 (qd, *J* = 3.3 Hz, *J* = 12.9 Hz, 1H) ; RMN ¹³C (CDCl₃ ; 101 MHz) *δ* 165.7 (Cq), 156.7 (Cq), 147.2 (Cq), 136.5 (Cq), 134.0 (Cq), 131.80 (Cq), 130.4 (CH), 129.4 (CH), 124.3 (CH), 120.5 (CH), 99.7 (CH), 58.2 (CH), 39.9 (CH₂), 30.4 (CH₂), 25.3 (CH₂), 23.7 (CH₂) ; SM (IS) m/z : 312.5 [M+H]⁺, 623.5 [2M+H]⁺.

### N,N-Diisopropyl-N'-(6-oxo-1,2,3,4,6,10b-hexahydropyrido[2,1-a]iso-indol-10-yl)urée (79).

Le composé **79** est obtenu avec un rendement de 70% sous forme de solide blanc à partir de l'amine 10 selon la procédure générale D après chromatographie sur gel de silice (CH₂Cl₂ / MeOH : 9 / 1 + 1% Et₃N). F : 156°C ; IR (NaCl ν cm⁻¹) : 3425 (N-H amide), 1673 (C=O amide), 1518 (N-H amide), 1435 (C=C arom), 1289-1248 (C-C) ; RMN ¹H (CDCl₃ ; 400 MHz) *δ* 7.60 (dd, *J* = 6.0 Hz, *J* = 2.4 Hz, 1H), 7.40-7.32 (m, 2H), 6.30 (sb, 1H), 4.55 (dd, *J =* 11.7 Hz, *J =* 3.6 Hz, 1H), 4.46 (dd, *J* = 13.3 Hz, *J =* 4.9 Hz, 1H), 3.96 (quint, *J =* 6.8 Hz, 1H), 2.97 (td, *J =* 12.9 Hz, *J =* 3.5 Hz, 1H), 2.30-2.24 (m, 1H), 1.97-1.92 (m, 1H), 1.82-1.75 (m, 1H), 1.61 (qt, *J =* 13.1 Hz, *J =* 3.2 Hz, 1H), 1.35 (m, 12H), 1.41-1.32 (m, 1H), 1.00 (dd, *J* = 11.8 Hz, *J* = 3.5 Hz, 1H) ; RMN ¹³C (CDCl₃; 101 MHz) *δ* 166.0 (Cq), 153.9 (Cq), 137.7 (Cq), 134.2 (2 x Cq), 128.7 (CH), 125.3 (CH), 119.6 (CH), 59.2 (CH), 46.2 (CH), 39.7 (CH₂), 29.9 (CH₂), 25.4 (CH₂), 23.7 (CH₂), 21.8 (2 x CH₃), 21.3 (2 x CH₃). SM (IS) m/z : 393.5 [M+H]⁺,

### N-[2-(Diméthylamino)éthyl]-N'-(6'-oxo-3',4,4',5,6',10b'-hexahydro-1'H-spiro [1,3-dithiolane-2,2'-pyrido[2,1-a]isoindol]-10'-yl)urée (80).

Le composé **80** est obtenu avec un rendement de 50% sous forme de solide blanc à partir de l'amine **7** selon la procédure générale D après chromatographie sur gel de silice (CH₂Cl₂ / MeOH : 9 / 1 + 1% Et₃N). F : 194°C ; IR (NaCl ν cm⁻¹) : 3384 (N-H amide), 1654 (C=O amide), 1486-1426 (C=C arom), 1291 (C-C) ; RMN ¹H (CDCl₃ ; 400 MHz) *δ* 8.20-7.80 (sb, 1H, ), 7.61 (d, *J =* 7.5 Hz, 1H), 7.50 (d, *J =* 6.6 Hz, 1H), 7.38 (t, *J* = 7.7 Hz, 1H), 5.89 (sb, 1H,), 4.74 (dd, *J* = 11.5 Hz, *J* = 3.2 = Hz, 1H), 4.47 (dd, *J =* 13.7 Hz, *J =* 5.0 Hz, 1H), 3.43-3.32 (m, 6H), 3.20 (td, *J =* 13.2 Hz, *J* = 3.2 Hz, 1H), 2.82 (d, *J* = 11.6 Hz, 2H), 2.55-2.46 (m, 2H), 2.26 (s, 6H), 2.15 (d, *J* = 12.0 Hz, 1H), 1.97 (td, *J* = 12.9 Hz, *J* = 5.0 Hz, 1H), 1.59 (dd, *J* = 12.9 Hz, *J =* 11.9 Hz, 1H) ; RMN ¹³C (CDCl₃; 101 MHz) *δ* 166.1 (Cq), 156.2 (Cq), 136.4 (Cq), 134.1 (Cq), 133.5 (Cq), 129.4 (CH), 126.0 (CH), 119.7 (CH), 65.7 (Cq), 59.4 (CH₂), 58.2 (CH), 45.3 (2 x CH₃), 45.0 (CH₂), 41.4 (CH₂), 39.3 (CH₂), 38.7 (2 x CH₂), 38.4 (CH₂) ; SM (m/z) (IS) : 407.0 [M+H]⁺.

### N-Cyclohexyl-N'-(6'-oxo-3',4,4',5,6',10b'-hexahydro-1'H-spiro[1,3-dithiolane-2,2'-pyrido[2,1-a]isoindol]-10'-yl)urée (81).

Le composé **81** est obtenu avec un rendement de 64% sous forme de solide blanc à partir de l'amine 7 selon la procédure générale D après chromatographie sur gel de silice (CH₂Cl₂ / MeOH 98 / 2 + 1% Et₃N). F : 172°C ; IR (NaCl ν cm⁻¹) : 3349 (N-H amide), 1652 (C=O amide), 1559 (C=C arom), 1510 (N-H amide), 1486-1452-1425 (C=C arom), 1287-1254 (C-C) ; RMN ¹H (CDCl₃ ; 400 MHz) *δ* 7.78 (sb, 1H), 7.44 (d, *J =* 7.4 Hz, 1H), 7.39 (d, *J =* 7.4 Hz, 1H), 7.28 (t, *J =* 7.7 Hz, 1H), 5.80 (d, *J* = 7.5 Hz, 1H), 4.70 (dd, *J* = 11.5 Hz, *J* = 3.2 Hz, 1H), 4.39 (dd, *J* = 13.5 Hz, *J* = 3.9 Hz, 1H), 3.62-3.61 (m, 1H), 3.29-3.14 (m, 5H), 2.83 (d, *J =* 11.9 Hz, 1H), 2.11-2.09 (m, 2H), 2.00 (d, *J =* 9.5 Hz, 1H), 1.95-1.89 (m, 2H), 1.67 (sb, 2H), 1.59-1.56 (m, 1H), 1.45 (t, *J* = 12.3 Hz, 1H), 1.34-1.26 (m, 2H), 1.10-1.08 (m, 2H) ; RMN ¹³C (CDCl₃ ; 101 MHz) *δ* 166.3 (Cq), 155.2 Cq), 136.5 (Cq), 134.2 (Cq), 133.3 (Cq), 129.2 (CH), 126.0 (CH), 119.1 (CH), 65.5 (Cq), 58.4 (CH), 49.0 (CH), 44.9 (CH₂), 41.3 (CH₂), 39.3 (CH₂), 38.9 (CH₂), 38.4 (CH₂), 33.9 (CH₂), 33.6 (CH₂), 25.7 (CH₂), 25.0 (CH₂), 25.0 (CH₂) ; SM (IS) m/z : 418.5 [M+H]⁺

### N-(6'-Oxo-3',4,4',5,6',10b'-hexahydro-1'H-spiro[1,3-dithiolane-2,2'-pyrido [2,1-a]isoindol]-10'-yl)-N'-pipéridin-1-yl-urée (82).

Le composé **82** est obtenu avec un rendement de 56% sous forme de solide blanc à partir de l'amine **7** selon la procédure générale D après chromatographie sur gel de silice (CH₂Cl₂ / MeOH 97 / 3 + 1% Et₃N). F : 158°C ; IR (NaCl ν cm⁻¹) : 3444 (N-H amide), 1688 (C=O amide), 1521 (N-H amide), 1434 (C=C arom), 1289-1242 (C-C) ; RMN ¹H (CDCl₃ ; 400 MHz) *δ* 8.32 (sb, 1H), 8.23 (dd, *J* = 8.0 Hz, *J* = 0.9 Hz, 1H), 7.55 (dd, *J* = 7.5 Hz, *J* = 1.0 Hz, 1H), 7.44 (t, *J* = 7.8 Hz, 1H), 6.03 (sb, 1H), 4.59 (dd, *J* = 11.7 Hz, *J* = 3.3 Hz, 1H), 4.53 (ddd, *J* = 8.9 Hz, *J* = 4.9 Hz, *J* = 1.7 Hz, 1H), 3.46-3.25 (m, 5H), 3.25-3.17 (m, 2H), 2.84 (ddd, *J =* 12.9 Hz, *J =* 3.3 Hz, *J =* 2.0 Hz, 1H), 2.45 (sb, 1H), 2.23-2.16 (m, 1H), 2.04 (sb, 1H), 2.00 (td, *J =* 13.4 Hz, *J* = 5.0 Hz, 1H), 1.85-1.68 (m, 6H), 1.26-1.19 (m, 1H) ; RMN ¹³C (CDCl₃; 101 MHz) δ 166.1 (Cq), 154.8 (Cq), 133.7 (Cq), 132.9 (Cq), 132.7 (Cq), 129.7 (CH), 122.8 (CH), 118.6 (CH), 65.6 (Cq), 57.9 (CH₂), 57.4 (CH), 45.6 (CH₂), 42.1 (CH₂), 39.8 (CH₂), 38.3 (CH₂), 38.3 (CH₂), 26.0 (CH₂), 25.9 (CH₂), 23.2 (CH₂) ; SM (IS) m/z : 419.5 [M+H]⁺.

### 4-Méthyl-N-(6'-oxo-3',4',6',10b'-tétrahydro-1'H-spiro[1,4-dithiane-2,2'-pyrido [2,1-a]isoindol]-10'-yl)pipérazine-1-carboxamide (83).

Le composé **83** est obtenu avec un rendement de 66% sous forme de solide blanc à partir de l'amine **7** selon la procédure générale D après chromatographie sur gel de silice (CH₂Cl₂ / MeOH 97 / 3 + 1% Et₃N). F : 168°C ; IR (NaCl ν cm⁻¹) : 3422 (N-H amide), 1643 (C=O amide), 1520 (C=C arom), 1429 (C=C arom), 1292 (C-C) ; RMN ¹H (CDCl₃ ; 250 MHz) *δ* 7.55 (dd, *J* = 7.1 Hz, *J* = 1.2 = Hz, 1H), 7.34 (t, *J* = 7.8 Hz, 1H), 7.27 (dd, *J =* 7.8 Hz, *J =* 1.2 Hz, 1H), 7.04 (sb, 1H), 4.75 (dd, *J =* 11.6 Hz, *J* = 3.2 Hz, 1H), 4.42 (dd, *J =* 13.8 Hz, *J =* 3.5 Hz, 1H), 3.72-3.44 (m, 5H), 3.41-3.34 (m, 4H), 3.21 (td, *J =* 13.2 Hz, *J =* 3.2 Hz, 1H), 2.68-2.61 (m, 1H), 2.50-2.42 (m, 1H), 2.33 (s, 1H), 2.18-2.07 (m, 2H), 1.94 (td, *J =* 12.9 Hz, *J =* 5.0 Hz, 1H), 1.56 (t, *J =* 12.2 Hz, 1H); RMN ¹³C (CDCl₃; 101 MHz) *δ* 166.1 (Cq), 154.7 Cq), 137.6 (Cq), 134.2 (Cq), 133.6 (Cq), 129.2 (CH), 126.3 (CH), 120.2 (CH), 65.7 (Cq), 58.6 (CH), 55.1 (2 x CH₂), 46.4 (CH₃), 45.1 (CH₂), 44.5 (2 x CH₂), 41.6 (CH₂), 39.5 (CH₂), 38.6 (2 x CH₂ SM (IS) m/z : 419.5 [M+H]⁺, 451.5 [M+K]⁺.

### N-1-Adamantyl-N'-(6'-oxo-3',4',5,6,6',10b'-hexahydro-1'H-spiro[1,4-dithiane-2,2'-pyrido[2,1-a]isoindol]-10'-yl)urée (84).

Le composé **84** est obtenu avec un rendement de 81% sous forme de solide blanc à partir de l'amine **7** selon la procédure générale D après chromatographie sur gel de silice (CH₂Cl₂ / MeOH : 96 / 4). F : 132°C ; IR (ATR-Ge ν cm⁻¹) : 1655 (C=O amide), 1545 (NH amide), 1219 (C-C) ; RMN ¹H (CDCl₃ ; 400 MHz) *δ* 7.63 (d, *J* = 7.2 Hz, 1H), 7.46-7.40 (m, 2H), 6.32 (sb, 1H), 4.79 (dd, *J* = 11.6 Hz, *J* = 3.4 Hz, 1H), 4.63 (sb, 1H), 4.50 (dd, *J* = 11.7 Hz, *J* = 4.8 Hz, 1H), 3.38-3.35 (m, 4H), 3.25 (td, *J* = 13.3 Hz, *J =* 3.2 Hz, 1H), 2.83-2.81 (m, 1H), 2.19 (d, J = 12.7 Hz, 1H), 2.10 (m, 3H), 2.05-1.97 (m, 8H), 1.69 (s, 6H) ; RMN ¹³C (CDCl₃ ; 101 MHz) *δ* 166.4 Cq), 154.2 Cq), 136.4 (Cq), 134.3 (Cq), 133.4 (Cq), 129.4 (CH), 125.9 (CH), 119.2 (CH), 65.5 (Cq), 58.4 (CH), 51.4 Cq), 45.0 (CH₂), 42.4 (3 x CH₂), 41.4 (CH₂), 39.4 (SCH₂), 39.0 (CH₂), 38.5 (CH₂), 36.5 (3 x CH₂), 29.7 (3 x CH) ; SM (IS) m/z : 470.5 [M+H]⁺,492.5 [M+Na]⁺.

### N-(6-Oxo-1,2,3,4,6,10b-hexahydropyrido[2,1-a]isoindol-10-yl)-N'-(piperidin-1-yl)urée (85).

Le composé **85** est obtenu avec un rendement de 60% sous forme de solide blanc à partir de l'amine **10** selon la procédure générale D après chromatographie sur gel de silice (CH₂Cl₂ / MeOH : 96 / 4). F : 166°C ; IR (ATR-Ge ν cm⁻¹) : 3306 (NH), 1692-1627 (C=O amide), 1557 (NH amide), 1441 (C-C) ; RMN ¹H (CDCl₃ ; 400 MHz) *δ* 8.03 (sb, 1H), 7.95 (d, *J* = 7.3 Hz, 1H), 7.35 (t, *J* = 7.6 Hz, 1H), 7.27 (d, *J* = 7.1 Hz, 1H), 6.55 (d, *J* = 7.6 Hz, 1H), 4.46 (dd, *J* = 11.3 Hz, *J* = 2.9 Hz, 1H), 4.24 (dd, *J* = 13.0 Hz, *J =* 4.2 Hz, 1H), 3.32 (s, 1H), 2.99 (td, *J* = 12.7 Hz, *J =* 3.0 Hz, 1H), 1.91-1.80 (m, 3H), 1.74 (d, *J =* 12.6 Hz, 1H), 1.68-1.53 (m, 4H), 1.35-1.14 (m, 6H), 0.78 (q, *J* = 12.2 Hz, 1H) ; RMN ¹³C (CDCl₃; 101 MHz) *δ* 164.7 (Cq), 154.1 (Cq), 135.2 (Cq), 134.3 (Cq), 132.7 (Cq), 128.5 (CH), 122.0 (CH), 116.2 (CH), 39.0 (CH₂), 57.5 (CH), 47.8 (CH₂), 33.0 (CH₂), 32.8 (CH₂), 29.8 (CH₂), 25.2 (CH₂), 25.0 (CH₂), 24.2 (CH₂), 23.0 (CH₂) ; SM (IS) m/z : 328.5 [M+H]⁺.

### N-(6'-Oxo-3',4',5,6,6',10b'-hexahydro-1'H-spiro[1,4-dithiane-2,2'-pyrido[2,1-a] isoindol]-10'-yl)-N'-phénylurée (86).

Le composé **86** est obtenu avec un rendement de 76% sous forme de solide blanc à partir de l'amine **7** selon la procédure générale D après chromatographie sur gel de silice (CH₂Cl₂ / MeOH 97 / 3 + 1% Et₃N). F : 161 °C ; IR (ATR-Ge ν cm⁻¹) : 1659-1594 (C=O amide), 1541 (NH amide), 1488-1447 (C=C arom), 1296 (C-C) ; RMN ¹H *(*CDCl₃ ; 400 MHz*) δ* 8.44 (sb, 1H), 8.35 (sb, 1H), 7.54 (d, *J* = 7.8 Hz, 1H), 7.45 (d, *J =* 7.4 Hz, 1H), 7.33 (d, *J =* 7.8 Hz, 2H), 7.27 *(t, J =* 7.6 Hz, 1H), 7.19 (t, *J =* 7.8 Hz, 2H), 6.98 (t, *J* = 7.3 Hz, 1H), 4.64 (dd, *J* = 11.4 Hz, *J* = 3.1 Hz, 1H), 4.38 (dd, *J =* 13.6 Hz, *J =* 3.6 Hz, 1H), 3.21-3.03 (m, 5H), 2.79 (d, *J =* 12.0 Hz, 1H), 2.09 (d, *J* = 13.0 Hz, 1H), 1.92 (td, *J* = 13.1 Hz, *J* = 5.0 Hz, 1H), 1.44 (d, *J* = 12.4 Hz, 1H) RMN ¹³C *(*CDCl₃; 101 MHz) *δ* 166.2 (Cq), 153.7 Cq), 138.6 (Cq), 136.4 (Cq), 133.5 (Cq), 133.1 (Cq), 129.4 (CH), 129.1 (2 x CH), 126.2 (CH), 123.5 (CH), 120.0 (2 x CH), 119.4 (CH), 65.4 (Cq), 58.2 (CH), 44.9 (CH₂), 41.0 (CH₂), 39.2 (CH₂), 39.0 (CH₂), 38.4 (CH₂). SM (IS) m/z : 412.5 [M+H]⁺, 410.5 [M-H]⁺.*

### N-(6-Oxo-1,2,3,4,6,10b-hexahydropyrido[2,1-a]isoindol-10-yl)-N'-phénylurée (87).

Le composé **87** est obtenu avec un rendement de 22% sous forme de solide blanc à partir de l'amine **10** selon la procédure générale D après chromatographie sur gel de silice (CH₂Cl₂ / MeOH : 97 / 3 + 1% Et₃N). F : 172°C ; IR (ATR-Ge ν cm⁻¹) : 1650 (CO amide), 1290 (C-C) ; RMN ¹H (CDCl₃ ; 400 MHz) *δ* 8.41 (sb, 1H), 8.25 (sb, 1H), 7.62 (d, *J =* 7.4 Hz, 1H), 7.50 (d, *J =* 7.5 Hz, 1H), 7.34-7.26 (m, 2H), 7.26-7.18 (m, 2H), 6.99 (t, *J =* 7.3 Hz, 1H), 4.50-4.37 (m, 2H), 2.89 (td, *J =* 12.5 Hz, *J =* 2.9 Hz, 1H), 2.56 (d, *J=* 10.2 Hz, 1H), 1.80-1.72 (m, 2H), 1.30-1.23 (m, 1H), 1.46 (q, *J =* 12.9 Hz, 1H), 0.92 (qd, *J =* 12.0 Hz, *J* = 3.0 Hz, 1H) ; RMN ¹³C *(*CDCl₃; 101 MHz) *δ* 166.6 (Cq), 153.6 Cq), 138.7 (Cq), 137.5 (Cq), 133.9 (Cq), 133.1 (Cq), 129.2 (3 x CH), 125.8 (CH), 123.4 (CH), 119.7 (CH), 119.2 (2 x CH), 54 9 (CH), 40.1 (CH₂), 30.1 (CH₂), 25.5 (CH₂), 23.5 (CH₂) ; SM (IS) m/z : 322.5 [M+H]⁺.

### N-Benzyl-N'-(6'-oxo-3',4,4',5,6',10b'-hexahydro-1'H-spiro[1,3-dithiolane-2,2'-pyrido[2,1-a]isoindol]-10'-yl)urée (88).

Le composé **88** est obtenu avec un rendement de 69% sous forme de solide blanc à partir de l'amine **7** selon la procédure générale D après chromatographie sur gel de silice (CH₂Cl₂ / MeOH 97 / 3). F : 165°C ; IR (ATR-Ge ν cm⁻¹) : 3327 (NH amide), 1663 (C=O amide), 1541 (NH amide), 1500-1455-1427 (C=C arom), 1231 (C-C) ; RMN ¹H (CDCl₃ ; 400 MHz) *δ* 7.81 (s, 1H), 7.54 (d, *J* = 7.9 Hz , 1H), 7.32 (d, *J* = 7.4 Hz, 1H), 7.28-7.22 (m, 6H), 6.25 (sb, 1H), 4.60 (dd, *J* = 14.9 Hz, *J* = 3.4 Hz, 1H), 4.42-4.33 (m, 2H), 4.30 (dd, *J* = 13.6 Hz, *J* =3.9 Hz, 1H), 3.26-3.03 (m, 4H), 2.77 (d, *J* = 12.0 Hz, 1H), 2.04(d,*J* = 13.1 Hz, 1H), 1.86 (td, *J* = 13.0 Hz, *J* = 5.0 Hz, 1H), 1.45 (t, *J* = 12.4 Hz, 1H) ; RMN ¹³C (CDCl₃ ; 101 MHz) δ 166.3 (Cq), 156.0 (Cq), 139.1 (Cq), 136.4 (Cq), 134.0 (Cq), 133.2 (Cq), 129.4 (CH), 128.8 (2 x CH), 127.5 (2 x CH), 127.5 (CH), 126.4 (CH), 119.4 (CH), 65.4 (Cq), 58.1 (CH), 44.9 (CH₂), 44.3 (CH₂), 41.2 (CH₂), 39.2 (SCH₂), 38.9 (SCH₂), 38.5 (CH₂) ; SM (IS) m/z : 426.5 [M+H]⁺, 424.0 [M-H]⁺.

### N-(2-Méthoxyphényl)-N'-(6'-oxo-3',4,4',5,6',10b'-hexahydro-1'H-spiro[1,3-dithiolane-2,2'-pyrido[2,1-a]isoindol]-10'-yl)urée (89).

Le composé **89** est obtenu avec un rendement de 67% sous forme de solide blanc à partir de l'amine 7 selon la procédure générale D après chromatographie sur gel de silice (CH₂Cl₂ / MeOH : 97 / 3 + 1% Et₃N). F : 178°C ; IR (NaCl ν cm⁻¹) : 3345 (N-H amide), 1659 (C=O amide), 1599 (C=C arom), 1540.4 (N-H amide), 1487-1459-1433 (C=C arom), 1287-1251 (C-C) ; RMN ¹H (CDCl₃ ; 400 MHz) δ 8.48 (sb, 1H), 8.22 (dd, *J* = 7.4 Hz, *J* = 2.0 Hz, 1H), 8.16 (sb, 1H), 7.79 (d, *J* = 7.9 Hz, 1H), 7.57 (d, *J* = 7.6 Hz, 1H), 7.34 (t, *J* = 7.8 Hz, 1H), 7.02-6.91 (m, 2H), 6.83 (dd, *J* = 7.6 Hz, *J* = 1.9 Hz, 1H), 4.77 (dd, *J* = 11.5 Hz, *J* = 3.2 Hz, 1H), 4.42 (dd, *J* = 13.4 Hz, *J* = 3.4 Hz, 1H), 3.65 (s, 3H), 3.25-2.86 (m, 6H), 2.09 (d, *J* = 13.4 Hz, 1H), 2.01-1.95 (m, 1H), 1.55 (t, *J* = 12.3 Hz, 1H) ; RMN ¹³C (CDCl*₃* ; 101 MHz) δ 166.6 Cq), 153.2 Cq), 148.5 (Cq), 135.8 (Cq), 134.0 (Cq), 133.2 (Cq), 129.4 (CH), 128.3 (Cq), 126.0 (CH), 123.0 (CH), 121.3 (CH), 120.0 (CH), 119.4 (CH), 110.3 (CH), 65.4 (Cq), 58.2 (CH), 55.8 (CH₃), 45.2 (CH₂), 41.1 (CH₂), 39.3 (CH₂), 39.2 (CH₂), 38.4 (CH₂) ; SM (IS) m/z : 442.5 [M+H]⁺, 464.5 [M+Na]⁺.

### N-(3-Méthoxyphényl)-N'-(6'-oxo-3',4,4',5,6',10b'-hexahydro-1'H-spiro[1,3-dithiolane-2,2'-pyrido[2,1-a]isoindol]-10'-yl)urée (90).

Le composé 90 est obtenu avec un rendement de 60% sous forme de solide blanc à partir de l'amine 7 selon la procédure générale D après chromatographie sur gel de silice (CH₂Cl₂ / MeOH 98 / 2). F : 182°C ; IR (NaCl ν cm⁻¹) : 3345 (N-H amide), 1652 (C=O amide), 1572 (N-H amide), 1470 (C=C arom), 1251 (C-C) ; RMN ¹H (CDCl₃ ; 400 MHz) δ 8.27 (sb, 1H), 8.18 (sb, 1H), 7.67 (d, *J* = 7.8 Hz, 1H), 7.47 (d, *J* = 7.0 Hz, 1H), 7.31 (t, *J* = 7.8 Hz, 1H), 7.12-7.09 (m, 1H), 7.09 (t, *J* = 8.1 Hz, 1H), 6.79 (dd, *J* = 7.9 Hz, *J* = 1.0 Hz, 1H), 6.53 (dd, *J* = 8.2 Hz, *J* = 1.9 Hz, 1H), 4.59 (dd, *J* = 11.4 Hz, *J* = 2.8 Hz, 1H), 4.37 (dd, *J* = 12.9 Hz, *J* = 3.0 Hz, 1H), 3.70 (s, 3H), 3.21-2.76 (m, 5H), 2.78 (d, *J* = 11.9 Hz, 1H), 2.09 (d, *J* = 14.6 Hz, 1H), 1.96-1.92 (m, 1H), 1.51 (t, *J* = 12.3 Hz, 1H) ; RMN ¹³C (CDCl₃ ; 101 MHz) δ 166.4 (Cq), 160.4 (Cq), 153.5 (Cq), 139.8 (Cq), 135.9 (Cq), 133.6 (Cq), 133.1 (Cq), 129.8 (CH), 129.5 (CH), 126.0 (CH), 119.3 (CH), 112.2 (CH), 109.0 (CH), 106.0 (CH), 65.4 (Cq), 58.2 (CH), 55.4 (CH₃), 45.0 (CH₂), 41.1 (CH₂), 39.2 (CH₂), 39.0 (CH₂), 38.5 (CH₂) ; SM (IS) m/z : 352.0 [M+H]⁺, 381.0 [M+K]⁺.

### N-(4-Méthoxyphényl)-N'-(6'-oxo-3',4,4',5,6',10b'-hexahydro-1'H-spiro[1,3-dithiolane-2,2'-pyrido[2,1-a]isoindol]-10'-yl)urée (91).

Le composé **91** est obtenu avec un rendement de 60% sous forme de solide blanc à partir de l'amine **7** selon la procédure générale D après chromatographie sur gel de silice (CH₂Cl₂ / MeOH 98 / 2). F : 183°C ; IR (NaCl ν cm⁻¹) : 3344 (N-H amide), 1651 (C=O amide), 1557 (C=C arom), 1511 (N-H amide), 1427 (C=C arom), 1293-1243 (C-C) ; RMN ¹H (CDCl₃; 400 MHz) *δ* 7.94 (sb, 1H), 7.86 (sb, 1H), 7.51-7.44 (m, 2H), 7.25 (t, *J* = 7.7 Hz, 1H), 7.12 (d, *J* = 8.9 Hz, 2H), 6.70 (d, *J* = 8.9 Hz, 2H), 4.49 (dd, *J* = 11.4 Hz, *J* = 3.0 Hz, 1H), 4.36 (dd, *J* = 13.0 Hz, *J* = 3.4 Hz, 1H), 3.68 (s, 3H), 3.25-3.12 (m, 4H), 3.06 (d, *J* = 12.8 Hz, 1H), 2.67 (d, *J* = 11.6 Hz, 1H), 2.07 (d, *J* = 13.7 Hz, 1H), 1.96-1.84 (m, 1H), 1.88 (td, *J* = 12.9 Hz, *J* = 4.8 Hz, 1H), 1.41 (t, *J* = 12.4 Hz, 1H) ; RMN ¹³C (CDCl₃ ; 101 MHz) δ 166.1 (Cq), 156.5 (Cq), 154.1 Cq), 136.3 (Cq), 133.5 (Cq), 133.2 (Cq), 130.9 (Cq), 129.3 (CH), 126.0 (CH), 123.2 (2 x CH), 119.5 (CH), 114.4 (2 x CH), 65.5 (Cq), 58.1 (CH), 55.5 (OCH₃), 44.8 (CH₂), 41.2 (CH₂), 39.3 (CH₂), 38.8 (CH₂), 38.5 (CH₂) ; SM (IS) m/z : 442.5 [M+H]⁺.

### N-Benzyl-N'-(6-oxo-1,2,3,4,6,10b-hexahydropyrido[2,1-a]isoindol-10-yl) urée (92).

Le composé 92 est obtenu avec un rendement quantitatif sous forme de solide blanc à partir de l'amine 10 selon la procédure générale D après chromatographie sur gel de silice (CH₂Cl₂ / MeOH 98 /2). F : 183°C ; IR (ATR Ge ν cm⁻¹) : 3310 (NH amide), 1696-1631 (C=O amide), 1570 (NH amide), 1525-1480-1419 (C=C arom), 1276 (C-C) ; RMN ¹H (DMSO-*d6* ; 400 MHz) δ 8.35 (s, 1H), 8.32 (sb, 1H), 7.94 (d, *J* = 7.9 Hz, 1H), 7.38-7.26 (m, 5H), 7.10 (t, *J* = 7.1 Hz, 1H), 4.48 (dd, *J* = 11.4 Hz, *J* =3.2 Hz, 1H), 4.32 (qd, *J* = 15.0 Hz, *J* =5.5 Hz, 2H), 4.23 (qd, *J* = 13.0 Hz, *J* = 4.5 Hz, 1H), 2.98 (td, *J* = 12.9 Hz, *J* = 3.3 Hz, 1H), 2.54-2.50 (m, 1H), 1.86 (d, *J* = 12.7 Hz, 1H), 1.74 (d, *J* = 12.7 Hz , 1H), 1.57 (q, *J* = 13.1 Hz, 1H), 1.23-1.21 (m, 1H), 0.77 (qd, *J* = 12.3 Hz, *J* = 2.6 Hz, 1H) ; RMN ¹³C (DMSO-*d6* ; 101 MHz) δ 164.6 (Cq), 154.9 (Cq), 140.0 (Cq), 135.0 (Cq), 134.7 (Cq), 132.7 (Cq), 128.4 (CH), 128.2 (CH), 127.1 (2 x CH), 126.7 (CH), 125.3 (CH), 122.3 (CH), 116.4 (CH), 57.5 (CH), 42.8 (-NCH₂), 38.9 (CH₂), 25.0 (CH₂), 23.0 (CH₂) ; SM (IS) m/z : 336.5 [M+H]⁺.

### N-(2-Méthoxyphényl)-N'-(6-oxo-1,2,3,4,6,10b-hexahydropyrido[2,1-a] isoindol-10-yl)urée (93).

Le composé 93 est obtenu avec un rendement de 45% sous forme de solide blanc à partir de l'amine 10 selon la procédure générale D après chromatographie sur gel de silice (CH₂Cl₂ / MeOH 97 / 3 + 1% Et₃N). F : 177°C ; IR (NaCl ν cm⁻¹) : 3344 (N-H amide), 1601 (C=O amide), 1542 (C=C arom), 1540.4 (N-H amide), 1489-1459-1430 (C=C arom), 1173-1121 (C-C) ; RMN ¹H (CDCl₃ ; 400 MHz) *δ* 8.53 (sb, 1H), 8.19 (dd, *J* = 7.5 Hz, *J* = 1.9 Hz, 1H), 8.14 (sb, 1H), 7.73 (d, *J* = 7.9 Hz, 1H), 7.55 (d, *J* = 6.8 Hz, 1H), 7.32 (t, *J* = 7.7 Hz, 1H), 6.95 (qd, *J* = 7.5 Hz, *J* = 1.9 Hz, 2H), 6.80 (dd, *J* = 7.8 Hz, *J* = 2.0 Hz, 1H), 4.53 (dd, *J* = 11.5 Hz, *J* = 3.2 Hz, 1H), 4.42 (dd, *J* = 13.0 Hz, *J* = 3.5 Hz, 1H), 3.60 (s, 3H), 2.88 (td, *J* = 13.0 Hz, *J* = 3.2 Hz, 1H), 2.61 (dd, *J* = 12.3 Hz, *J* = 2.1 Hz, 1H), 1.82 (d, *J* = 13.0 Hz, 1H), 1.71 (d, *J* = 13.0 Hz, 1H), 1.47 (q, *J* = 13.0 Hz, 1H), 1.28-1.21 (m, 1H), 0.92 (d, *J* = 12.0 Hz, 1H) ; RMN ¹³C (CDCl₃ ; 101 MHz) δ 166.5 (Cq), 153.4 (Cq), 148.4 (Cq), 137.3 (Cq), 133.9 (Cq), 133.2 (Cq), 129.1 (CH), 128.4 (Cq), 125.5 (CH), 122.9 (CH), 121.2 (CH), 119.7 (CH), 119.2 (CH), 110.3 (CH), 59.3 (CH), 55.6 (CH₃), 40.1 (CH₂), 30.2 (CH₂), 25.5 (CH₂), 23.5 (CH₂) ; SM (IS) m/z : 352.0 [M+H]⁺.

### N-(3-Méthoxyphényl)-N'-(6-oxo-1,2,3,4,6,10b-hexahydropyrido[2,1-a]isoindol-10-yl)urée (94).

Le composé 94 est obtenu avec un rendement de 58% sous forme de solide blanc à partir de l'amine 10 selon la procédure générale D après chromatographie sur gel de silice (CH₂Cl₂ / MeOH 98 / 2). F : 183°C ; IR (NaCl ν cm⁻¹) : 3345 (N-H amide), 1652 (C=O amide), 1572 (N-H amide), 1470 (C=C arom), 1251 (C-C) ; RMN ¹H (CDCl₃ ; 400 MHz) δ 8.15 (sb, 1H), 7.99 (sb, 1H), 7.67 (d, *J* = 7.9 Hz, 1H), 7.55 (d, *J* = 7.5 Hz, 1H), 7.35 (t, *J* = 7.7 Hz, 1 ), 7.16-7.13 (m, 2H), 6.82 (d, *J* = 8.0 Hz, 1H), 6.59 (dd, *J* = 8.3 Hz, *J* = 2.0 Hz, 1H), 4.47 (dd, *J* = 11.6 Hz, *J* = 3.4 Hz, 1H), 4.43 (dd, *J* = 13.2 Hz, *J* = 4.3 Hz, 1H), 3.74 (s, 3H), 2.92 (td, *J* = 13.0 Hz, *J* = 3.2 Hz, 1H), 2.54 (d, *J* = 15.0 Hz, 1H), 1.86 (d, *J* = 13.4 Hz), 1H, 1.76 (d, *J* = 12.7 Hz, 1H), 1.49 (q, *J*= 13.2Hz, 1H), 1.32-1.27 (m, 1H), 0.97 (qd, *J* = 12.5 Hz, *J* = 2.7 Hz) , 1H ; RMN ¹³C (CDCl*₃* ; 101 MHz) δ 166.6 Cq), 160.4 Cq), 153.5 (Cq), 139.9 (Cq), 137.5 (Cq), 133.7 (Cq), 133.2 (Cq), 129.9 (CH), 129.3 (CH), 125.8 (CH), 119.4 (CH), 112.2 (CH), 109.0 (CH), 105.9 (CH), 59.3 (CH), 55.4 (CH₃), 40.1 (CH₂), 30.2 (CH₂), 25.5 (CH₂), 23.6 (CH₂) ; SM (IS) m/z : 352.0 [M+H]⁺, 381.0 [M+K]⁺.

### N-(4-Méthoxyphényl)-N'-(6-oxo-1,2,3,4,6,10b-hexahydropyrido[2,1-a] isoindol-10-yl)urée (95).

Le composé 95 est obtenu avec un rendement de 43% sous forme de solide blanc à partir de l'amine 10 selon la procédure générale D après chromatographie sur gel de silice (CH₂Cl₂ / MeOH 96 / 4 + 1% Et₃N). F : 193°C ; IR (NaCl ν cm⁻¹) : 3417 (N-H amide), 1652 (C=O amide), 1553 (C=C arom), 1510 (N-H amide), 1428 (C=C arom), 1289-1242 (C-C). RMN ¹H (CDCl₃; 400 MHz) δ7.77 (sb, 1H), 7.71 (sb, 1H), 7.63 (d, *J* = 7.9 Hz, 1H), 7.52 (d, *J* = 7.4 Hz, 1H), 7.32 (t, *J* = 7.7 Hz, 1H), 7.21 (d, *J* = 8.8 Hz, 2H), 6.79 (d, *J* = 8.8 Hz, 2H), 4.42-4.37 (m, 2H), 3.74 (s, 3H), 2.88 (td, *J* = 13.0 Hz, *J* = 3.1 Hz, 1H), 2.43 (d, *J* = 2.4 Hz, 1H), 1.84 (d, *J* = 13.4 Hz, 1H), 1.75-1.73 (m, 1H), 1.47 (q, *J* = 13.1 Hz, 1H), 1.31-1.24 (m, 1H), 0.92 (qd, *J* = 14.0 Hz, *J* = 2.5 Hz, 1H) ; RMN ¹³C (CDCl₃ ; 101 MHz) δ 166.4 (Cq), 158.8 (Cq), 154.0 Cq), 137.3 (Cq), 133.7 (Cq), 133.4 (Cq), 131.0 (Cq), 129.3 (2 x CH), 125.6 (CH), 123.0 (CH), 119.4 (CH), 114.6 (2 x CH), 59.1 (CH), 55.6 (CH₃), 40.0 (CH₂), 30.1 (CH₂), 25.4 (CH₂), 23.6 (CH₂) ; SM (IS) m/z : 352.0 [M+H]⁺.

### N-(6-Méthoxypyridin-3-yl)-N'-(6-oxo-1,2,3,4,6,10b-hexahydropyrido [2,1-a]isoindol-10-yl)urée (96).

Le composé 96 est obtenu avec un rendement de 72% sous forme de solide blanc à partir de l'amine 10 selon la procédure générale D après chromatographie sur gel de silice (CH₂Cl₂ / MeOH 97 / 3 + 1% Et₃N). F : 175°C ; IR (ATR-Ge ν cm⁻¹) : 1655 (C=O amide), 1549 (NH amide), 1484-1427 (C=C arom), 1252 (C-C) ; RMN ¹H (CDCl₃ ; 400 MHz) δ 8.62 (sb, 1H), 8.41 (sb, 1H), 8.08 (d, *J* = 2.6 Hz, 1H), 7.77 (dd, *J* = 2.7 Hz, *J* = 8.8 Hz, 1H), 7.73 (d, *J* = 7.8 Hz, 1H), 7.48 (d, *J* = 7.8 Hz, 1 H), 7.32 (t, *J* = 7.8 Hz, 1H), 6.66 (d, *J* = 8.8 Hz, 1H), 4.51 (dd, *J* = 3.3 Hz, *J* = 11.6 Hz, 1H), 4.39 (dd, *J* = 4.4 Hz, *J* = 13.0 Hz, 1H), 3.86 (s, 3H), 3.07 (q, *J* = 7.3 Hz, 1H), 2.89 (td, *J* = 3.2 Hz, *J* = 13.0 Hz, 1H), 2.64 (m, 1H), 1.85 (d, *J* = 12.7 Hz, 1H), 1.75 (d, *J* = 13.3 Hz, 1H), 1.51 (q, *J* = 13.3 Hz, 1H), 0.93 (qd, *J* = 12.8 Hz, *J* = 2.6 Hz, 1H) ; RMN ¹³C (CDCl₃ ; 101 MHz) δ 166.6 (Cq), 160.7 (Cq), 153.9 (Cq), 138.7 (CH), 137.2 (Cq), 133.9 (Cq), 133.1 (Cq), 132.5 (CH), 129.4 (Cq), 129.3 (CH), 125.5 (CH), 119.1 (CH), 110.6 (CH), 59.4 (CH), 53.7 (OCH₃), 40.1 (CH₂), 30.2 (CH₂), 25.5 (CH₂), 23.5 (CH₂) ; SM (IS) m/z : 353.5 [M+H]⁺, m/z = 351.0 [M-H]⁺.

### 2.5. Procédure générale E

### Synthèse des cétones (97) à (103) à partir des acétals correspondants.

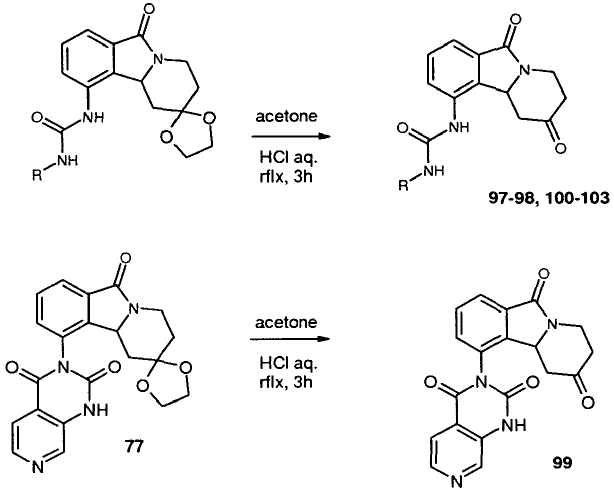

Dans un ballon de 50 mL, l'acétal (0.52 mmol) est solubilisé dans 4 mL d'acétone puis une solution aqueuse d'acide chlorhydrique 10% (2 mL) est ajoutée. Le mélange réactionnel est porté au reflux pendant 3h. Après refroidissement, le milieu réactionnel est concentré à 50% environ par évaporation puis le précipité est filtré, lavé avec un peu d'eau (2 mL) et séché sous pression réduite pour donner le produit cétonique attendu.

### 1-(2,6-Dioxo-1,2,3,4,6,10b-hexahydropyrido[2,1-a]isoindol-10-yl)-3-(pyridin-2-yl)urée (97).

Le composé **97** est obtenu selon la procédure E à partir du dérivé 25 sous forme de solide blanc avec un rendement quantitatif. F : 254°C ; RMN ¹H (DMSO-*d6* ; 400 MHz) δ 11.26 (s, 1H), 9.99 (s, 1H), 8.40-8.34 (m, 1H), 8.30 (d, *J* = 8.0 Hz, 1H), 7.85 - 7.76 (m, 1H), 7.51 (t, *J* = 7.7 Hz, 1H), 7.45 (d, *J* = 6.8 Hz, 1H), 7.30 (d, *J* = 8.3 Hz, 1H), 7.10 (dd, *J* = 5.4 Hz, *J* = 6.7 Hz, 1H), 5.08 (dd, *J* = 3.9 Hz, 11.9 Hz, 1H), 4.48 (m, 1H), 3.49 (td, *J* = 4.4 Hz, *J* = 12.6 Hz, 1H), 3.26 (dd, *J* = 2.8 Hz, *J* = 14.0 Hz, 1H), 2.66 - 2.57 (m, 1H), 2.45 - 2.38 (m, 1H), 2.34 (t, *J* = 12.0 Hz, 1H) ; RMN ¹³C (DMSO-*d6* ; 101 MHz) δ : 206.35 (Cq), 165.28 (Cq), 152.82 (Cq), 152.13 (Cq), 146.23 (CH), 139.11 (CH), 134.00 (Cq), 133.75 (Cq), 132.51 (Cq), 129.41 (CH), 122.74 (CH), 117.56 (CH), 117.55 (CH), 112.16 (CH), 55.98 (CH), 43.98 (CH₂), 39.00 (CH₂), 36.59 (CH₂) ; IR (ATR-Ge ν cm⁻¹) : 1692 (C=O amide), 1623 (C=O cétone), 1568 (N-H amide), 1479- 1457-1421 (C=C arom), 1309 (C-N), 1243 (C-C), 751 (C-H arom) ; SM (IS) m/z : 337.0 [M+H]⁺.

### 1-(2,6-Dioxo-1,2,3,4,6,10b-hexahydropyrido[2,1-a]isoindol-10-yl)-3-(pyrazin-2-yl)urée (98).

Le composé 98 est obtenu selon la procédure E à partir du dérivé 26 sous forme de solide beige avec 95% de rendement. F > 260°C ; IR (ATR-Ge ν cm⁻¹) : 1689 (C=O amide), 1660 (C=O cétone), 1569 (N-H amide), 1502- 1477-1430 (C=C arom), 1304 (C-N), 1143- 1065 (C-C) ; RMN ¹H (DMSO-*d6* ; 400 MHz) δ 10.12 (s, 1H), 9.86 (s, 1H), 8.90 (d, *J* = 1.2 Hz, 1H), 8.37 - 8.35 (m, 1H), 8.32 (d, *J* = 2.7 Hz, 1H), 8.18 (dd, *J* = 0.9 Hz, 7.9 Hz, 1H), 7.53 (t, *J* = 7.7 Hz, 1H), 7.48 (dd, *J* = 1.0 Hz, *J* = 7.4 Hz, 1H), 5.05 (dd, *J* = 3.9 Hz, *J* = 11.9 Hz, 1H), 4.49 - 4.44 (m, 1H), 3.48 (td, *J* = 4.4 Hz, *J* = 12.8 Hz, 1H), 3.17 (dd, *J* = 2.7 Hz, *J* = 14.0 Hz, 1H), 2.67 - 2.57 (m, 1H), 2.44 - 2.37 (m, 1H), 2.31 (dd, *J* = 2.7 Hz, *J* = 13.6 Hz, 1H) ; RMN ¹³C (DMSO-d6 ;101 MHz) δ 206.34 (Cq), 165.17 (Cq), 151.66 (Cq), 149.14 (Cq), 140.97 (CH), 137.87 (CH), 135.48 (CH), 134.40 (Cq), 133.51 (Cq), 132.62 (Cq), 129.42 (CH), 123.43 (CH), 118.14 (CH), 55.99 (CH), 43.92 (CH₂), 38.99 (CH₂), 36.58 (CH₂) ; SM (IS) m/z : 338.0 [M+H]⁺.

### 10-(2,4-dioxo-1,2-dihydropyrido[3,4-d]pyrimidin-3(4H)-yl)-1,3,4,10b-tétrahydropyrido[2,1-a]isoindole-2,6-dione (99).

Le composé 99 est obtenu selon la procédure à partir du dérivé 77 sous forme de solide blanc avec 76 % de rendement après chromatographie sous gel de silice flash (CH₂Cl₂ / MeOH : 99 /1). F : 230°C ; IR (ATR-Ge ν cm⁻¹) : 3403 (N-H amide), 1677 (C=O cétone), 1489-1421 (C=C arom), 1272 (C-N) ; RMN ¹H (DMSO-*d6* ; 400 MHz) δ 12.03 (s, 1H), 8.71 (s, 1H), 8.48 (t, *J* = 4.5 Hz, 1H), 7.87 - 7.82 (m, 2H), 7.69 (t, *J* = 7.6 Hz, 1H), 7.63 (d, *J* = 7.7 Hz, 1H), 4.93 - 4.85 (m, 1H), 4.43 - 4.29 (m, 1H), 3.46 - 3.37 (m, 1H), 2.57 - 2.51 (m, 2H), 2.45 - 2.29 (m, 1H), 2.20 (dd, *J* = 13.9 Hz, 26.1 Hz, 1H) ; RMN ¹³C (DMSO-*d6* ; 101 MHz) δ 205.6 (Cq), 164.7 (Cq), 161.7 (Cq), 161.1 (Cq), 149.8 (Cq), 149.2 (Cq), 143.0 (CH), 142.8 (Cq), 138.7 (CH), 133.2 (Cq), 132.8 (CH), 130.6 (Cq), 129.8 (CH), 123.7 (CH), 119.6 (CH), 55.5 (CH), 44.0 (CH₂), 38.6 (CH₂), 36.3 (CH₂). SM (IS) m/z : 363.5 [M+H]⁺.

### 1-(2,6-dioxo-1,2,3,4,6,10b-hexahydropyrido[2,1-a]isoindol-10-yl)-3-(3-méthylpyridin-2-yl)urée (100).

Le composé 100 a été obtenu selon la procédure D à partir du composé 45 avec un rendement de 85%, sous la forme d'un solide blanc. p.f. > 260°C; ¹H RMN (DMSO- d₆, 400 MHz) δₚₚₘ : 11.51 (s, 1H), 10.91 (s, 1H), 8.30 (d, *J* = 8.0 Hz, 1H), 8.18 (d, *J* = 7.5 Hz, 1H), 8.06 (d, *J* = 7.5 Hz, 1H), 7.58 (m, 2H), 6.84 (dd, *J* = 7.5 Hz, *J* = 8.0 Hz, 1H), 5.21 (dd, *J* = 2.5 Hz, *J* = 12.5 Hz, 1H), 4.41-4.50 (m, 1 H), 3.33-3.54 (m, 2H), 2.39-2.65 (m, 5H), 2.24 (t, *J* = 12.5 Hz, 1H). ¹³C RMN (DMSO- d₆, 100 MHz) : 206.3 (Cq), 165.5 (Cq), 153.2 (Cq), 148.4 (Cq), 145.5 (CH), 137.0 (CH), 136.1 (Cq), 133.3 (Cq), 132.9 (Cq), 129.9 (CH), 124.7 (CH), 125.3 (CH), 119.5 (CH), 118.8 (CH), 56.5 (CH), 44.1 (CH₂), 39.3 (CH₂), 36.9 (CH₂), 17.9 (CH₃). IR (ATR-Ge ν cm⁻¹) : 1684 (C=O amide), 1621 (C=O cétone), 1559 (N-H amide), 1479- 1434-1414 (C=C arom), 1329 (C-N), 1289 (C-C), 751 (C-H arom). SM (IS) m/z : 351.0 [M+H]⁺

### 1-(2,6-dioxo-1,2,3,4,6,10b-hexahydropyrido[2,1-a]isoindol-10-yl)3-(6-méthyl-pyridin-2-yl)urée (101).

Le composé 101 a été obtenu selon la procédure D à partir du composé **46** avec un rendement de 79%, sous la forme d'un solide blanc. p.f. 226-228°C; IR (ATR-Ge ν cm⁻¹) : 1683 (C=O amide), 1621 (C=O cétone), 1566 (N-H amide), 1467- 1435 (C=C arom), 1323 (C-N), 1287 (C-C), 752 (C-H arom). ¹H RMN (DMSO-d₆, 400 MHz) δₚₚₘ: 10.70 (s, 1H), 9.84 (s, 1H), 8.18 (d, *J* = 7.5 Hz, 1H), 7.63 (dd, *J* = 7.5 Hz, *J* = 8.0 Hz, 1H), 7.46-7.55 (m, 2H), 7.17 (d, *J* = 8.0 Hz, 1H), 6.91 (d, *J* = 7.5 Hz, 1H), 5.08 (dd, *J* = 2.8 Hz, *J* = 12.2 Hz, 1H), 4.48 (dd, *J* = 2.8 Hz, *J* = 12.2 Hz, 1H), 3.45 (td, *J* = 5.0 Hz, *J* = 12.7 Hz, 1H), 3.17 (d, *J* = 5.0 Hz, 1H), 3.11 (dd, *J* = 2.5 Hz, J = 12.8 Hz, 1H), 2.54-2.68 (m, 1H), 2.44 (s, 3H), 2.37 (t, *J* = 12.5 Hz, 1H). ¹³C RMN (DMSO- d₆, 100 MHz) : 206.8 (Cq), 165.7 (Cq), 155.9 (Cq), 152.7 (Cq), 152.6 (Cq), 139.6 (CH), 134.8 (Cq), 134.2 (Cq), 132.9 (Cq), 129.7 (CH), 124.3 (CH), 118.3 (CH), 117.3 (CH), 109.4 (CH), 55.9 (Cq), 43.6 (CH₂), 39.0 (CH₂), 36.5(CH₂), 23.5 (CH₃). SM (IS) m/z : 351.0 [M+H]⁺

### 1-(2,6-dioxo-1,2,3,4,6,10b-hexahydropyrido[2,1-a]isoindol-10-yl)-3-(4-methoxyquinolin-2-yl)urea (102).

Le composé **102** a été obtenu selon la procédure **D** à partir du composé **48** avec un rendement de 83%, sous la forme d'un solide blanc. p.f. 223-225°C. IR (ATR-Ge ν cm⁻¹) : 1683 (C=O amide), 1621 (C=O cétone), 1558 (N-H amide), 1469-1435 (C=C arom), 1329 (C-N), 1289 (C-C), 751 (C-H arom). ¹H RMN (DMSO- d₆, 400 MHz) δₚₚₘ, : 11.33 (s, 1H), 11.01 (s, 1H), 7.99-8.10 (m, 3H), 7.81 (dd, *J* = 7.5 Hz, J = 8.0 Hz, 1H), 7.54-7.57 (m, 3H), 7.01 (s, 1H), 5.18 (dd, *J* = 2.8 Hz, *J* = 12.5 Hz, 1H), 4.43 (dd, *J* = 2.8 Hz, *J* = 12.2 Hz, 1H), 4.12 (s, 3H), 3.50 (td, *J* = 2.5 Hz, *J* = 12.8 Hz, 1H), 3.18(d, *J* = 12.5 Hz, 1H), 2.52-2.60 (m, 1H), 2.40 (d, *J*= 12.5 Hz, 1H), 0.76 (t, *J* = 12.5 Hz, 1H). ¹³C RMN (DMSO- d₆, 100 MHz) : 205.7 (Cq), 165.1 (Cq) , 165.0 (Cq), 152.2 (Cq), 151.9 (Cq), 135.7 (Cq), 132.7 (Cq), 132.6 (Cq), 132.3 (CH), 129.3 (2 CH), 125.4 (CH), 124.8 (CH), 122.6 (Cq), 121.9 (CH), 118.9 (CH), 117.5 (Cq), 91.9 (CH), 57.0 (CH), 55.9 (CH₃), 43.4 (CH₂), 38.8 (CH₂), 36.4 (CH₂). HRMS : calc. pour C₂₃ H₂₁ N₄ O₄ : 417,1563 ; trouvé 417,1571.

### 1-(2,6-Dioxo-1,2,3,4,6,10b-hexahydropyrido[2,1-a]isoindol-10-yl)-3-(4-hydroxyquinolin-2-yl)urea (103).

Le composé 103 a été obtenu selon la procédure D à partir du composé **61** avec un rendement de 80%, sous la forme d'un solide blanc. p.f. > 260°C. IR (ATR-Ge ν cm⁻¹) : 2872 (O-H), 1695 (C=O amide), 1592 (C=O cétone), 1535 (N-H amide), 1419 (C=C arom), 1348 (C-N), 1296 (C-C), 760 (C-H arom). ¹H RMN (DMSO- d₆, 400 MHz) δₚₚₘ: 11.94 (s, 1H), 10.23 (s, 1H), 8.13 (dd, *J* = 8.4 Hz, *J* = 10.0 Hz, 2H), 8.01 (d, *J* = 8.4 Hz, 1H), 7.88 (t, *J* = 8.0 Hz, 1H), 7.54-7.62 (m, 3H), 7.02 (s, 1H), 5.12 (dd, *J* = 4.0 Hz, *J* = 11.6 Hz, 1H), 4.39-4.45 (m, 1H), 3.46 (td, *J* = 4.0 Hz, *J* = 12.8 Hz, 1H), 3.26 (dd, *J* = 4.0 Hz, *J* = 14.0 Hz, 1H), 2.51-2.60 (m, 1H), 2.40 (d, *J* = 13.6 Hz, 1H), 2.23 (t, *J* = 13.6 Hz, 1H). ¹³C RMN (DMSO- *d*₆, 100 MHz) δₚₚₘ: 206.3 (Cq), 165.3 (Cq), 152.4 (2 Cq), 151.2 (Cq), 136.5 (Cq), 133.9 (CH), 133.3 (Cq), 132.7 (Cq), 129.9 (2 CH), 126.4 (Cq), 125.4 (CH), 123.4 (CH), 120.6 (Cq), 119.8 (CH), 118.2 (Cq), 94.6 (CH), 56.4 (CH), 44.0 (CH₂), 39.3 (CH₂), 36.9 (CH₂). HRMS : calc. pour C₂₂ H₁₉ N₄ O₄ : 403,1406 ; trouvé 403,1397.

### 2.6. Procédure générale F

### Réduction de cétones en alcools (104)-(110).

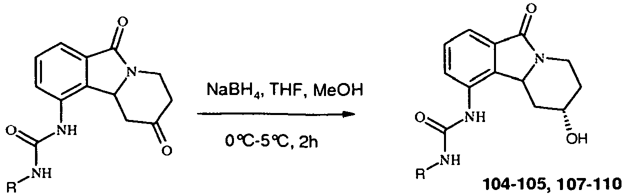

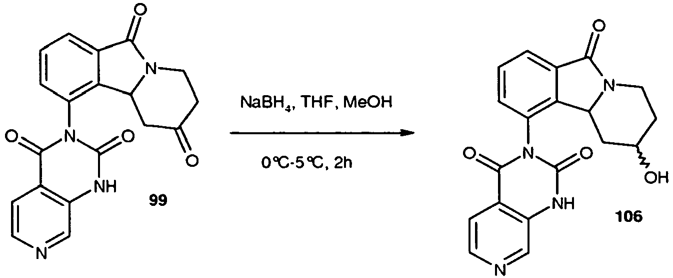

Une solution de cétone (0.2 mmol) dissoute dans un mélange THF : MeOH /1:2 (2 mL / 4 mL) est placée à -20°C sous agitation. Le borohydrure de sodium (15 mg, 0.4 mmol, 2.0 éq.)) est ajouté par portions. Le milieu réactionnel est agité pendant 30 min. puis à une température comprise entre 0°C et 5°C pendant 2 heures. Les solvants sont évaporés sans chauffer puis le résidu est filtré, lavé successivement avec de l'eau (0.4 mL) puis une solution aqueuse saturée en NaHCO₃ (0.9 mL). Après dissolution dans du dichlorométhane (10 mL), la phase organique est séchée sur MgSO₄, filtrée puis évaporée sous pression réduite. Le brut réactionnel est purifié par chromatographie sur gel de silice pour donner l'alcool attendu sous forme d'un seul diastéréoisomère.

### 1-((2S)-2-Hydroxy-6-oxo-1,2,3,4,6,10b-hexahydropyrido[2,1-a]iso-indol-10-yl)-3-(pyridin-2-yl)urée (104).

Le composé **104** est obtenu selon la procédure F à partir du dérivé 97 sous forme de solide blanc avec 60% de rendement après chromatographie sou gel de silice flash (dichlorométhane / méthanol 93 / 7). F : 220°C ; IR (ATR-Ge ν cm⁻¹) : 3217 (O-H), 1695 (C=O amide), 1562 (N-H amide), 1512- 1478-1430 (C=C arom), 1309 (C-O) ; RMN ¹H (DMSO-d6 ; 400 MHz) δ 11.21 (s, 1H), 9.96 (s, 1H), 8.32 (d, *J* = 4.5 Hz, 1H), 8.21 (d, *J* = 7.9 Hz, 1H), 7.81 (t, *J* = 7.1 Hz, 1H), 7.46 (t, *J* = 7.7 Hz, 1H), 7.38 (d, *J* = 7.3 Hz, 1H), 7.33 (d, *J* = 8.4 Hz, 1H), 7.13 - 7.04 (m, 1H), 4.69 (dd, J = 2.8 Hz, *J* = 11.7 Hz, 1H), 4.24 (dd, *J* = 4.1 Hz, *J* = 13.2 Hz, 1H), 3.96 (t, *J* = 10.7 Hz, 1H), 3.60 (s, 1H), 3.08 (td, *J* = 4.1 Hz, *J* = 12.0 Hz, 1H), 2.89 (d, *J* = 11.7 Hz, 1H), 1.95 (d, *J* = 11.6 Hz, 1H), 1.28 - 1.10 (m, 1H), 0.77 (q, *J* = 12.0 Hz, 1H) ; RMN ¹³C (DMSO-*d6* ; 101 MHz) δ 64.68 (Cq), 152.77 (Cq), 152.13 (Cq), 146.18 (CH), 139.13 (CH), 134.40 (Cq), 133.82 (Cq), 132.74 (Cq), 128.94 (CH), 122.56 (CH), 117.61 (CH), 117.51 (CH), 112.15 (CH), 66.72 (CH), 56.04 (CH), 38.75 (CH₂), 36.62 (CH₂), 34.50 (CH₂) ; SM (IS) m/z : 339.0 [M+H]⁺.

### 1-((2S)-2-Hydroxy-6-oxo-1,2,3,4,6,10b-hexahydropyrido[2,1-a]isoindol-10-yl)-3-(pyrazin-2-yl)urée (105).

Le composé 105 est obtenu selon la procédure F à partir du dérivé 98 sous forme de solide blanc avec 76% de rendement après chromatographie sou gel de silice flash (dichlorométhane / méthanol 96 / 4). F : 250°C ; IR (ATR-Ge ν cm⁻¹) : 1694 (C=O amide), 1671 (C-N), 1622 (N-H amide), 1568- 1545-1500-1485 (C=C arom), 1427 (C-N), 1296 (C-O), 1056 (C-C) ; RMN ¹H (DMSO-*d6* ; 400 MHz) δ 10.08 (s, 1H), 9.85 (s, 1H), 8.94 (d, *J* = 1.2 Hz, 1H), 8.38 - 8.32 (m, 1H), 8.31 (d, *J* = 2.7 Hz, 1H), 8.10 (dd, *J* = 0.6 Hz, *J* = 7.8 Hz,, 1H), 7.48 (t, *J* = 7.7 Hz, 1H), 7.42 (dd, *J* = 0.8 Hz, *J* = 7.4 Hz, 1H), 5.01 (d, *J* = 4.9 Hz, 1H), 4.65 (dd, *J* = 3.1 Hz, *J* = 11.8 Hz, 1H), 4.24 (dd, *J* = 3.9 Hz, *J* = 13.4 Hz, 1H), 3.95 - 3.88 (m, 1H), 3.07 (td, *J* = 3.1 Hz, *J* = 13.2 Hz, 1H), 2.78 (d, *J* = 11.8 Hz, 1H), 1.94 (d, *J* = 12.1 Hz, 1H), 1.28 - 1.08 (m, 1H), 0.75 (q, *J* = 11.7 Hz, 1H) ; RMN ¹³C (DMSO-*d6* ; 101 MHz) δ 164.59 (Cq), 151.67 (Cq), 149.17 (Cq), 141.14 (CH), 137.90 (CH), 135.39 (CH), 134.97 (Cq), 133.37 (Cq), 132.85 (Cq), 128.96 (CH), 123.27 (CH), 118.07 (CH), 66.69 (CH), 56.05 (CH), 38.71 (CH₂), 36.63 (CH₂), 34.40 (CH₂) ; SM (IS) m/z : 340.5 [M+H]⁺, 679.5 [2M+H]⁺.

### 3-(2-hydroxy-6-oxo-1,2,3,4,6,10b-hexahydropyrido[2,1-a]isoindol-10-yl)pyrido[3,4-d]pyrimidine-2,4(1H,3H)-dione (106).

Le composé 106 est obtenu selon la procédure F à partir de du dérivé 99 sous forme de solide beige avec 52 % de rendement après chromatographie sous gel de silice flash (CH₂Cl₂ / MeOH : 99 /1). IR (ATR-Ge ν cm⁻¹) : RMN ¹H *(CDCl₃;* 400 MHz) δ 8.68 (s, 1H), 8.44 (dd, *J* = 5.3 Hz, 6.2 Hz, 1H), 7.96 (t, *J* = 4.0 Hz, 1H), 7.91 (d, *J* = 7.6 Hz, 1H), 7.70 (t, *J* = 7.7 Hz, 1H), 7.62 - 7.53 (m, 1H), 4.59 - 4.48 (m, 1H), 4.40 (dd, *J* = 4.7 Hz, 13.5 Hz, 1H), 3.94 - 3.80 (m, 1H), 3.18 - 3.04 (m, 1H), 2.27 - 2.14 (m, 1H), 2.02(d,*J*= 12.4Hz, 1H), 1.41 - 1.24 (m, 1H), 1.13 - 0.95 (m, 1H).

### 1-(2-Hydroxy-6-oxo-1,2,3,4,6,10b-hexahydropyrido[2,1-a]isoindol-10-yl)-3-(3-méthylpyridin-2-yl)urée (107).

Le composé 107 a été obtenu selon la procédure F à partir du composé 100 après purification par chromatographie flash (CH₂Cl₂ / MeOH : 99 /1) sous la forme d'un solide blanc. Rendement : 82 %. p.f. 204-206°C; IR (ATR-Ge ν cm⁻¹) : 2960 (OH), 1684 (C=O amide), 1584 (N-H amide), 1503- 1486-1420 (C=C arom), 1262 (C-O). ¹H RMN (DMSO- d₆, 400 MHz) δₚₚₘ : 12.41 (s, 1H), 8.91 (s, 1H), 8.34 (d, *J* = 8.0 Hz, 1H), 8.28 (d, *J* = 7.5 Hz, 1H), 7.73 (d, *J* = 7.5 Hz, 1H), 7.53 (dd, *J* = 7.5 Hz, *J* = 8.0 Hz, 1H), 7.44 (d, *J*= 7.5 Hz, 1H), 7.12 (dd, *J* = 7.5 Hz, *J*= 8.0 Hz, 1H), 5.03 (d, *J* = 5.0 Hz, 1H), 4.77 (dd, *J* = 2.8 Hz, *J* = 12.2 Hz, 1H), 4.30 (dd, *J* = 2.8 Hz, *J* = 12.2 Hz, 1H), 4.00-4.09 (m, 1H), 3.15 (td, *J* = 2.5 Hz, *J* = 12.8 Hz, 1H), 2.96 (d, *J* = 12.5 Hz, 1H), 2.14 (s, 3H), 2.02 (d, *J* = 12.5 Hz, 1H), 1.09-1.25 (m, 1H), 0.85 (q, *J* = 12.5 Hz, 1H). ¹³C RMN (DMSO- d₆, 100 MHz) : 165.2 (Cq), 152.8 (Cq), 151.6 (Cq), 143.4 (CH), 140.5 (CH), 134.8 (Cq), 134.3 (Cq), 133.1 (Cq), 129.4 (CH), 122.9 (CH), 121.7 (Cq), 118.3 (CH), 117.9 (CH), 67.2 (CH), 56.4 (CH), 37.1 (CH₂), 35.0 (CH₂), 31.1 (CH₂), 17.4 (CH₃). HRMS : calc. pour C₁₉ H₂₁ N₄ O₃ : 353, 1614 ; trouvé 353,1616.

### 1-(2-Hydroxy-6-oxo-1,2,3,4,6,10b-hexahydropyrido[2,1-a]isoindol-10-yl)-3-(6-méthylpyridin-2-yl)urée (108).

Le composé **108** a été obtenu selon la procédure F à partir du composé **101** après purification par chromatographie flash (CH₂Cl₂ / MeOH : 99 /1) sous la forme d'un solide blanc. Rendement : 80 %. p.f. 240-242°C ; IR (ATR-Ge ν cm⁻¹) : 3143 (O-H), 1685 (C=O amide), 1589 (N-H amide), 1508-1484-1424 (C=C arom), 1270 (C-O). ¹H RMN (DMSO- d₆, 400 MHz) δₚₚₘ: 10.72 (s, 1H), 9.82 (s, 1H), 8.15 (d, *J* = 7.5 Hz, 1H), 7.66 (dd, *J* = 7.5 Hz, *J* = 8.0 Hz, 1H), 7.38-7.49 (m, 2H), 7.16 (d, *J* = 8.0 Hz, 1H), 6.92 (d, *J* = 7.5 Hz, 1H), 4.99 (d, *J* = 5.0 Hz, 1H), 4.71 (dd, *J* = 2.8 Hz, *J* = 12.2 Hz, 1H), 4.26 (dd, *J* = 2.8 Hz, *J* = 12.2 Hz, 1H), 3.84-3.87 (m, 1H), 3.05 (td, *J* = 2.5 Hz, *J* = 12.8 Hz, 1H), 2.75-2.80 (m, 1H), 2.51 (s, 3H), 1.92 (d, *J* = 12.5 Hz, 1H), 1.12-1.18 (m, 1H), 0.76 (q, *J* = 12.5 Hz, 1H). ¹³C RMN (DMSO- d₆, 100 MHz) : 164.6 (Cq), 155.3 (Cq), 152.1 (Cq), 152.0 (Cq), 139.0 (CH), 134.6 (Cq), 133.6 (Cq), 132.6 (Cq), 128.7 (CH), 123.4 (CH), 117.6 (CH), 116.7 (CH), 108.8 (CH), 66.6 (CH), 55.9 (CH), 38.2 (CH₂), 36.5 (CH₂), 34.2 (CH₂), 23.7 (CH₃). HRMS : calc. pour C₁₉ H₂₁ N₄ O₃ : 353, 1614 ; trouvé 353,1619.

### 1-(2-Hydroxy-6-oxo-1,2,3,4,6,10b-hexahydropyrido[2,1-a]isoindol-10-yl)-3-(4-méthoxyquinolin-2-yl)urée (109).

Le composé **109** a été obtenu selon la procédure **F** à partir du composé **102** après purification par chromatographie flash (CH₂Cl₂ / MeOH : 99 /1) sous la forme d'un solide blanc. Rendement : 82 %. p.f. > 260°C ; IR (ATR-Ge ν cm⁻¹) : 2977 (OH), 1686 (C=O amide), 1585 (N-H amide), 1512- 1479-1414 (C=C arom), 1289 (C-O). ¹H RMN (DMSO- d₆, 400 MHz) δₚₚₘ: 11.97 (s, 1H), 10.15 (s, 1 H), 8.20 (d, *J* = 7.5 Hz, 1H), 8.03 (d, *J* = 8.0 Hz, 1H), 7.88 (d, *J* = 8.0 Hz, 1H), 7.73 (dd, *J* =7.5 Hz, *J* = 8.0 Hz, 1H), 7.41-7.53 (m, 3H), 6.87 (s, 1H), 4.89 (d, *J* = 12.5 Hz, 2H), 4.26 (dd, *J* = 2.8 Hz, *J* = 12.5 Hz, 1H), 4.02 (s, 3H), 3.77-3.88 (m, 1H), 3.14 (td, *J* = 2.8 Hz, *J* = 12.5 Hz, 1H), 2.79 (d, *J* = 12.5 Hz, 1H), 1.94 (d, *J* = 12.5 Hz, 1H), 1.03-1.23 (m, 1H), 0.76 (q, *J* = 12.5 Hz, 1H). ¹³C RMN (DMSO- d₆, 100 MHz) : 164.5 (Cq), 162.9 (Cq), 153.6 (Cq), 152.2 (Cq), 145.6 (Cq), 134.9 (Cq), 133.4 (Cq), 132.7 (Cq), 130.7 (CH), 128.8 (CH), 126.0 (CH), 123.9 (CH), 123.7 (CH), 121.3 (CH), 118.0 (Cq), 117.8 (CH), 91.8 (CH), 66.6 (CH), 56.1 (CH), 56.0 (CH₃), 38.0 (CH₂), 36.4 (CH₂), 34.4 (CH₂). HRMS : calc. pour C₂₃H₂₃N₄O₄ 419,1719 ; trouvé 419,1728.

### 1-(2-Hydroxy-6-oxo-1,2,3,4,6,10b-hexahydropyrido[2,1-a]isoindol-10-yl)-3-(4-hydroxyquinolin-2-yl)urée (110).

Le composé 110 a été obtenu selon la procédure F à partir du composé 103 après purification par chromatographie flash (CH₂Cl₂ / MeOH : 99 /1) sous la forme d'un solide blanc. Rendement : 82 %. p.f. > 260°C ; IR (ATR-Ge ν cm⁻¹) : 3343 (OH), 1680 (C=O amide), 1564 (C=O cétone), 1504 (N-H amide), 1425 (C=C arom), 1353 (C-N), 1294 (C-C), 754 (C-H arom). ¹H RMN (DMSO- d₆, 400 MHz) δₚₚₘ: 13.66 (s, 1H), 8.98 (s, 1H), 8.27 (d, *J* = 8.0 Hz, 1H), 8.96 (d, *J* = 8.4 Hz, 1H), 7.46 (t, *J* = 8.4 Hz, 2H), 7.42 (d, *J* = 7.6 Hz, 1H), 7.31-7.35 (m, 2H), 6.96 (t, *J* = 7.6 Hz, 1H), 5.60 (s, 1H), 4.87- 4.90 (m, 2H), 4.24 (dd, *J* = 3.6 Hz, *J* = 12.8 Hz, 1H), 3.83-3.90 (m, 1H), 3.15 (td, *J* = 3.6 Hz, *J* = 12.8 Hz, 1H), 3.93 (d, *J* = 12.0 Hz, 1H), 1.93 (d, *J* = 12.8 Hz, 1H), 1.06-1.17 (m, 1H), 0.73 (q, *J* = 12.0 Hz, 1H). ¹³C RMN (DMSO- d₆, 100 MHz) δₚₚₘ: 174.6 (Cq), 164.9 (Cq), 154.9(Cq), 153.8 (Cq), 147.5 (Cq), 134.8 (Cq), 134.2 (Cq), 132.5 (Cq), 128.7 (CH), 128.0 (CH), 126.8 (Cq), 124.7 (CH), 123.9 (CH), 123.0 (CH), 119.1 (CH), 116.6 (CH), 93.9 (CH), 66.6 (CH), 56.4 (CH), 38.0 (CH₂), 36.6 (CH₂), 34.6 (CH₂). HRMS : calc. pour C₂₂ H₂₀ N₄ O₄ Na 427,1382 ; trouvé 427,1378.

### 2.7. Procédure générale G

### Synthèse des urées (113)-(117) à base de pyridopyrimidinediones à partir des amines (5) ou (10).

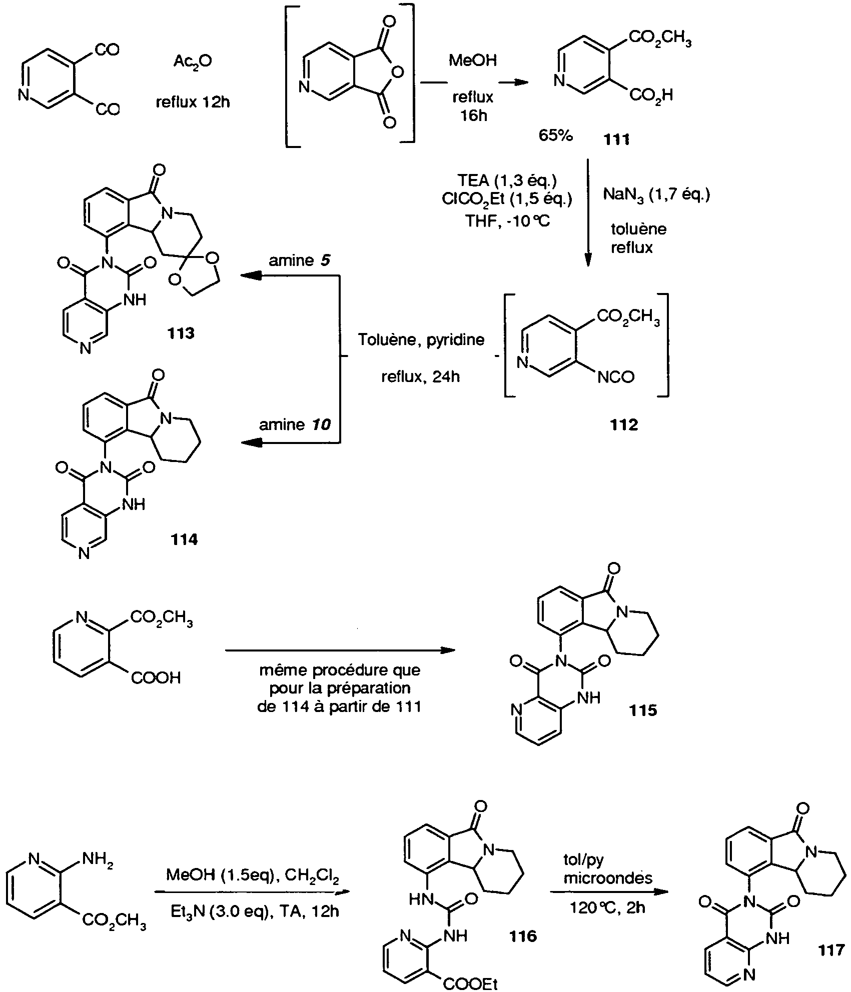

***Synthèse de l'hémi-ester 111*** : L'acide pyridine-3,4-dicarboxylique (2.0 g, 11.05 mmol) est chauffé dans l'anhydride acétique (15 mL) à 100°C pendant 12 heures. Après refroidissement, le solvant est évaporé et le brut réactionnel est placé au reflux du méthanol (20 mL) pendant 16 heures. Après évaporation du solvant, une recristallisation dans 10 ml de méthanol conduit au monoester 111 avec un rendement de 65% qui est utilisé sans autre purification dans l'étape suivante.

### Acide 4-(méthoxycarbonyl)nicotinique (111).

Le composé 111 est obtenu sous forme de solide marron avec 67% de rendement après recristalisation dans le méthanol. IR (ATR-Ge ν cm⁻¹) : RMN ¹H (DMSO-*d6* ; 400 MHz) δ 13.79 (s, 1H), 9.03 (s, 1H), 8.86 (d, *J* = 4.9 Hz, 1H), 7.62 (d, *J* = 4.9 Hz, 1H), 3.84 (s, 3H) ; RMN ¹³C (DMSO-*d6* ; 101 MHz) δ 166.9 (Cq), 166.1 (Cq), 153.1 (CH), 150.2 (CH), 140.4 (Cq), 125.2 (Cq), 121.6 (CH), 52.9 (CH₃). SM (IS) m/z : 182.5 [M+H]⁺

### Synthèse de l'isocyanate (112)

Sous une atmosphère d'argon, on a ajouté de la triéthylamine (0.24 ml, 1.3 eq.) à une solution agitée de 111 (1.35 mmol, 1.0 eq.) dans du THF anhydre (10 mL). La solution a été refroidie à -10°C et on a ajouté goutte à goutte du chloroformiate de méthyle (0.2 ml, 1.5 eq.). Le mélange resultant a été agité pendant 1 h à -10°C, puis on a ajouté doucement de l'azoture de sodium (149 mg, 1.7 eq.) dans 2 mL d'eau. Après 1 h à -10°C, la reaction était finie et le mélange réactionnel a été extrait avec de l'acétate d'éthyle (3 x 10 mL). Les couches organiques combinées ont été séchées sur MgSO₄, filtrées et évaporées. L'azoture d'acyle obtenu a été redissous dans du toluène sec (10 mL) et chauffé à reflux pendant 1 h sous atmosphère d'Ar, pour donner l'isocyanate correspondant 112 qui a été utilisé sans purification supplémentaire.

### Procédure générale G: Synthèse des pyridopyrimidinediones 113-117.

Sous atmosphère d'argon, on a ajouté une solution de l'isocyanate **112** (1.0 mmol, 1.0 eq.) à une solution d'amine isoindolonique (1 mmol, **5** or **10)** dans 5 mL de pyridine. Le mélange a été mis à reflux pendant 24 h, refroidi et concentré sous vide. Le résidu brut a été purifié par chromatographie flash pour obtenir les pyridopyrimidinediones.

### 3-(6'-oxo-3',4',6',10b'-tétrahydro-1'H-spiro[[1,3]dioxolane-2,2'-pyrido[2,1-a]isoindole]-10'-yl)pyrido[3,4-d]pyrimidine-2,4(1H,3H)-dione (113).

Le composé **113** est obtenu selon la procédure G à partir de l'amine **5** et **111** sous forme de solide blanc avec 67% de rendement après chromatographie sous gel de silice flash (CH₂Cl₂ / MeOH : 99 /1). F : 204 °C ; IR (ATR-Ge ν cm⁻¹) : ; RMN ¹H (DMSO-*d6* ; 400 MHz) δ 12.06 (s, 1H), 8.70 (s, 1H), 8.49 (d, *J* = 4.7 Hz, 1H), 7.86 (dd, *J*= 2.7 Hz, 4.8 Hz, 1H), 7.81 (d, *J* = 7.1 Hz, 1H), 7.68 - 7.61 (m, 2H), 4.46 (m, 1H), 4.24 (dd, *J* = 5.1 Hz, 13.1 Hz, 1H), 3.88 - 3.68 (m, 4H), 3.15 - 3.00 (m, 1H), 1.85 - 1.74 (m, 2H), 1.50 - 1.45 (m, 1H), 1.38 - 1.20 (m, 1H) ; RMN ¹³C (DMSO-*d6* ; 101 MHz) δ 164.1 (Cq), 161.6 (Cq), 161.0 (Cq), 149.7 (Cq), 149.1 (Cq), 143.3 (CH), 142.3 (Cq), 138.8 (CH), 133.4 (Cq), 132.6 (CH), 130.5 (Cq), 129.5 (CH), 123.6 (CH), 119.6 (CH), 106.4 (Cq), 64.0 (CH₂), 63.8 (CH₂), 55.5 (CH), 38.2 (CH₂), 36.0 (CH₂), 32.7 (CH₂). SM (IS) m/z : 407.5 [M+H]⁺.

### 3-(6-oxo-1,2,3,4,6,10b-hexahydropyrido[2,1-a]isoindol-10-yl)pyrido[3,4-d]pyrimidine-2,4(1H,3H)-dione (114).

Le composé **114** est obtenu selon la procédure G à partir de l'amine 10 et de l'isocyanate **112** sous forme de solide gris avec 52 % de rendement après chromatographie sous gel de silice flash (CH₂Cl₂ / MeOH : 99 /1). F > 260°C ; IR (ATR-Ge ν cm⁻¹) : 1656 (C=O amide), 1590-1416 (C=C arom), 1260 (C-N), 1153 (C-C) ; RMN ¹H (DMSO-*d6* ; 400 MHz) δ 11.97 (d, *J* = 9.9 Hz, 1H), 8.69 (d, *J* = 3.6 Hz, 1H), 8.47 (d, *J* = 5.0 Hz, 1H), 7.84 (dd, *J* = 2.9 Hz, 5.0 Hz, 1H), 7.79 (d, *J* = 7.0 Hz, 1H), 7.69 - 7.54 (m, 2H), 4.35 - 4.27 (m, 1H), 4.21 (d, *J* = 9.9 Hz, 1H), 2.98 - 2.91 (m, 1H), 2.02 - 1.88 (m, 1H), 1.68 (d, *J* = 12.6 Hz, 2H), 1.50 (dd, *J* = 13.1 Hz, 26.3 Hz, 1H), 1.28 - 1.11 (m, 1H), 0.99 - 0.79 (m, 1H) ; RMN ¹³C (DMSO-*d6* ; 101 MHz) δ 163.9 (Cq), 161.7 (Cq), 161.0 (Cq), 149.8 (Cq), 149.2 (Cq), 143.0 (CH), 138.7 (CH), 135.2 (Cq), 133.5 (Cq), 132.3 (CH), 130.6 (Cq), 129.2 (CH), 123.3 (CH), 119.6 (CH), 57.2 (CH), 39.0 (CH₂), 30.1 (CH₂), 24.8 (CH₂), 22.7 (CH₂). SM (IS) m/z : 349.0 [M+H]⁺, 697.5 [2M+H]⁺.

### 3-(6-Oxo-1,2,3,4,6,10b-hexahydro-pyrido-pyrido[2,1-a]isoindol-10-yl)-1H-pyrido[3,2-d]pyrimidine-2,4-dione (115).

Le composé **115** a été obtenu selon la procédure **G** à partir de l'isocyanate correspondant et l'amine **10** après purification par chromatographie flash (CH₂Cl₂ /MeOH : 99 /1) sous la forme d'un solide beige. Rendement : 66 %. p.f. 232°C ; IR (ATR-Ge ν cm-1): 2929 (Csp3-H), 1669 (C=O amide), 1597-1457 (C=C arom), 1286 (C-N) ; ¹H RMN (DMSO-d6 ; 400 MHz) δ 11.80 (d, J = 9.8 Hz, 1H), 8.55 (d, J = 2.5 Hz, 1H), 7.79 (d, J = 7.2 Hz, 1H), 7.75 - 7.68 (m, 2H), 7.67 - 7.54 (m, 2H), 4.46 - 4.27 (m, 1H), 4.21 (d, J = 10.5 Hz, 1H), 2.95 (t, J = 12.9 Hz, 1H), 2.09 - 1.83 (m, 1H), 1.69 - 1.66 (m, 2H), 1.50 (dd, J = 24.2 Hz, 11.8 Hz, 1H), 1.32 - 1.10 (m, 1H), 0.98 - 0.85 (m, 1H); ¹³C RMN (DMSO-d6 ; 101 MHz) δ 164.0 (Cq), 160.3 (Cq), 149.5 (Cq), 148.9 (Cq), 145.1 (CH), 143.3 (Cq), 137.5 (Cq), 133.5 (Cq), 132.4 (CH), 131.0 (Cq), 129.2 (2CH), 124.0 (CH), 123.2 (CH), 57.2 (CH), 39.0 (CH₂), 30.1 (CH₂), 24.9 (CH₂), 22.8 (CH₂) ; HRMS : calc. pour C₁₉H₁₆N₄O₃Na 371,1120; trouvé 371,1110.

### Ester méthylique de l'acide 2-[3-(6-Oxo-1,2,3,4,6,10b-hexahydro-pyrido[2,1-a]isoindol-10-yl)-uréido]-nicotinique (116).

On a ajouté à 0 °C sous Ar du bis(trichlorométhyl) carbonate (297 mg; 1.0 eq.) à une solution agitée de 10 (198 mg; 1 mmol) et de Et₃N (0.28 mL; 2. eq.) dans du CH₂Cl₂ sec (10 mL), et le mélange réactionnel a ensuite été amené graduellement à reflux. Après 5 h à reflux, le mélange réactionnel a été refroidi. Une solution d'ester méthylique d'acide 2-amino-nicotinique (152 mg ; 1.0 eq.) dans de la pyridine sèche (1,6 mL) a été ajoutée à une solution refroidie de l'isocyanate brut dans CH₂Cl₂. Le mélange réactionnel a été agité à température ambiante pendant 18 h et ensuite dilué avec de l'eau et extrait avec AcOEt. La phase organique a été lavée avec de l'eau, séchée sur MgSO₄ et évaporée sous vide. Le résidu a été purifié par chromatographie sur gel de silice (méthanol / dichlorométhane : 1/99). Le composé **116** a été obtenu sous la forme d'un solide beige. Rendement : 40%. p.f. 205°C ; IR (ATR-Ge v cm⁻¹) : 3269 (N-H amide), 2921 (Cₛₚ₃-H), 1727 (C=O ester), 1680 (C=O amide), 1589-1484 (C=C arom), 1418 (C-O), 1253 (C-N) ; ¹H RMN (CDCl₃ ; 400 MHz) δ 11.85 (s, 1H), 10.41 (s, 1H), 8.50 - 8.28 (m, 2H), 8.17 (d, *J* = 8.0 Hz, 1H), 7.60 (d, *J* = 7.4 Hz, 1H), 7.43 (t, *J* = 7.8 Hz, 1H), 7.04 (dd, *J* = 7.8 Hz, 5.0 Hz, 1H), 4.54 - 4.44 (m, 2H), 3.96 (s, 3H), 3.00 (td, *J* = 13.0 Hz, 3.3 Hz, 1H), 2.71 (dd, *J* = 12.8 Hz, 2.5 Hz, 1H), 2.01 (d, *J* = 13.2 Hz, 1H), 1.93 - 1.77 (m, 1H), 1.76 - 1.58 (m, 1H), 1.52 - 1.33 (m, 1H), 1.07 (qd, *J* = 13.0 Hz, 3.2 Hz, 1H) ; ¹³C RMN (CDCl₃ ; 101 MHz) δ 166.3 (Cq), 166.1 (Cq), 153.5 (Cq), 151.8 (Cq), 150.4 (CH), 141.7 (CH), 135.7 (Cq), 133.5 (Cq), 133.3 (Cq), 129.3 (CH), 124.0 (CH), 119.4 (CH), 116.8 (CH), 110.0 (Cq), 58.6 (CH), 53.1 (CH₃), 39.9 (CH₂), 30.4 (CH₂), 25.5 (CH₂), 24.1 (CH₂) ; HRMS : calc. pour C₂₀H₂₁N₄O₄ 381,1563 ; trouvé 381,1550.

### 3-(6-Oxo-1,2,3,4,6,10b-hexahydro-pyrido[2,1-a]isoindol-10-yl)-1H-pyrido [2,3-d]pyrimidine-2,4-dione (117).

Le composé **116** (60 mg; 380,41 g/mol; 0,16 mmol) a été cyclisé par irradiation ondes µ dans une solution toluène/pyridine (1/1 ; 2 mU2 mL) à 120°C pendant 2h. Le résidu a été purifié par chromatographie sur gel de silice (Méthanol /dichlorométhane : 1/99). Le composé 117 a été obtenu sous la forme d'un solide beige. Rendement : 57%. p.f. > 290°C ; IR (ATR-Ge v cm⁻¹) : 2928 (Cₛₚ₃-H), 1678 (C=O amide), 1397 (C-N) ; ¹H RMN (DMSO-d6 ; 400 MHz) δ 12.19 (s, 1H), 8.71 (dd, *J* = 4.8 Hz, 1.8 Hz, 1H), 8.36 - 8.33 (m, 1H), 7.83 - 7.74 (m, 1H), 7.68 - 7.54 (m, 2H), 7.35 - 7.32 (m, 1H), 4.36 - 4.27 (m, 1H), 4.21 (dd, *J* = 12.8 Hz, 3.2 Hz, 1H), 3.01 - 2.88 (m, 1H), 1.95 (dd, *J* = 35.9 Hz, 9.8 Hz, 1H), 1.75 - 1.66 (m, 2H), 1.56 - 1.46 (m, 1H), 1.34 - 1.10 (m, 1H), 0.97 - 0.82 (m, 1H) ; ¹³C RMN (DMSO-d6 ; 101 MHz) δ 164.0 (Cq), 162.0 (Cq), 155.1 (CH), 151.5 (Cq), 150.2 (Cq), 143.5 (Cq), 137.2 (CH), 133.6 (Cq), 132.5 (CH), 130.8 (Cq), 129.2 (CH), 123.3 (CH), 119.3 (CH), 109.8 (Cq), 57.2 (CH), 39.0 (CH₂), 30.3 (CH₂), 24.9 (CH₂), 22.7 (CH₂) ; HRMS : calc. pour C₁₉H₁₇N₄O₃ : 349,1301 ; trouvé : 349,1290.

### Ester butylique de l'acide 3-[3-(6-Oxo-1,2,3,4,6,10b-hexahydro-pyrido[2,1-a]isoindol-10-yl)-uréido]-pyrazole-1-carboxylique (118).

On a ajouté à 0°C sous Ar du bis(trichlorométhyl) carbonate (207 mg; 1.0 eq.) à une solution agitée de **10** (140 mg; 0.7 mmol) et de Et₃N (0.2 mL; 2.0 eq.) dans du CH₂Cl₂ sec (10 mL), et le mélange réactionnel a ensuite été amené graduellement à reflux. Après 5 h à reflux, le mélange réactionnel a été refroidi. Une solution d'ester méthylique d'ester tertiobutylique d'acide 3-amino-pyrazole-1-carboxylique (128 mg ; 1.0 eq.) dans de la pyridine sèche (1,2 mL) a été ajoutée à une solution refroidie de l'isocyanate brut dans CH₂Cl₂. Le mélange réactionnel a été agité à température ambiante pendant 18 h et ensuite dilué avec de l'eau et extrait avec AcOEt. La phase organique a été lavée avec de l'eau, séchée sur MgSO₄ et évaporée sous vide. Le résidu a été purifié par chromatographie sur gel de silice (méthanol / dichlorométhane : 1/99). Le composé **118** a été obtenu sous la forme d'un solide jaune. Rendement : 56%. p.f. 136 C ; IR (ATR-Ge v cm⁻¹) : 3269 (N-H amide), 2937 (Cₛₚ₃-H), 1668 (C=O amide), 1575-1484 (C=C arom), 1320 (C-O), 1287 (C-N), 1141 (C-C) ; ¹H RMN (CDCl₃ ; 400 MHz) δ 10.32 (s, 1H), 9.59 (s, 1H), 7.99 (s, 1H), 7.91 (d, *J* = 2.9 Hz, 1H), 7.63 (d, *J* = 7.4 Hz, 1H), 7.42 (t, *J* = 7.8 Hz, 1H), 6.38 (s, 1H), 4.64 (d, *J* = 9.8 Hz, 1H), 4.49 (dd, *J* = 13.0 Hz, 3.8 Hz, 1H), 2.99 (t, *J* = 11.8 Hz, 1H), 2.65 (s, 1H), 2.18 (s, 1H), 1.85 (d, *J* = 13.0 Hz, 1H), 1.76 (d, *J* = 11.8 Hz, 1H), 1.62 (s, 9H), 1.45 - 1.27 (m, 1H), 0.98 (qd, *J* = 12.7 Hz, 3.2 Hz, 1H) ; ¹³C RMN (CDCl₃ ; 101 MHz) δ 166.2 (2Cq), 153.1 (Cq), 152.3 (Cq), 133.7 (Cq), 133.2 (2Cq), 131.9 (2CH), 129.0 (2CH), 119.8 (CH), 85.7 (Cq), 58.6 (CH), 40.1 (CH₂), 30.3 (CH₂), 28.2 (3CH₃), 25.6 (CH₂), 23.5 (CH₂) ; HRMS : calc. pour C₂₁H₂₅N₅O₄Na 434,1804 ; trouvé 434,1791.

### 1-(6-Oxo-1,2,3,4,6,10b-hexahydro-pyrido[2,1-a]isoindol-10-yl)-3-(1H-pyrazol-3-yl)-urée (119).

Le mélange de **118** (60 mg ; 0.146 mmol) et de NaOH (1 M; 20 mL) a été agité à température ambiante pendant la nuit. Le mélange réactionnel a été extrait avec de l'acétate d'éthyle chaud (3 x 20 mL). Les extraits organiques combinés ont été séchés sur MgSO₄. L'élimination des solvants a fourni le résidu qui a été purifié par chromatographie flash. Le composé **119** a été obtenu sous la forme d'un solide blanc. Rendement : 66%. p.f. 185 C ; IR (ATR-Ge v cm⁻¹) : 3342 (N-H amine), 2919 (Cₛₚ₃-H), 1707 (C=O amide), 1600-1547 (C=C arom), 1266 (C-N) ; ¹H RMN (DMSO-d₆; 400 MHz) δ 12.33 (s, 1H), 9.40 (s, 1H), 9.06 (s, 1H), 8.12 (d, *J* = 7.9 Hz, 1H), 7.63 (s, 1H), 7.42 (t, *J* = 7.7 Hz, 1H), 7.35 (d, *J* = 7.2 Hz, 1H), 6.20 (s, 1H), 4.48 (dd, *J* = 11.5 Hz, 3.2 Hz, 1H), 4.26 (dd, *J* = 13.1 Hz, 4.3 Hz, 1H), 3.01 (td, *J* = 12.7 Hz, 3.0 Hz, 1H), 2.66 (d, *J* = 10.7 Hz, 1H), 1.90 (d, *J* = 13.2 Hz, 1H), 1.83 - 1.56 (m, 2H), 1.34 - 1.16 (m, 1H), 0.84 (qd, *J* = 12.8 Hz, 3.2 Hz, 1H) ; ¹³C RMN (DMSO-d₆ ; 101 MHz) δ 164.6 (Cq), 151.8 (Cq), 147.9 (Cq), 134.7 (Cq), 134.4 (Cq), 132.8 (Cq), 129.2 (CH), 128.7 (CH), 122.2 (CH), 117.0 (CH), 94.1 (CH), 57.4 (CH), 39.1 (CH₂), 29.9 (CH₂), 25.0 (CH₂), 23.0 (CH₂) ; SM (IS) m/z : 312,0 [M+H]⁺; 623,5 [2M+H]⁺

### RÉSULTATS BIOLOGIQUES

### 1. Méthodes de dosages

Les activités d'inhibition des protéines kinases des composés de l'invention sont testées selon le protocole général suivant :

### Solutions tampons

*Tampon A :* 10 mM MgCl₂, 1 mM EGTA, 1 mM DTT, 25 mM Tris-HCl pH 7.5 et 50 µg héparine/ml.

*Tampon C :* 60 mM β-gtycérophosphate, 15 mM p-nitrophényl-phosphate, 25 mM Mops (pH 7.2), 5 mM EGTA, 15 mM MgCl₂, 1 mM DTT, 1 mM vanadate de sodium et 1 mM phénylphosphate.

### Préparations des kinases et dosages

Les activités kinases sont dosées dans les tampons A ou C, à 30 °C, à une concentration finale d'ATP de 15 µM. Les valeurs des blancs ont été soustraites et les activités sont exprimées en % de l'activité maximale, c'est-à-dire en l'absence d'inhibiteurs. Les contrôles ont été effectués avec des dilutions appropriées de DMSO.

Les *CDK1*/*cycline B* (ovocytes d'étoile de mer en phase M, native) et *CDK5*/*p25* (humain, recombinant) ont été préparées comme décrit précédemment (Leclerc, S.; Garnier, M.; Hoessel, R.; Marko, D.; Bibb, J.A.; Snyder, G.L.; Greengard, P.; Biernat, J.; Mandelkow, E.-M.; Eisenbrand, G.; Meijer, L. Indirubins inhibit glycogen synthase kinases-3β and CDK5/p25, two kinases involved in abnormal tau phosphorylation in Alzheimer's disease - A property common to most CDK inhibitors? J. Biol. Chem. 2001, 276, 251-260; Bach S, Knockaert M, Reinhardt J, Lozach O, Schmitt S, Baratte B et al. (2005). Roscovitine targets, protein kinases and pyridoxal kinase. J Biol Chem 280: 31208-31219). Leur activité kinase a été analysée dans le tampon C, avec 1 mg d'histone H1/ml, en présence de 15 µM de [γ-³³P] ATP (3,000 Ci/mmol; 10 mCi/ml) dans un volume final de 30 µl. Après 30 min d'incubation à 30°C, 25 µl d'aliquots de surnageant ont été déposés sur des morceaux de 2,5 x 3 cm de papier de phosphocellulose Whatman P81, et, 20 secondes après, les filtres ont été lavés cinq fois (pendant au moins 5 minutes à chaque fois) dans une solution de 10 ml d'acide phosphorique / litre d'eau. Les filtres mouillés ont été comptés en présence d'1 ml de fluide de scintillation ACS (Amersham).

La *GSK-3αβ* (cerveau de porc, native) a été dosée, comme décrit pour CDK1 mais dans le tampon A et en utilisant un substrat spécifique de GSK-3 (GS-1 : YRRAAVPPSPSLSRHSSPHQSpEDEEE)(Sp représente une sérine phosphorylée)(Primot, A., Baratte, B., Gompel, M., Borgne, A., Liabeuf, S., Romette, J.L., Costantini, F. and Meijer, L., 2000. Purification of GSK-3 by affinity chromatography on immobilised axin. Protein Expr. & Purif. 20 (3), 394-404). GS-1 a été synthétisé par Millegen (Labège, France).

La DYRK1A (humaine, recombinante, exprimée chez E. coli comme protéine de fusion GST) a été purifiée par chormatographie d'affinité sur billes de glutathion-agarose et mesurée dans le tampon A (+ 0,5 mg sérum albumine bovine/mL) avec le substrat Woodtide (1,5µg/dosage).

Dosages *in vivo* sur les cellules humaines (Huh7, Caco, MDA-MB 231, HCT 116, PC3, NCI, fibroblaste)

Cytotoxicité : Cette méthode est basée sur une analyse d'imagerie automatisée. 4.10³ cellules ont été mises en culture sur des plaques à 96 puits et laissées ainsi pendant 24h pour qu'elles se lient, se répandent et se prolifèrent.

Ces cellules ont ensuite été exposées pendant 24h et 48h à des concentrations croissantes des composés de l'invention, de 0,1 à 25 µM dans un volume final de 80 µL de milieu de culture. Les cellules ont ensuite été fixées avec une solution de paraformaldéhyde 4% et les noyaux ont été teintés avec Hoechst 3342 et comptées selon une quantification d'imagrie automatisée.

Toutes les lignées cellulaires ont été cultivées dans un milieu DMEM ou RPMI (Invitrogen). Tous ces milieux ont été complétés avec des antibiotiques (pénicilline-streptomycine)(Lonza) et 10% en volume de sérum de veau foetal (Invitrogen). Les cellules ont été cultivées à 37°C avec 5% CO₂. Les traitements avec les molécules à tester ont été effectués avec des concentrations croissantes. Les expériences témoins ont également été effectuées en utilisant des dilutions appropriées de DMSO (maximum 1% DMSO). La viabilité cellulaire a été déterminée en mesurant la réduction de MTS comme décrit dans l'article Ribas J, Boix J. (2004). Cell differentiation caspase inhibition and macromolecular synthesis blockage but not BCL-2 or BCL-XL proteins protect SH-SY5Y cells from apoptosis triggered by two CDK inhibitory drugs. Exp. Cell Res., 295: 9-24.

### 2. Résultats

Les résultats obtenus sont indiqués dans les tableaux ci-après.

| Numéro | Espèce | IC₅₀ µM | | | | | | | | | | |
|---|---|---|---|---|---|---|---|---|---|---|---|---|
| | | Cellules humaines | | | | | | | Kinases | | | |
| | | Huh7 (foie) | Caco (colon) | MDA-MB 231 (sein) | HCT 116 (colon) | PC3 (prostate) | NCI (poumon) | fibroblaste | CDK1 | DYRK1A | CDK5 | GSK3 |
| 16 | 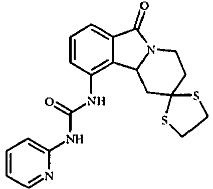 | 15 | 15 | 20 | 10 | 6 | 25 | >25 | 0.22 | >10 | 0.24 | 0.033 |
| 17 | 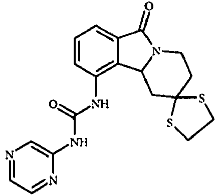 | 0.1 ( | 0.5 | 0.15 | 0.1 | 0.6 | 0.4 | 0.8 | 0.13 | >10 | 0.15 | 0.044 |
| 18 | 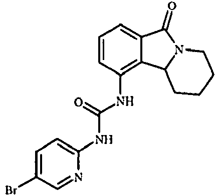 | >25 | >25 | >25 | >25 | >25 | >25 | >25 | ND | 21 | 0.13 | 0.076 |
| 19 | 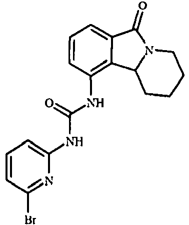 | >25 | >25 | >25 | >25 | >25 | >25 | >25 | ND | 3,4 | 0.040 | 0.043 |
| 20 | 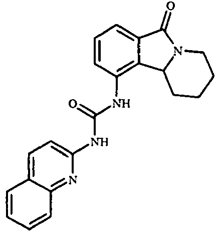 | 0.01 | <0.01 | <0.03 | 0.01 | 0.01 | 0.08 | 0,3 | ND | 9 | 0,4 | 0,022 |
| 21 | 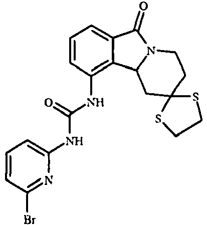 | 2 | 3 | 4 | 2,5 | 4 | 6 | 9 | ND | 8,7 | >10 | 0,71 |
| 22 | 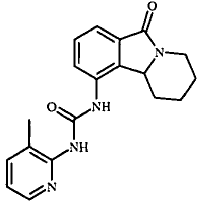 | 0.04 | 0.07 | 0,15 | 0.01 | 0.1 | 0,15 | 0,4 | ND | 4 | 0,096 | 0,042 |
| 23 | 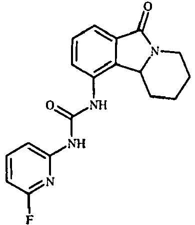 | 0.1 | 0.03 | 0.03 | <0.0 1 | 0.03 | 0,3 | 0,3 | ND | 3,5 | 0,9 | 0,026 |
| 24 | 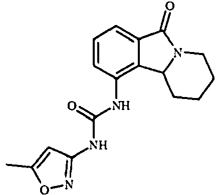 | 3 | 3 | 3 | 2 | 6 | 10 | 12 | ND | >10 | 2,7 | 0,033 |
| 25 | 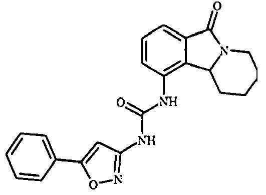 | 0,25 | 0.1 | 0,2 | 0.03 | 0,1 | 1,5 | 0.1 | >10 | >10 | 1.5 | 0.27 |
| 26 | 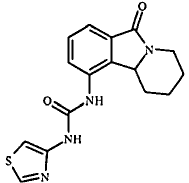 | 1,2 | 0,8 | 0,4 | 0,8 | 2 | 5 | 4 | ND | >10 | 0,43 | 0,76 |
| 27 | 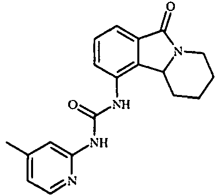 | 0.01 | 0.01 | <0.1 (0.01) | 0.01 | 0.01 | 0.03 | 0,1 | ND | >10 | 0,04 | 0,02 |
| 28 | 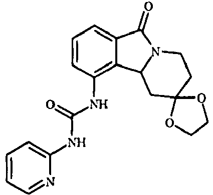 | 1 | 0,6 | 1 | 0,6 | 1,5 | 4 | 3 | ND | 80 | 11 | 0,7 |
| 29 | 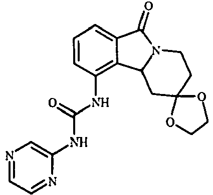 | 3 | 5 | 3 | 1,5 | 5 | 8 | 10 | ND | 93 | 7,1 | 0,26 |
| 30 | 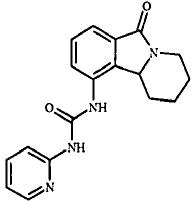 | 0.03 | 0.1 | 0.05 | 0.08 | 0.1 | 0.2 | 0.2 | 0.075 | 27 | 0.08 | 0.17 |
| 31 | 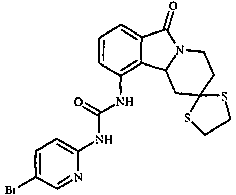 | 0,8 | 5 | 4 | 1,2 | 3 | 1,5 | 8 | ND | ≥10 | 7,3 | 0,068 |
| 32 | 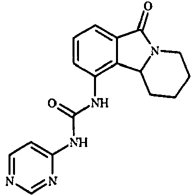 | | | | | | | | ND | 71 | 1,5 | 0,36 |
| 33 | 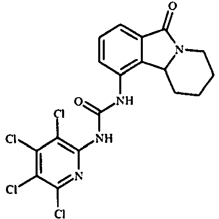 | 0,25 | 1,5 | 0,5 | 0,5 | 1 | 0,8 | 0,5 | ND | > 100 | 10 | 4,3 |
| 34 | 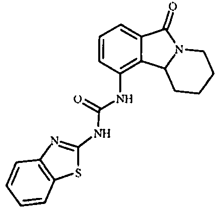 | 0,6 | 0,7 | 1,5 | 0,5 | 1 | 4 | 2 | ND | 88 | 16 | 3,5 |
| 35 | 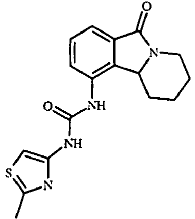 | 0,9 | 0,7 | 1,5 | 0,6 | 1,5 | 3 | 0,4 | ND | >10 | 0,53 | 0,12 |
| 36 | 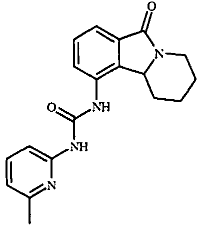 | 0.04 | 0.01 | <0.1 | 0.04 | 0.04 | 0,15 | 0.04 | ND | 2.2 | 0,035 | 0,0066 |
| 37 | 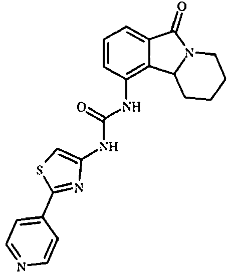 | 2 | 1,5 | 1,5 | 0,7 | 1 | 4 | 3 | ND | 2,4 | 1,7 | 0,13 |
| 38 | 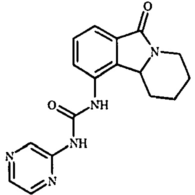 | 0,2 | 1 | 0,2 | 0,1 | 0,3 | 0,8 | 0,8 | 0.07 | > 100 | 0.07 | 0.51 |
| 39 | 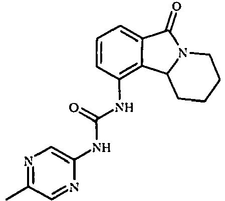 | 0,15 | 0,4 | 0,15 | 0,15 | 0,25 | 0,5 | 0,3 | ND | >10 | 0,023 | 0,06 |
| 40 | 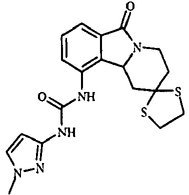 | | | | | | | | ND | >10 | ≥10 | 1,9 |
| 41 | 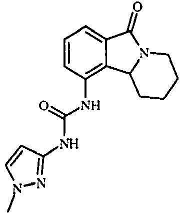 | | | | | | | | ND | >10 | 0,51 | 0,31 |
| 42 | 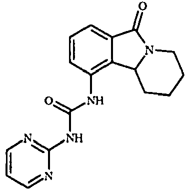 | 0.6 (60%) | 2 | 4 | 4 | 2 | 8 | 2.5 (50%) | ND | >10 | >10 | 0.75 |
| 43 | 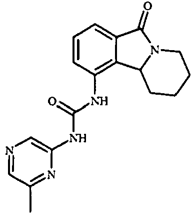 | 0,4 | 0,8 | 0,5 | 0,25 | 0,4 | 0,5 | 0,8 | ND | >10 | 0.81 | 0.072 |
| 44 | 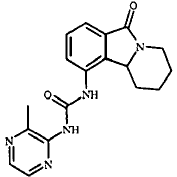 | 0,6 | 0,6 | 1,5 | 0,5 | 0,9 | 1 | 1,5 | ND | 4.5 | 0.16 | 0.17 |
| 45 | 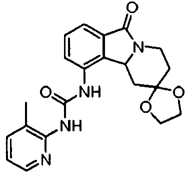 | >25 | >25 | >25 | 25 | >25 | >25 | >25 | ND | >10 | 3.1 | 0.61 |
| 46 | 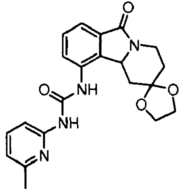 | 12 | 10 | 15 | 10 | 12 | 12 | 20 | ND | >10 | >10 | 0.92 |
| 47 | 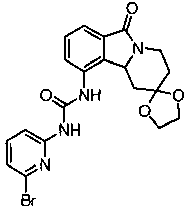 | 8 | 12 | 20 | 6 | 6 | 20 | 20 | ND | >10 | >10 | 0.89 |
| 48 | 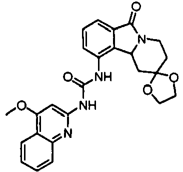 | 8 | >25 | 4 | 8 | 8 | 20 | 20 | ND | >10 | >10 | 1 |
| 49 | 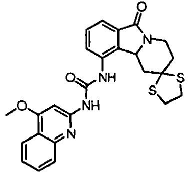 | 5 | 15 | 20 | 10 | 15 | 10 | 25 | ND | >10 | >10 | 4 |
| 50 | 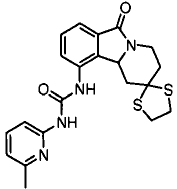 | 5 | 10 | 15 | 12 | 6 | 15 | 2 | ND | >10 | >10 | 2 |
| 51 | 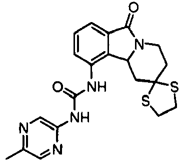 | 0,3 | 0,5 | 0,4 | 0,2 | 0,4 | 0,8 | 8 | ND | >10 | 0.22 | 0.05 |
| 52 | 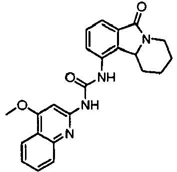 | 2 | 4 | 2 | 8 | 20 | 7 | 5 | ND | >10 | 0.15 | 0.08 |
| 53 | 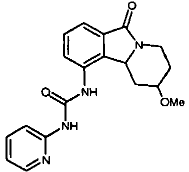 | 0,7 | 2,5 | 2 | 1 | 2 | 4 | 3 | ND | >10 | 0,55 | 0,081 |
| 54 | 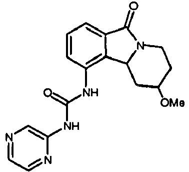 | 4 | 20 | 5 | 4 | 7 | 10 | 10 | ND | >10 | 0,48 | 0,2 |
| 55 | 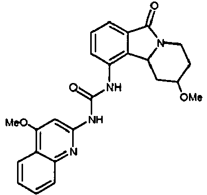 | 3 | 10 | 15 | 2,5 | 3 | 5 | 10 | ND | >10 | 8,5 | 0,18 |
| 56 | 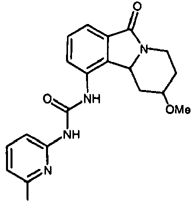 | 3 | 6 | 7 | 3 | 6 | 10 | 7 | ND | >10 | 4,3 | 0,32 |
| 57 | 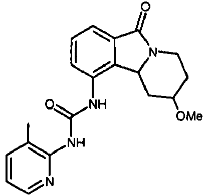 | 1,2 | 2 | 2,2 | 1 | 1,2 | 4 | 3 | ND | >10 | 1,1 | 0,25 |
| 58 | 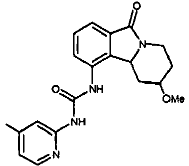 | 0,1 | 0,5 | 0,3 | 0.03 | 0,4 | 0,4 | 0,5 | ND | >10 | 0,32 | 0,032 |
| 59 | 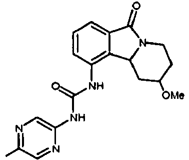 | 1,2 | 12 | 6 | 3 | 5 | 6 | 5 | ND | >10 | 0,32 | 0,14 |
| 60 | 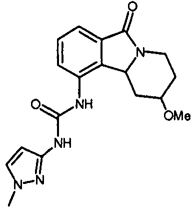 | 2 | 15 | 10 | 4 | 7 | 2 | 15 | ND | >10 | 2,3 | 1,1 |
| 62 | 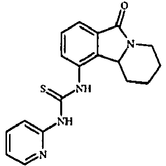 | >25 | 20 | 25 | 8 | 20 | >25 | >25 | ND | 47 | 10 | 2.3 |
| 63 | 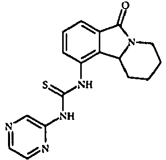 | >25 | >25 | >25 | >25 | >25 | >25 | >25 | ND | >100 | 26 | 8 |
| 64 | 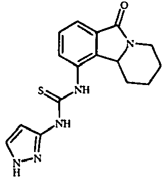 | 8 | 20 | 25 | 20 | 6 | 25 | >25 | ND | ≥ 100 | 56 | 16 |
| 65 | 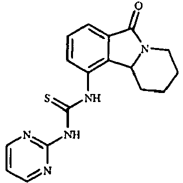 | >25 | 20 | 25 | >25 | >25 | >25 | >25 | ND | ≥ 100 | >10 | >10 |
| 66 | 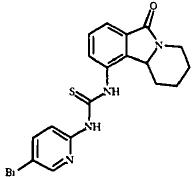 | >25 | 2 | 2 | 3 | >25 | 25 | 4 | ND | > 100 | 8.7 | 2.1 |
| 67 | 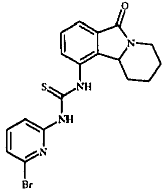 | 10 | 3 | 4 | 2 | 8 | 12 | 10 | ND | 92 | 8.1 | 4.5 |
| 68 | 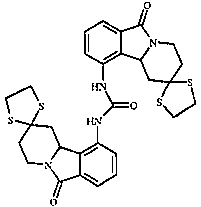 | 1 | >25 | >25 | 3 | 7 | 5 | >25 | ND | >10 | >10 | >10 |
| 69 | 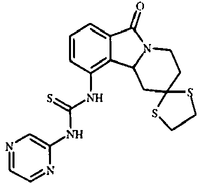 | >25 | 4 | 10 | 15 | >25 | >25 | 25 | ND | >10 | >10 | >10 |
| 70 | 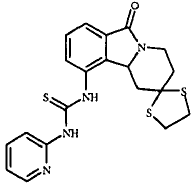 | 15 | 2 | 3 | 2 | >25 | 10 | 20 | ND | 22 | 19 | 2.5 |
| 71 | 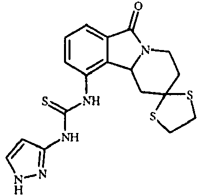 | 20 | 15 | 15 | 7 | >25 | 15 | 25 | ND | >10 | >10 | >10 |
| 74 | 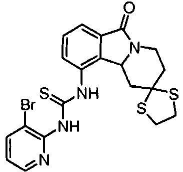 | 10 | 20 | >25 | 25 | 15 | 12 | 25 | ND | >10 | >10 | 3 |
| 75 | 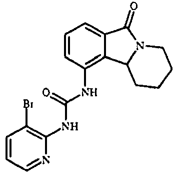 | 0,25 | 5 | 1,5 | 0,4 | 7 | 2 | 0,6 | ND | 3 | 0,48 | 0,17 |
| 76 | 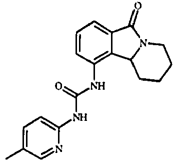 | 0.04 | <0.1 | <0.1 | <0.1 | <0.1 | <0.1 | <0.1 | ND | >10 | 0,044 | 0,038 |
| 96 | 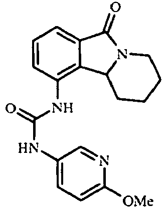 | 0,5 | 0,8 | 0,2 | 0,2 | 0,6 | 0,6 | 1,5 | 25 | 25 | 1.6 | 0.23 |
| 97 | 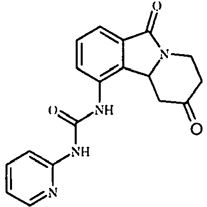 | 3 | 7 | 3 | 2 | 5 | 2 | 6 | ND | 50 | 3,6 | 1,2 |
| 98 | 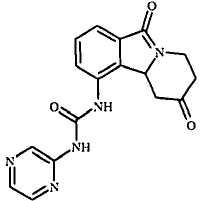 | >25 | 25 | >25 | 20 | >25 | >25 | >25 | ND | >10 | >10 | 4,2 |
| 103 | 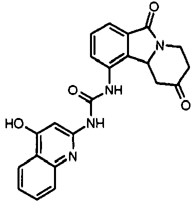 | | | | | | | | ND | ≥10 | >10 | 1,3 |
| 104 | 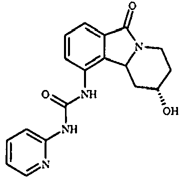 | 2 | 7 | 3 | 1 | 3 | 3 | 2 | ND | 34 | 2.1 | 0.26 |
| 105 | 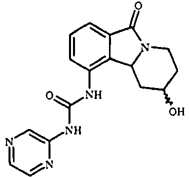 | 4 | >25 | 25 | 8 | 20 | 8 | 12 | ND | >10 | 0.084 | 0.032 |
| 107 | 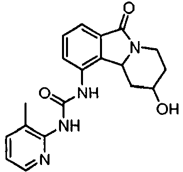 | 5 | 12 | 8 | 1,5 | 5 | 7 | 6 (50%) | ND | 6.2 | 0.63 | 0.16 |
| 108 | 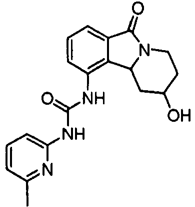 | 3 | 15 (brusque) | 8 | 3 | 2 | 10 | 10 | ND | 7 | 1 | 0.03 |
| 109 | 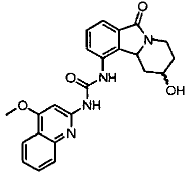 | 1 | 15 | 6 | 1 | 5 | 1 (50%) | 5 | ND | >10 | 4.9 | 0.035 |
| 110 | 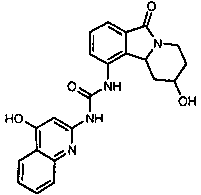 | | | | | | | | ND | ≥ 10 | >10 | 2,5 |
| 118 | 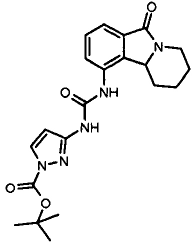 | | | | | | | | ND | >10 | 1,2/1,8 | 0,4 |
| 119 | 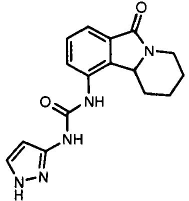 | | | | | | | | ND | >10 | 0,17/0,26 | 0,47 |

## Revendications

1. Composé de formule générale (I) suivante : 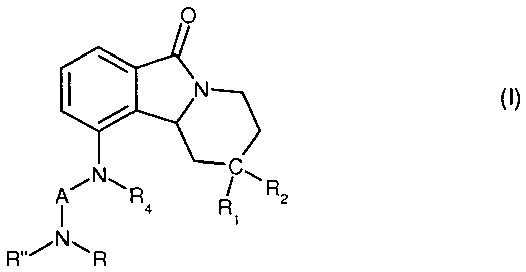 dans laquelle :
- A représente un groupe 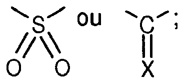 X représentant O ou S ;
- R" représente H ou un groupe alkyle comprenant de 1 à 10 atomes de carbone,
- R₁ représente H et R₂ représente un atome d'hydrogène, un groupe NR₃R'₃ ou un groupe OR₃, R₃ et R'₃ représentant, indépendamment l'un de l'autre, un atome d'hydrogène ou un groupe alkyle comprenant de 1 à 10 atomes de carbone ;
ou R₁ et R₂ forment ensemble, avec l'atome de carbone sur lequel ils sont fixés, un groupe C=O , 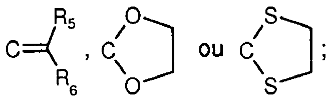
R₅ et R₆ représentant indépendamment l'un de l'autre un atome d'hydrogène ou un groupe alkyle comprenant de 1 à 10 atomes de carbone ;
- R₄ représente un atome d'hydrogène ou un groupe alkyle comprenant de 1 à 10 atomes de carbone ;
- R représente un groupe alkyle comprenant de 1 à 10 atomes de carbone, un groupe aryle comprenant de 6 à 30 atomes de carbone ou un groupe cycloalkyle comprenant de 3 à 20 atomes de carbone, lesdits groupes alkyle, aryle ou cycloalkyle susmentionnés comprenant éventuellement un ou plusieurs hétéroatomes, notamment O, S ou N, et pouvant le cas échéant être substitués,
R représentant notamment un groupe alkyle éventuellement substitué par un groupe amine,
ou lorsque A est un groupe -CO-, R et R₄ peuvent former ensemble, avec les atomes d'azote sur lesquels ils sont fixés un cycle de formule (II) suivante : 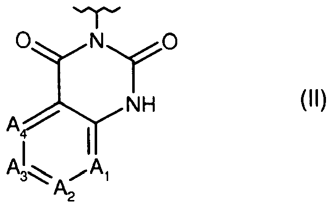 dans laquelle l'un des atomes parmi A₁, A₂, A₃ et A₄ représente N, et les trois autres atomes parmi A₁, A₂, A₃ et A₄ représentent CH,
ainsi que ses sels pharmaceutiquement acceptables,
ledit composé de formule (I) étant sous forme de stéréoisomère pur ou sous forme de mélange d'énantiomères et/ou de diastéréoisomères, y compris de mélanges racémiques.

2. Composé selon la revendication 1, **caractérisé en ce que** R représente un groupe aryle choisi dans le groupe constitué des groupes phényle, benzyle, pyridinyle, pyrimidyle, pyrazinyle, triazinyle, pyrazolyle, isoxazolyle, thiazolyle, benzothiazothiazolyle et quinolinyle, le cas échéant substitués, ou **en ce que** R représente un groupe cyclohexyle ou pipéridinyle, le cas échéant substitués.

3. Composé selon l'une quelconque des revendications 1 ou 2, **caractérisé en ce que** R est choisi parmi l'un des groupes suivants : 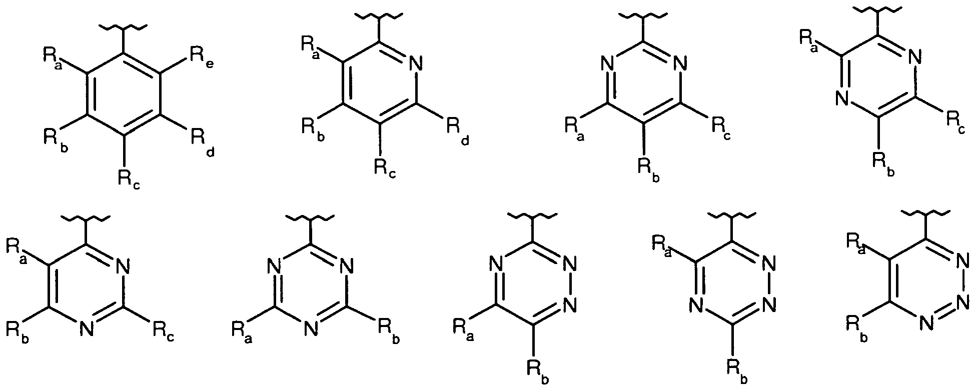 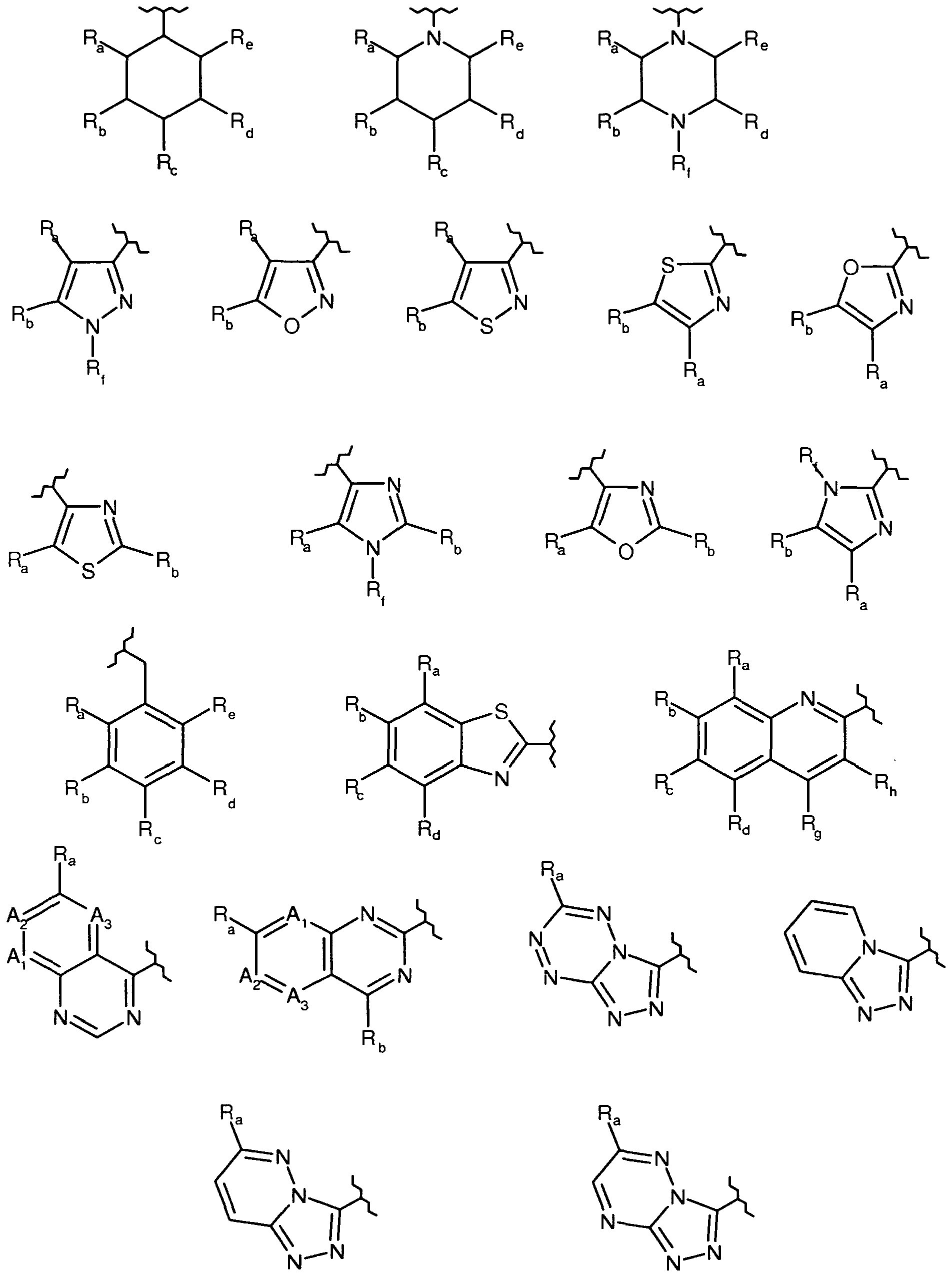 les groupes Rₐ, R_{b}, R_{c}, R_{d}, Rₑ, R_{g} et Rₕ étant choisis, indépendamment les uns des autres, dans le groupe constitué des substituants suivants :
- un atome d'hydrogène,
- un atome d'halogène, notamment Br, Cl ou F,
- un groupe alkyle comprenant de 1 à 10 atomes de carbone, et étant de préférence un groupe méthyle,
ledit groupe alkyle étant éventuellement substitué notamment par un ou plusieurs substituants choisis dans le groupe constitué des substituants suivants :
. atomes d'halogène,
. groupes alcényles ou alcynyles comprenant de 2 à 10 atomes de carbone,
. groupes aryles comprenant de 6 à 30 atomes de carbone,
. groupes COR_{α}, COOR_{α}, SR_{α}, OR_{α} ou NR_{α}R_{β}, R_{α} et R_{β} représentant indépendamment l'un de l'autre un atome d'hydrogène, un groupe alkyle comprenant de 1 à 10 atomes de carbone, ou un groupe aryle comprenant de 6 à 30 atomes de carbone,
- un groupe -CHO,
- un groupe -CN,
- un groupe phényle,
- un groupe -SR_{α} ou OR_{α}, R_{α} étant tel que défini ci-dessus, notamment un groupe -OH, -OCH₃, -SH, -SCH₃, -O-CH(CH₃)₂, -O-CH₂-CH₂-CH₃,
- un groupe -NR_{α}R_{β}, R_{α} et R_{β} étant tels que définis ci-dessus, notamment un groupe NH₂,
- un groupe -CONR_{α}R_{β}, R_{α} et R_{β} étant tels que définis ci-dessus, notamment - CONH₂,
- un groupe -NHCOR_{α}, R_{α} étant tel que défini ci-dessus, et
- un groupe 2-pyridinyle.
l'un des atomes parmi A₁, A₂ et A₃ représentant N, et les deux autres atomes parmi A₁, A₂ et A₃ représentant CH,
le groupe R_{f} étant un atome d'hydrogène ou un groupe alkyle comprenant de 1 à 10 atomes de carbone.

4. Composé selon l'une quelconque des revendications 1 à 3, répondant à la formule générale (I-1-a) suivante : 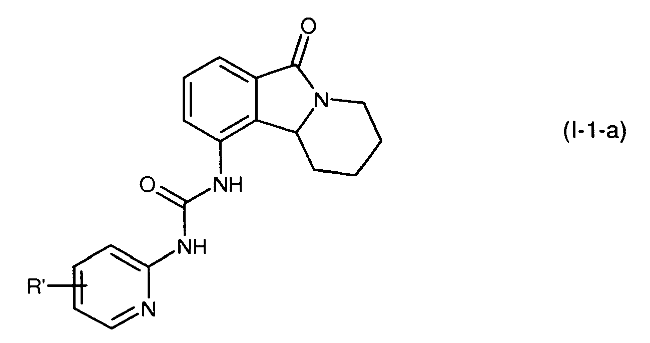 R' représentant un atome d'hydrogène, un atome d'halogène tel que Br ou F ou un groupe alkyle comprenant de 1 à 10 atomes de carbone, de préférence un groupe méthyle.

5. Composé selon l'une quelconque des revendications 1 à 4, pour son utilisation comme médicament.

6. Composé selon l'une quelconque des revendications 1 à 4, pour son utilisation en tant qu'inhibiteur des kinases CDK1, CDK5 et/ou GSK3.

7. Composé selon l'une quelconque des revendications 1 à 4, pour son utilisation dans le cadre du traitement ou de la prévention de maladies liées à une dérégulation des kinases CDK1, CDK5 et/ou GSK3.

8. Procédé de préparation d'un composé de formule générale (I) selon la revendication 1, dans laquelle R₄ et R" sont H et A est un groupe CO, et R₁ et R₂ sont H ou forment ensemble, avec l'atome de carbone sur lequel ils sont fixés, un groupe 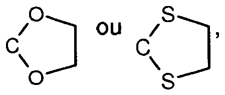 ledit procédé comprenant : une étape de réaction de l'amine de formule suivante : 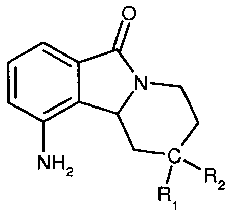 avec un isocyanate de formule RNCO, R étant tel que défini dans la revendication 1.

9. Procédé de préparation d'un composé de formule générale (I) selon la revendication 1, dans laquelle R₄ et R" sont H et A est un groupe CS, et R₁ et R₂ sont H ou forment ensemble, avec l'atome de carbone sur lequel ils sont fixés, un groupe 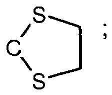 ledit procédé comprenant :
une étape de réaction de l'isothiocyanate de formule suivante : 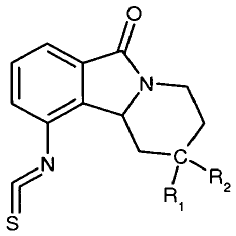
avec une amine de formule RNH₂, R étant tel que défini dans la revendication 1.

10. Procédé de préparation d'un composé de formule générale (I) selon la revendication 1, dans laquelle R₄ et R" sont H et A est un groupe CO, et R₁ et R₂ sont H ou forment ensemble, avec l'atome de carbone sur lequel ils sont fixés, un groupe 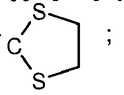 ledit procédé comprenant :
une étape de réaction de la thiourée de formule suivante : 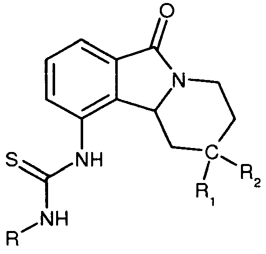 R étant tel que défini dans la revendication 1,
en présence d'un mélange CH₃CN/eau et de HgO à température ambiante pendant une durée comprise de 24 à 40 heures.

11. Procédé de préparation d'un composé de formule générale (I) selon la revendication 1, dans laquelle R₄ et R" sont H et A est un groupe CO, et R₁ et R₂ sont H ou forment ensemble, avec l'atome de carbone sur lequel ils sont fixés, un groupe 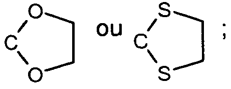 ledit procédé comprenant :
une étape de réaction de l'amine de formule suivante : 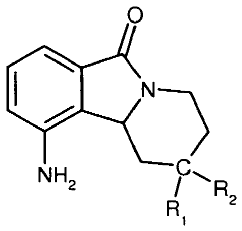
avec une amine de formule RNH₂ et du triphosgène, R étant tel que défini dans la revendication 1.

12. Procédé de préparation d'un composé de formule générale (I) selon la revendication 1, dans laquelle R₁ et R₂ forment ensemble, avec l'atome de carbone sur lequel ils sont fixés, un groupe C=O ;
ledit procédé comprenant :
une étape de réaction de l'acétal de formule suivante : 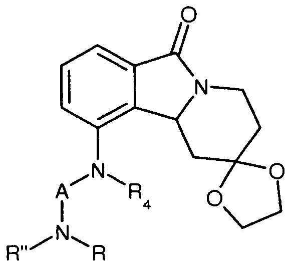
A, R, R" et R₄ étant tels que définis dans la revendication 1,
avec de l'acétone et de l'acide chlorhydrique.

13. Procédé de préparation d'un composé de formule générale (I) selon la revendication 1, dans laquelle R₁ est H et R₂ est OH,
ledit procédé comprenant :
une étape de réduction de la cétone de formule suivante : 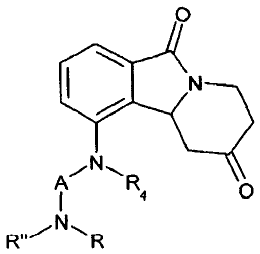
A, R, R" et R₄ étant tels que définis dans la revendication 1,
notamment avec NaBH₄ en présence de THF et de méthanol.

14. Composés intermédiaires répondant à l'une des formules suivantes :

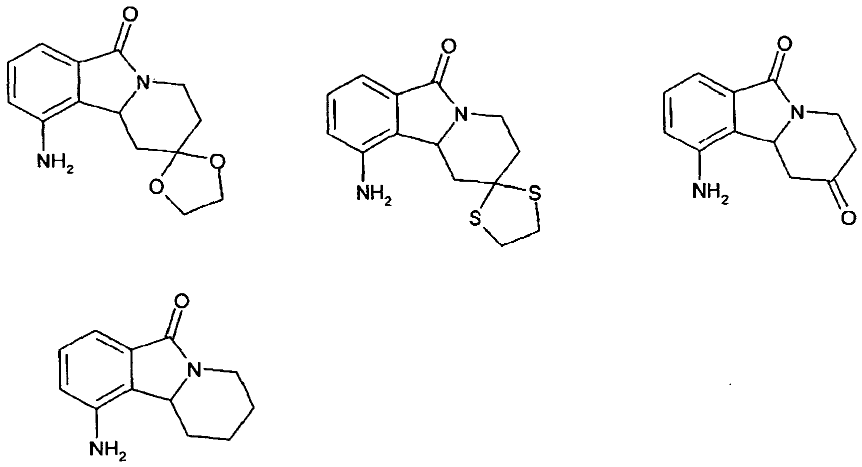

## Claims

1. A compound of the following general formula (I): 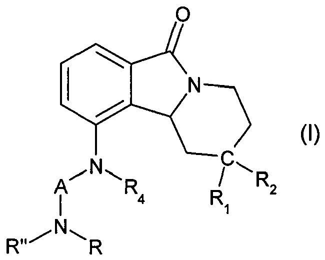 wherein:
- A represents a group 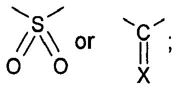 X representing O or S;
- R" represents H or an alkyl group comprising from 1 to 10 carbon atoms,
- R₁ represents H and R₂ represents an hydrogen atom, an NR₃R'₃ group or an OR₃ group, R₃ and R'₃ representing independently of each other a hydrogen atom or an alkyl group comprising from 1 to 10 carbon atoms;
or R₁ and R₂ form together with the carbon atom on which they are bound,
a group 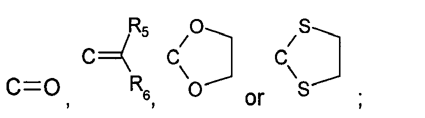 , 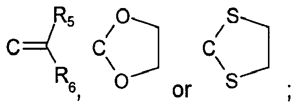
R₅ and R₆ representing independently of each other a hydrogen atom or an alkyl group comprising from 1 to 10 carbon atoms;
- R₄ represents a hydrogen atom or an alkyl group comprising from 1 to 10 carbon atoms;
- R represents an alkyl group comprising from 1 to 10 carbon atoms, an aryl group comprising from 6 to 30 carbon atoms or a cycloalkyl group comprising from 3 to 20 carbon atoms, said aforementioned alkyl, aryl or cycloalkyl groups optionally comprising one or more heteroatoms, notably O, S or N, optionally substituted,
R notably representing an alkyl group optionally substituted with an amine group, or when A is a -CO- group, R and R₄ may form together with the nitrogen atoms on which they are attached, a ring of the following formula (II): 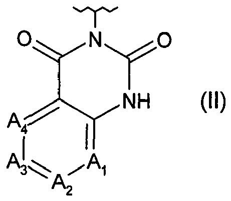
wherein one of the atoms among A₁, A₂, A₃ and A₄ represents N, and the three other atoms among A₁, A₂, A₃ and A₄ represent CH,
as well as its pharmaceutically acceptable salts,
said compound of formula (I) being in the form of a pure stereoisomer or in the form of a mixture of enantiomers or diastereoisomers including racemic mixtures.

2. The compound according to claim 1, wherein R represents an aryl group selected from the group formed by phenyl, benzyl, pyridinyl, pyrimidyl, pyrazinyl, triazinyl, pyrazolyl, isoxazolyl, thiazolyl, benzothiazothiazolyl and quinolinyl groups optionally substituted, or in that R represents cyclohexyl or piperidinyl, optionally substituted.

3. The compound according to any of claims 1 or 2, wherein R is selected from one of the following groups: 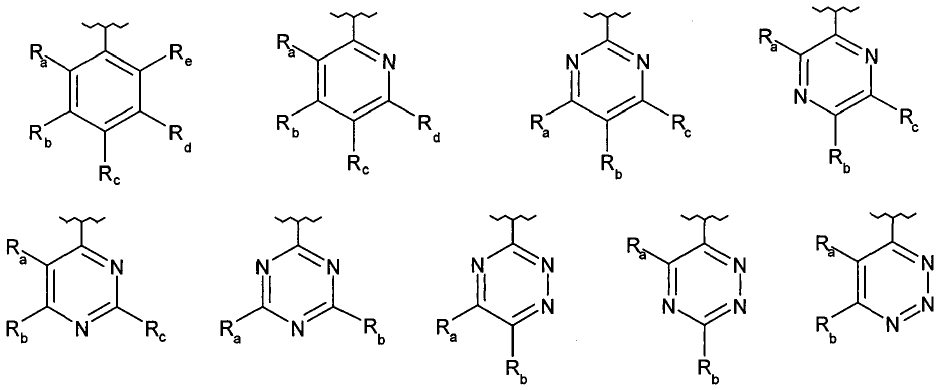 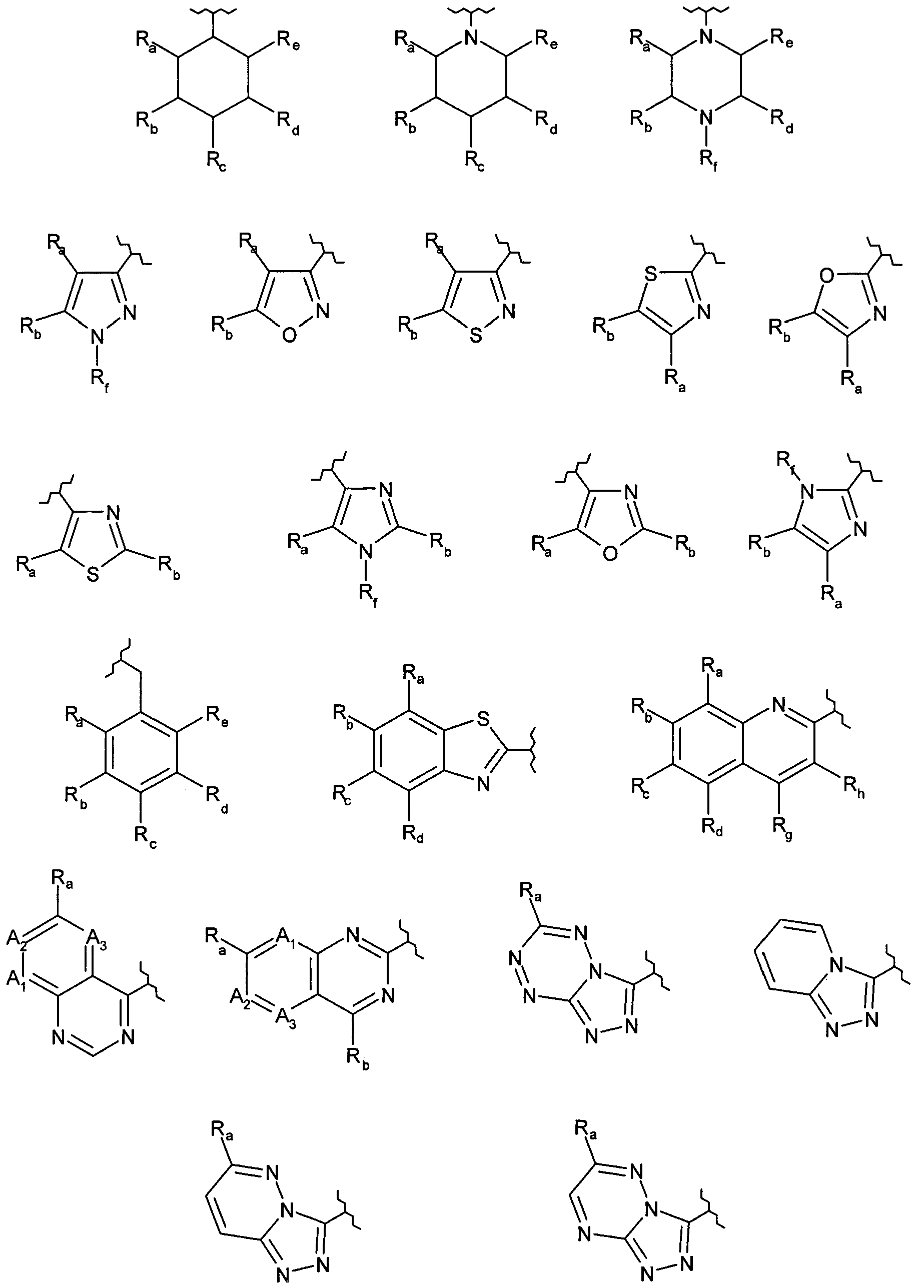 the groups Rₐ, R_{b}, R_{c}, R_{d}, Rₑ, R_{g} and Rₕ being selected independently of each other from the group formed by the following substituents:
- a hydrogen atom,
- a halogen atom notably Br, Cl or F,
- an alkyl group comprising from 1 to 10 carbon atoms and preferably being a methyl group,
said alkyl group being optionally substituted notably with one or more substituents selected from the group formed by the following substituents:
. halogen atoms,
. alkenyl or alkynyl groups comprising from 2 to 10 carbon atoms,
. aryl groups comprising from 6 to 30 carbon atoms,
. COR_{α}, COOR_{α}, SR_{α}, OR_{α} or NR_{α}R_{β} groups, R_{α} and R_{β} representing independently of each other a hydrogen atom, an alkyl group comprising from 1 to 10 carbon atoms or an aryl group comprising from 6 to 30 carbon atoms,
- a -CHO group,
- a -CN group,
- a phenyl group,
- a -SR_{α} or OR_{α} group, R_{α} being as defined above, notably a -OH, -OCH₃, -SH, - SCH₃, -O-CH(CH₃)₂, -O-CH₂-CH₂-CH₃ group,
- a -NR_{α}R_{β} group, R_{α} and R_{β} being as defined above, notably an NH₂ group,
- a -CONR_{α}R_{β} group, R_{α} and R_{β} being as defined above, notably -CONH₂,
- a -NHCOR_{α} group, R_{α} being as defined above, and
- a 2-pyridinyl group,
one of the atoms among A₁, A₂ and A₃ representing N, and the two other atoms from A₁, A₂ and A₃ representing CH,
the R_{f} group being a hydrogen atom, an alkyl group comprising from 1 to 10 carbon atoms.

4. The compound according to any of claims 1 to 3, fitting the following general formula (I-1-a): 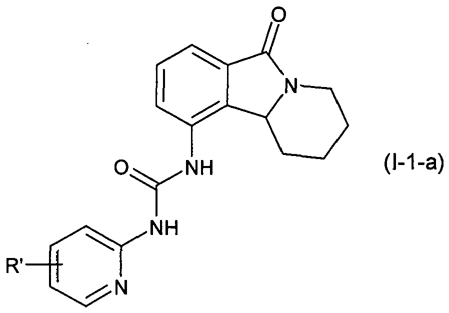 wherein R' represents a hydrogen atom, a halogen atom such as Br or F or an alkyl group comprising from 1 to 10 carbon atoms, preferably a methyl group.

5. The compound according to any of claims 1 to 4, for its use as drug.

6. The compound according to any of claims 1 to 4, for its use as CDK1, CDK5 and/or GSK3 kinase inhibitors.

7. The compound according to any of claims 1 to 4, for its use for treating or preventing a disease related to deregulation of CDK1, CDK5 and/or GSK3 kinases.

8. A method for preparing a compound of general formula (I) according to claim 1, wherein R₄ and R" are H and A is a CO group, and R₁ and R₂ are H or form together with the carbon atom to which they are attached,
a group 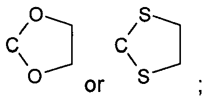 said method comprising:
a reaction step of the amine of the following formula: 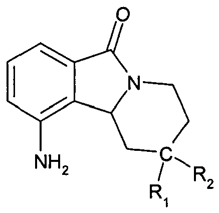 with an isocyanate of formula RNCO, R being as defined in claim 1.

9. A method for preparing compound of general formula (I) according to claim 1, wherein R₄ and R" are H and A is a CS group, and R₁ and R₂ are H or form together with the carbon atom to which they are attached,
a group 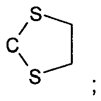 said method comprising:
a reaction step of the isothiocyanate of the following formula: 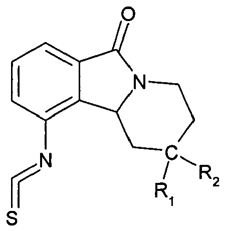
with an amine of formula RNH₂, R being as defined in claim 1.

10. A method for preparing a compound of general formula (I) according to claim 1, wherein R₄ and R" are H and A is a CO group, and R₁ and R₂ are H or form together with the carbon atom to which they are attached,
a group 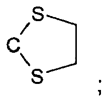 said method comprising:
a reaction step of the thiourea of the following formula: 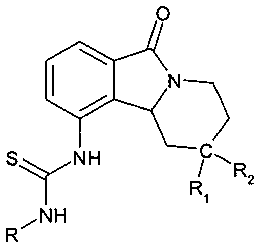 R being as defined in claim 1,
in the presence of a CH₃CN/water mixture and of HgO at room temperature for a duration comprised from 24 to 40 hours.

11. A method for preparing a compound of general formula (I) according to claim 1, wherein R₄ and R" are H and A is a CO group CO, and R₁ and R₂ are H or form together, with the carbon atom to which they are attached,
a group 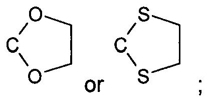 said method comprising:
a reaction step of the amine of the following formula: 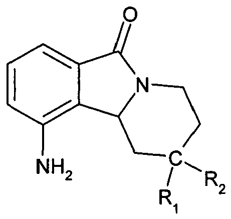
with an amine of formula RNH₂ and triphosgen, R being as defined in claim 1.

12. A method for preparing a compound of general formula (I) according to claim 1, wherein R₁ and R₂ form together, with the carbon atom to which they are attached, a C=O group;
said method comprising:
a reaction step of the acetal of the following formula: 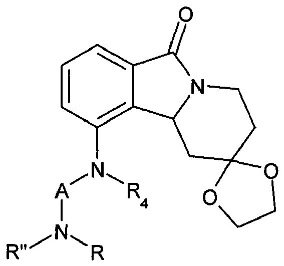 A, R, R" and R₄ being as defined in claim 1,
with acetone and hydrochloric acid.

13. A method for preparing a compound of general formula (I) according to claim 1,
wherein R₁ is H and R₂ is OH,
said method comprising:
a reduction step of the ketone of the following formula: 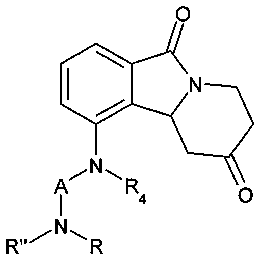 A, R, R" and R₄ being as defined in claim 1,
notably with NaBH₄ in the presence of THF and methanol.

14. Intermediate compounds fitting one of the following formulae:

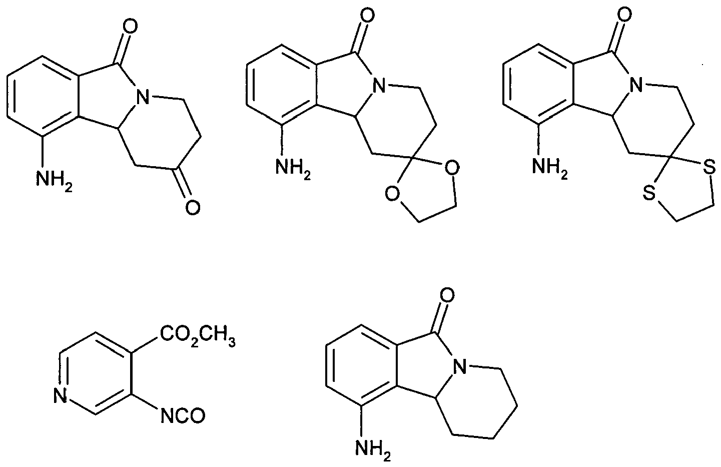

## Patentansprüche

1. Verbindung der folgenden allgemeinen Formel (I): 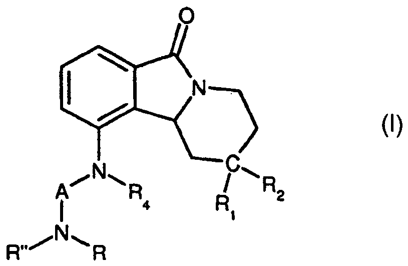 worin:
- A eine Gruppe 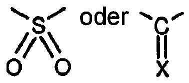 darstellt;
- X O oder S darstellt;
- R" H oder eine 1 bis 10 Kohlenstoffatome umfassende Alkylgruppe darstellt;
- R₁ H darstellt und R₂ ein Wasserstoffatom, eine Gruppe NR₃R'₃ oder eine Gruppe OR₃ darstellt, wobei R₃ und R'₃ unabhängig von einander ein Wasserstoffatom oder eine 1 bis 10 Kohlenstoffatome umfassende Alkylgruppe darstellen;
oder R₁ und R₂ zusammen mit dem Kohlenstoffatom, an das sie gebunden sind,
eine Gruppe C=O, 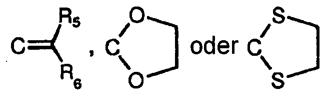 bilden, wobei R₅ und R₆ unabhängig von einander ein Wasserstoffatom oder eine 1 bis 10 Kohlenstoffatome umfassende Alkylgruppe darstellen;
- R₄ ein Wasserstoffatom oder eine 1 bis 10 Kohlenstoffatome umfassende Alkylgruppe darstellt;
- R eine 1 bis 10 Kohlenstoffatome umfassende Alkylgruppe, eine 6 bis 30 Kohlenstoffatome umfassende Arylgruppe oder eine 3 bis 20 Kohlenstoffatome umfassende Cycloalkylgruppe darstellen, wobei die vorgenannten Alkyl-, Aryl- oder Cycloalkylgruppen gegebenenfalls ein oder mehrere Heteroatome, insbesondere O, S oder N, umfassen und gegebenenfalls substituiert sein können,
wobei R insbesondere eine gegebenenfalls durch eine Amingruppe substituierte Alkylgruppe darstellt,
oder wenn A eine Gruppe -CO- ist, R und R₄ zusammen mit den Stickstoffatomen,
an die sie gebunden sind, einen Cyclus der folgenden Formel (II) bilden können: 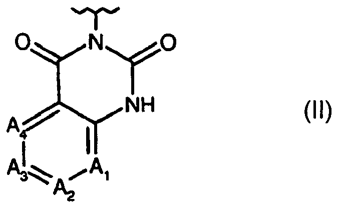 worin eines der Atome aus A₁, A₂, A₃ und A₄ N darstellt und die drei anderen Atome aus A₁, A₂, A₃ und A₄ CH darstellen,
sowie ihre pharmazeutisch annehmbaren Salze,
wobei die Verbindung der Formel (I) in Form eines reinen Stereoisomers oder in Form eines Gemischs von Enantiomeren und/oder Diastereomeren einschließlich racemischer Gemische vorliegt.

2. Verbindung gemäß Anspruch 1, **dadurch gekennzeichnet, dass** R eine Arylgruppe darstellt, die aus der Gruppe ausgewählt ist, die sich aus gegebenenfalls substituierten Phenyl-, Benzyl-, Pyridinyl-, Pyrimidyl-, Pyrazinyl-, Triazinyl-, Pyrazolyl-, Isoxazolyl-, Thiazolyl, Benzothiazolyl- und Chinolinylgruppen zusammensetzt, oder dadurch, dass R eine gegebenenfalls substituierte Cyclohexyl- oder Piperidinylgruppe darstellt.

3. Verbindung gemäß einem der Ansprüche 1 oder 2, **dadurch gekennzeichnet, dass** R aus einer der folgenden Gruppen ausgewählt ist: 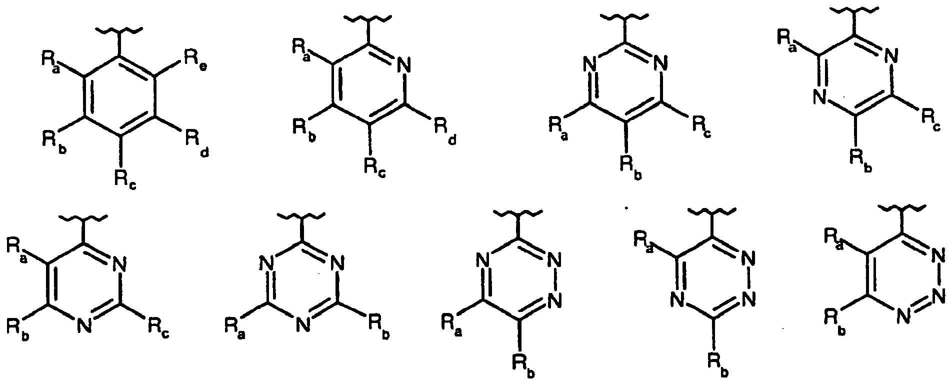 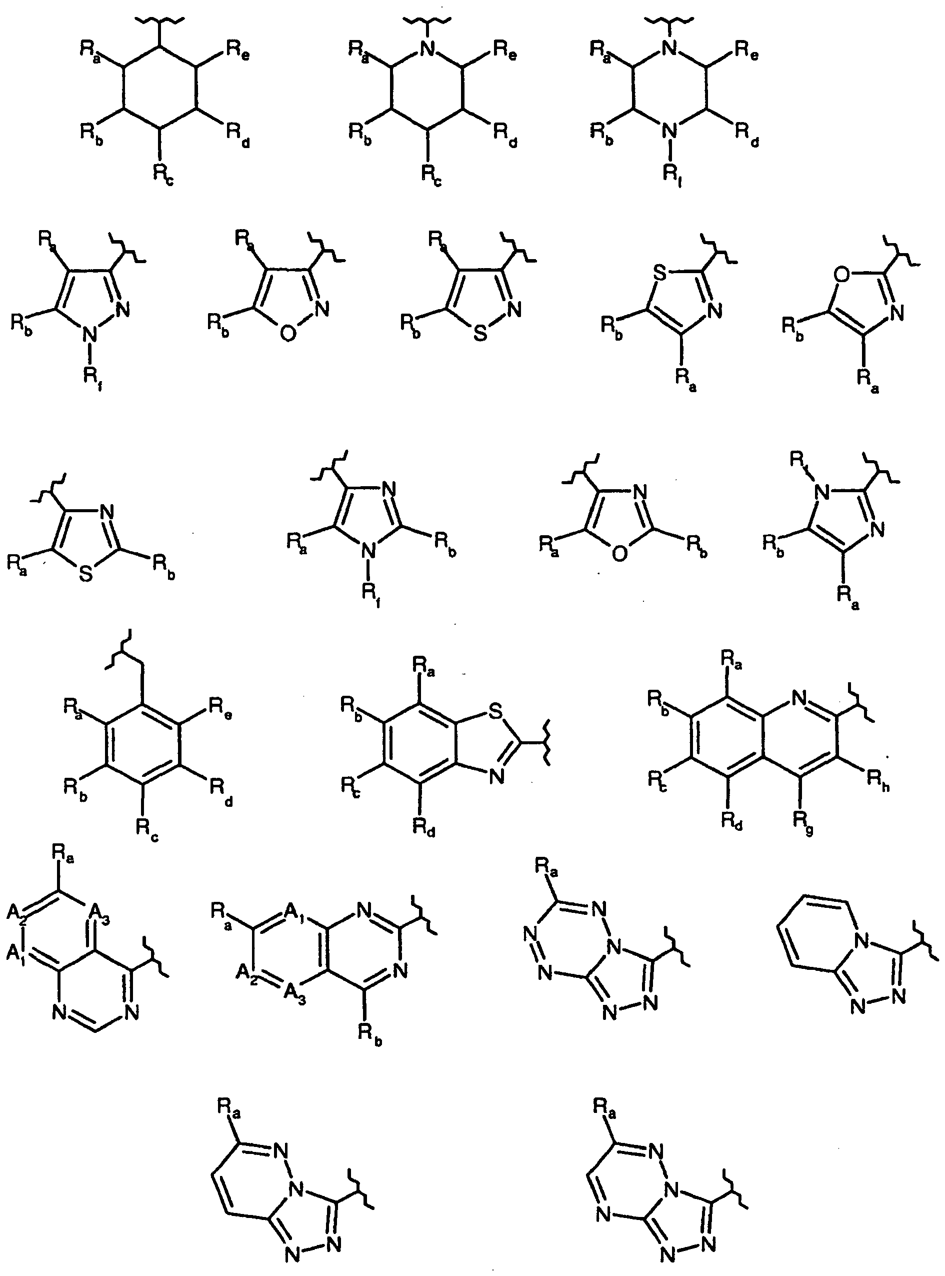 wobei die Gruppen Rₐ, R_{b}, R_{c}, R_{d}, Rₑ, R_{g} und Rₕ unabhängig von einander aus der Gruppe ausgewählt sind, die sich aus den folgenden Substituenten zusammensetzt:
- einem Wasserstoffatom,
- einem Halogenatom, insbesondere Br, Cl oder F,
- einer 1 bis 10 Kohlenstoffatome umfassenden Alkylgruppe, vorzugsweise einer Methylgruppe, wobei die Alkylgruppe gegebenenfalls insbesondere durch einen oder mehrere Substituenten substituiert ist, die aus der Gruppe ausgewählt sind, die sich aus den folgenden Substituenten zusammensetzt:
. Halogenatomen,
. 2 bis 10 Kohlenstoffatome umfassende Alkenyl- oder Alkinylgruppen,
. 6 bis 30 Kohlenstoffatome umfassende Arylgruppen,
. Gruppen COR_{α}, COOR_{α}, SR_{α}, OR_{α} oder NR_{α}R_{β}, worin R_{α} und R_{β} unabhängig von einander ein Wasserstoffatom, eine 1 bis 10 Kohlenstoffatome umfassende Alkylgruppe oder eine 6 bis 30 Kohlenstoffatome umfassende Arylgruppe darstellen,
- einer Gruppe -CHO,
- einer Gruppe -CN,
- einer Phenylgruppe,
- einer Gruppe -SR_{α} oder OR_{α}, wobei R_{α} wie vorstehend definiert ist, insbesondere einer Gruppe -OH, -OCH₃, -SH, -SCH₃, -O-CH(CH₃)₂, -O-CH₂-CH₂-CH₃,
- einer Gruppe -NR_{α}R_{β}, worin R_{α} und R_{β} wie vorstehend definiert sind, insbesondere einer NH₂-Gruppe,
- einer Gruppe -CONR_{α}R_{β}, wobei R_{α} und R_{β} wie vorstehend definiert sind, insbesondere -CONH₂,
- einer Gruppe -NHCOR_{α}, wobei R_{α} wie vorstehend definiert ist, und einer
- einer 2-Pyridinylgruppe,
eines der Atome aus A₁, A₂ und A₃ N darstellt und die beiden anderen Atome aus A₁, A₂ und A₃ CH darstellen,
wobei die Gruppe R_{f} ein Wasserstoffatom oder eine 1 bis 10 Kohlenstoffatome umfassende Alkylgruppe ist.

4. Verbindung gemäß einem der Ansprüche 1 bis 3, die der folgenden allgemeinen Formel (I-1-a) entspricht: 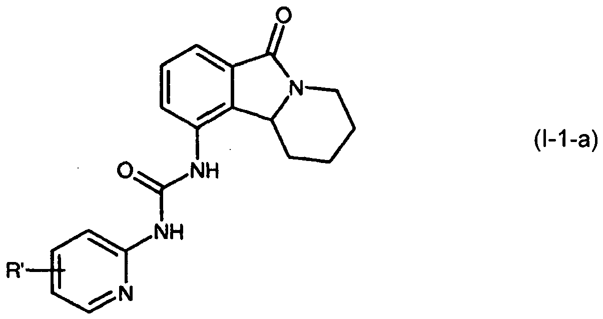 wobei R' ein Wasserstoffatom, ein Halogenatom wie etwa Br oder F oder eine 1 bis 10 Kohlenstoffatome umfassende Alkylgruppe, bevorzugt eine Methylgruppe darstellt.

5. Verbindung gemäß einem der Ansprüche 1 bis 4 zur Verwendung als Arzneimittel.

6. Verbindung gemäß einem der Ansprüche 1 bis 4 zur Verwendung als Hemmer der Kinasen CDK1, CDK5 und/oder GSK3.

7. Verbindung gemäß einem der Ansprüche 1 bis 4 zur Verwendung bei der Behandlung oder Prophylaxe mit einer Deregulierung der Kinasen CDK1, CDK5 und/oder GSK3 verbundener Krankheiten.

8. Verfahren zur Herstellung einer Verbindung der allgemeinen Formel (I) gemäß Anspruch 1, worin R₄ und R" H sind und A eine Gruppe CO ist und R₁ und R₂ H sind oder zusammen mit dem Kohlenstoffatom, an das sie gebunden sind, eine Gruppe 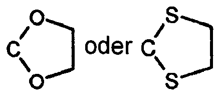 bilden, wobei das Verfahren Folgendes umfasst:
einen Schritt der Reaktion des Amins der folgenden Formel: 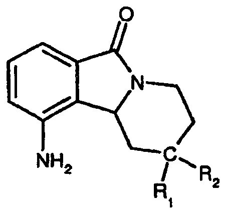 mit einem Isocyanat der Formel RNCO, wobei R wie in Anspruch 1 definiert ist.

9. Verfahren zur Herstellung einer Verbindung der allgemeinen Formel (I) gemäß Anspruch 1, worin R₄ und R" H sind und A eine Gruppe CS ist und R₁ und R₂ H sind oder zusammen mit dem Kohlenstoffatom, an das sie gebunden sind, eine Gruppe 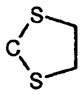 bilden, wobei das Verfahren Folgendes umfasst:
einen Schritt der Reaktion des Isothiocyanats der folgenden Formel: 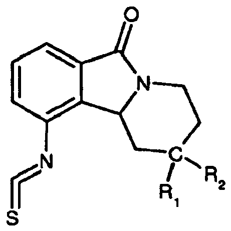 mit einem Amin der Formel RNH₂, wobei R wie in Anspruch 1 definiert ist.

10. Verfahren zur Herstellung einer Verbindung der allgemeinen Formel (I) gemäß Anspruch 1, worin R₄ und R" H sind und A eine Gruppe CO ist und R₁ und R₂ H sind oder zusammen mit dem Kohlenstoffatom, an das sie gebunden sind, eine Gruppe 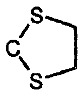 bilden, wobei das Verfahren Folgendes umfasst:
einen Schritt der Reaktion des Thioharnstoffs der folgenden Formel: 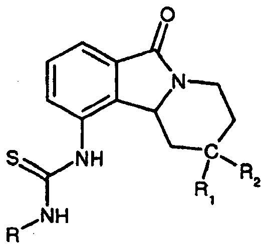 wobei R wie in Anspruch 1 definiert ist, in Gegenwart eines CH₃CN/Wasser-Gemischs und HgO bei Raumtemperatur über einen Zeitraum zwischen 24 und 40 Stunden.

11. Verfahren zur Herstellung einer Verbindung der allgemeinen Formel (I) gemäß Anspruch 1, worin R₄ und R" H sind und A eine Gruppe CO ist und R₁ und R₂ H sind oder zusammen mit dem Kohlenstoffatom, an das sie gebunden sind, eine Gruppe 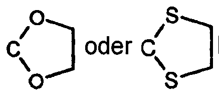 bilden, wobei das Verfahren Folgendes umfasst:
einen Schritt der Reaktion des Amins der folgenden Formel: 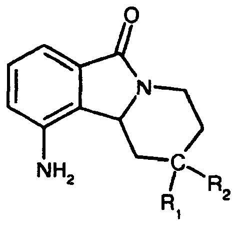 mit einem Amin der Formel RNH₂ und Triphosgen, wobei R wie in Anspruch 1 definiert ist.

12. Verfahren zur Herstellung einer Verbindung der allgemeinen Formel (I) gemäß Anspruch 1, worin R₁ und R₂ zusammen mit dem Kohlenstoffatom, an das sie gebunden sind, eine Gruppe C=O bilden, wobei das Verfahren Folgendes umfasst:
einen Schritt der Reaktion des Acetals der folgenden Formel: 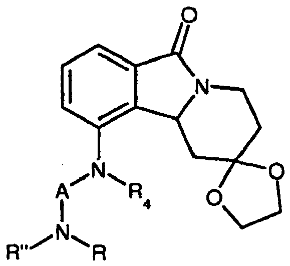 wobei A, R, R" und R₄ wie in Anspruch 1 definiert sind, mit Aceton und Salzsäure.

13. Verfahren zur Herstellung einer Verbindung der allgemeinen Formel (I) gemäß Anspruch 1, worin R₁ H ist und R₂ OH ist, wobei das Verfahren Folgendes umfasst:
einen Schritt der Reduktion des Ketons der folgenden Formel: 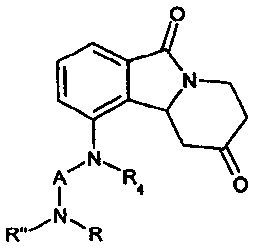 wobei A, R, R" und R₄ wie in Anspruch 1 definiert sind, insbesondere mit NaBH₄ in Gegenwart von THF und Methanol.

14. Zwischenverbindungen entsprechend einer der folgenden Formeln:
